# EUROPEAN PATENT APPLICATION

(11) **EP 4 471 049 A1**
(43) Date of publication of application: **04.12.2024**
(21) Application number: 23176863.1
(22) Date of filing: 01.06.2023
(51) Int. Cl.: C07K 7/06, C07K 7/08, A61P 17/06, A61P 37/00, A61P 1/04, A61K 38/00

(54) **PEPTIDE INHIBITORS OF INTERLEUKIN-23 RECEPTOR**

(71) Applicant: Zealand Pharma A/S, 2860 Søborg (DK)
(72) Inventor: The designation of the inventor has not yet been filed
(74) Representative: D Young & Co LLP

(57) **Abstract**

The present invention relates to compounds that are peptide inhibitors of interleukin-23 receptor (IL-23R) and to their use in the treatment or prevention of a variety of diseases, conditions or disorders, including inflammatory diseases, such as inflammatory bowel disease, such as Crohn's disease, ulcerative colitis, and psoriasis. The compounds have good physical and chemical stability in the gastrointestinal tract.

## Description

### FIELD OF THE INVENTION

The present invention relates to peptide inhibitors of interleukin-23 receptor (IL-23R), and to their medical use in the treatment and/or prevention of a variety of diseases, conditions or disorders, including inflammatory bowel disease, such as Crohn's disease, ulcerative colitis, and psoriasis, and other conditions and disorders described herein.

### BACKGROUND OF THE INVENTION

Interleukin-23 (IL-23) is a heterodimeric cytokine composed of a unique p19 subunit and the p40 subunit of interleukin-12 (IL-12). IL-12 is a cytokine involved in the development of interferon-gamma (IFN-γ)-producing T helper 1 (Th1) cells. Although both IL-23 and IL-12 contain the p40 subunit, they have different phenotypic properties. Animals deficient in IL-12 are susceptible to inflammatory autoimmune diseases, whereas IL-23 deficient animals are resistant. This is thought to be due to a reduced number of CD4⁺ T cells producing interleukin-6 (IL-6), interleukin-17 (IL-17), and tumor necrosis factor (TNF) in the central nervous system (CNS) of IL-23-deficient animals. Furthermore, in contrast to IL-12 which acts mainly on naive CD4⁺ T cells, IL-23 preferentially acts on memory CD4⁺ T cells.

The receptor that binds IL-23 is the interleukin-23 receptor (IL-23R). IL-23R is a heterodimeric receptor composed of IL-12Rβ1 and IL-23R subunits. Binding of IL-23 to IL-23R activates the JAK-STAT signalling pathway: activating the Janus kinase (JAK) molecules JAK2 and tyrosine kinase 2 (TYK2), as well as the signal transducer and activator of transcription proteins (STATs) STAT1, STAT3, STAT4, and STATS. STAT4 activation is substantially weaker and different DNA-binding STAT complexes form in response to IL-23 as compared with IL-12. IL-23R associates constitutively with JAK2 and in a ligand-dependent manner with STAT3.

IL-23R is expressed on various adaptive and innate immune cells, including: T-helper 17 (Th17) cells, gamma-delta (γδ) T cells, natural killer (NK) cells, dendritic cells, macrophages, and innate lymphoid cells. These cells are abundantly found in the intestine. In particular, the gene expression and protein levels of IL-23R at the intestine mucosal surface are found to be elevated in inflammatory bowel disease (IBD) patients. It is thought that IL-23 mediates this effect by promoting the development of a pathogenic CD4⁺ T cell population that produces IL-6, IL-17, and TNF.

IL-23 production is enriched in the intestine, where it is believed to play a key role in regulating the balance between tolerance and immunity through both T-cell-dependent and independent pathways of intestinal inflammation through effects on Th1 and Th17-associated cytokines. IL-23 is also thought to restrain regulatory T-cell responses in the gut, favoring inflammation. Furthermore, IL-23R polymorphisms have been associated with susceptibility to inflammatory bowel diseases (IBDs), further establishing the critical role of the IL-23 pathway in intestinal homeostasis.

Therefore, IL-23 is thought to play a crucial role in the pathogenesis of autoimmune inflammation and related diseases and disorders, such as multiple sclerosis, asthma, rheumatoid arthritis, psoriasis, and inflammatory bowel diseases (IBDs), e.g., ulcerative colitis and Crohn's disease. Studies in acute and chronic mouse models of IBD have revealed a primary role of IL-23R and downstream effector cytokines in disease pathogenesis.

Protagonist Therapeutics, Inc. had peptide PTG-200 in Phase II clinical trials for Crohn's disease. Protagonist in collaboration with Janssen Biotech, Inc. also have two second generation peptides in clinical trials: JNJ-77242113 (or JNJ-2113; formerly PN-235) for psoriasis; and PN-232. Protagonist have filed several patent applications in the area of IL-23R inhibitors: WO 2016/011208, WO 2017/011820, WO 2018/022937, WO 2018/136646, WO 2020/014646, WO 2021/007433, WO 2021/146441, WO 2021/146458, WO 2023/288017, WO 2023/288019, and WO 2023/288028. Protagonist also disclose another peptide, Compound C, as an IL-23R inhibitor in WO 2016/011208, WO 2017/011820, and Sayago et al., 2018.

Notably, only single bridging moieties are present in the peptides disclosed by the Protagonist patent applications WO 2016/011208, WO 2017/011820, WO 2018/022937, WO 2018/136646, WO 2020/014646, WO 2021/007433, WO 2021/146441, WO 2021/146458, and WO 2023/288028. None of these applications disclose the use of two bridging moieties to stabilise the peptide inhibitor of IL-23R.

Heinis *et al.,* 2020 discloses the development of proteolytically resistant therapeutic peptides for oral administration. The authors generated peptides as inhibitors of coagulation Factor Xla and other peptides as gastrointestinal-protease resistant peptide antagonists of IL-23R. The peptides generated as antagonists of IL-23R comprised of two dithioether bridges (specifically 1,3-dithio-propan-2-one bridges) between two pairs of cysteine residues in the peptide chain. The authors identified peptide I5 as the most promising candidate for further development as an oral treatment of inflammatory disorders such as Crohn's disease on the basis of IL-23R inhibition.

WO 2023/288017 discloses bicyclic (and some tricyclic) peptide inhibitors of IL-23R. The peptides are up to 15 amino acid residues long, all of which have (at least) two bonds bridging between certain amino acid residues. WO 2023/288019 discloses lipidated peptide inhibitors of IL-23R. The peptides are up to 15 amino acid residues long, and either have one or two bonds bridging between certain amino acid residues. Of the peptides with two bridges, one is either a disulfide or dithioether bond and the other is an amide bond. Both applications propose the use of the inhibitors for the treatment of autoimmune inflammation and related diseases and disorders, including IBD, Crohn's disease, ulcerative colitis, psoriasis, and psoriatic arthritis.

However, none of the bridges in the peptides disclosed in Heinis et al., 2020, WO 2023/288017 and WO 2023/288019 - either by position, type of bond, and/or number of amino acid residues between the bridging amino acids - are the same as the present invention.

Challenges still remain with respect to identifying stable and selective agents that preferentially target the IL-23 pathway, which can be used for the treatment of intestinal inflammation. In particular, the inventors have identified the gastrointestinal stability and IL-23R potency of the peptides disclosed in Heinis *et al.*, 2020 could still be further improved for an orally dosed IL-23R peptide blocker. In particular, the most-promising candidate, peptide I5 from Heinis *et al*., 2020, exhibited a lower stability under a simulated intestinal fluid (SIF) assay and a lower potency for IL-23R in comparison with Protagonist's Compound C (Sayago *et al*., 2018).

Therefore, there remains a need for new therapeutics targeting the IL-23 pathway, which may be used to treat and prevent IL-23-associated diseases, including those associated with autoimmune inflammation in the intestinal tract. Furthermore, compounds and methods for specific targeting of IL-23R from the luminal side of the gut may provide therapeutic benefit to IBD patients suffering from local inflammation of the intestinal tissue.

The inventors have also filed PCT/EP2022/084077 directed to peptide inhibitors of IL-23R. The present invention is directed to further peptide inhibitors with improved stability in the gastrointestinal tract and/or more potent inhibition of IL-23R than the peptides disclosed in PCT/EP2022/084077.

### SUMMARY OF THE INVENTION

The present invention relates to compounds which are peptide inhibitors of interleukin-23 receptor (IL-23R). These compounds exhibit a good combination of properties such as highly potent inhibition of IL-23R and/or high stability in the gastrointestinal tract. Furthermore, the compounds described herein may be useful in the treatment of various diseases, conditions and disorders related to IL-23R such as inflammatory bowel disease, Crohn's disease, ulcerative colitis, and psoriasis. The compounds described herein exhibit improved properties as compared to the compounds disclosed in PCT/EP2022/084077.

The present invention addresses these needs by providing novel peptide inhibitors that bind IL-23R to inhibit IL-23 mediated signalling. The novel peptide inhibitors are also suitable for oral administration due to their stability in the gastrointestinal tract.

In a first aspect, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula I:

   X1-X2-X3-X4-X5-X6-X7-X8-X9-X10-X11-X12-X13-X14 (I)

   wherein
   X1 is absent or is selected from 3-(3-Pyridyl)-Ala or 3-aminopropanoyl;
   X2 is selected from the group consisting of 4-aminomethyl-phenylacetyl, 3-(3-Pyridyl)-Ala, and 3-aminopropanoyl;
   X3 is selected from the group consisting of Ser, Ala, Phe, and N-Me-Ser;
   X4 is Glu or Cys;
   X5 is Trp;
   X6 is selected from the group consisting of Gln, Q(Me), Q(2Me), Q(pyrrolidin), Ala, Phe, Lys(Ac), Dab(Ac), Cit, and Orn;
   X7 is Dab or Cys;
   X8 is selected from the group consisting of Y(2-aminoethoxy), homo-Phe, 7-AzaTrp, beta-homo-Trp, and 7-F-Trp;
   X9 is selected from the group consisting of 2-Nal, 7-AzaTrp, beta-homo-Trp, and Cyclopropyl-Ala;
   X10 is 2-Me-Leu or Aib;
   X11 is Glu;
   X12 is selected from the group consisting of Dab, iso-Dab, D-Arg, Gly-CF3, D-Gly-CF3, Nle, Gln, His, THP, Ser, D-Ser, 2-Me-Ser, Ser(OMe), and homo-Ser;
   X13 is selected from the group consisting of 3-(3-Pyridyl)-Ala, D-3-(3-Pyridyl)-Ala, 2-Me-3-(3-Pyridyl)-Ala, N-Me-3-(3-Pyridyl)-Ala, 3-(3,5-Pyrimidyl)-Ala, His, D-His, and His(Me), or is absent; and
   X14 is absent or is selected from the group consisting of Sar, D-His, beta-homo-Leu, and 3-(3-Pyridyl)-Ala;
   wherein
      (i) the Glu at X11 forms a lactam bridge with the amino acid residue at X1 or at X2 when X1 is absent; and
      (ii) X4 and X7 are amino acid residues who together form a lactam bridge or a dithioether bridge;
      or a pharmaceutically acceptable salt or solvate thereof.

In some embodiments, the compound is not:

| | |
|---|---|
| Ref 80 | [4-Aminomethyl-phenylacetyl](1c)*SC(2a)WQC(2a)[Y(2-aminoethoxy)][2-Nal][2-Me-Leu]E(1c)[{d}R]-[NH2] |
| Ref 81 | [4-Aminomethyl-phenylacetyl](1c)*SC(2a)WQC(2a)[Y(2-aminoethoxy)][2-Nal][2-Me-Leu]E(1c)S-[NH2] |
| Ref 82 | [4-Aminomethyl-phenylacetyl](1c)*SC(2a)WQC(2a)[Y(2-aminoethoxy)][2-Nal][2-Me-Leu]E(1c)[Dab]-[NH2] |
| Ref 120 | [4-Aminomethyl-phenylacetyl](1c)*SC(2a)WQC(2a)[Y(2-aminoethoxy)][2-Nal][2-Me-Leu]E(1c)[Dab][3-(3-Pyridyl)-Ala]-[NH2] |
| Ref 122 | [4-Aminomethyl-phenylacetyl](1c)*SE(2c)WQ[Dab](2c)[Y(2-aminoethoxy)][2-Nal][2-Me-Leu]E(1c) [Dab] [3-(3-Pyridyl)-Ala]-[NH2] |
| Ref 125 | [4-Aminomethyl-phenylacetyl](1c)*SE(2c)WQ[Dab](2c)[Y(2-aminoethoxy)][2-Nal][2-Me-Leu]E(1c)[Dab]-[NH2] |
| Ref 141 | [4-Aminomethyl-phenylacetyl](1c)*[N-Me-Ser]C(2a)WQC(2a)[Y(2-aminoethoxy)][2-Nal][2-Me-Leu]E(1c)[Dab]-[NH2] |
| Ref 146 | [4-Aminomethyl-phenylacetyl](1c)*SE(2c)W[Q(pyrrolidin)][Dab](2c)[Y(2-aminoethoxy)][2-Nal][2-Me-Leu]E(1c)[Dab]-[NH2] |

and pharmaceutically acceptable salts and solvates thereof;
wherein:
* denotes that the (1c), (2a) and (2c) bridges use the peptide backbone amine or carboxylic acid at the *N*- or *C*-terminus, not the side chain amine or carboxylic acid
(1c) denotes the [2,11] lactam bridge; (2a) denotes the [4,7] 1,3-dithio-propan-2-one bridge; (2c) denotes the [4,7] lactam bridge.

Reference compounds Ref 80, Ref 81, Ref 82, Ref 120, Ref 122, Ref 125, Ref 141, and Ref 146 were disclosed in PCT/EP2022/084077. The present invention is not directed to the compounds disclosed in PCT/EP2022/084077.

In some embodiments, the compound is not when X1 is absent; X2 is 4-aminomethyl-phenylacetyl; X3 is Ser or N-Me-Ser; X6 is Gln or Q(pyrrolidin); X8 is Y(2-aminoethoxy); X9 is 2-Nal, X10 is 2-Me-Leu; X12 is Dab, D-Arg, or Ser; X13 is 3-(3-Pyridyl)-Ala or absent; X14 is absent; and R² is NH₂.

In some embodiments, X1 is absent.

In some embodiments, X2 is 4-aminomethyl-phenylacetyl.

In some embodiments, X3 is Ser or Ala. In some embodiments, X3 is Ser.

In some embodiments, X4 is Glu, X7 is Dab, and wherein X4 and X7 together form a lactam bridge.

In some embodiments, X6 is Gln.

In some embodiments, X8 is Y(2-aminoethoxy).

In some embodiments, X9 is 2-Nal.

In some embodiments, X10 is 2-Me-Leu.

In some embodiments, X12 is Dab.

In some embodiments, X13 is 3-(3-Pyridyl)-Ala or is absent. In some embodiments, X13 is 3-(3-Pyridyl)-Ala.

In some embodiments, X14 is absent.

In some embodiments, R² is NHR³ wherein R³ is hydrogen or C₁₋₄ alkyl. In some embodiments, R² is NH₂. In some other embodiments, R² is NHMe.

In some embodiments, X4 and X7 are both Cys, and X4 and X7 together form a dithioether bridge, wherein the dithioether bridge between X4 and X7 is of the formula -S-L-Y-L-S-, wherein:
each S is a sulfur atom and is part of the amino acid residue at X4 and X7;
each L is independently C₁₋₄ alkylene; and
Y is either absent or C(=O).

In some embodiments, L is independently C₁₋₂ alkylene. In some embodiments, L is methylene. In some embodiments, Y is C(=O).

In some embodiments, the dithioether bridge between X4 and X7 is of the formula - SCH₂C(=O)CH₂S-, wherein each S is a sulfur atom and is part of the amino acid residue at X4 and X7.

In some embodiments, X1 is absent and X2 is 4-aminomethyl-phenylacetyl.

In some embodiments, X1 is absent; X2 is 4-aminomethyl-phenylacetyl; X8 is Y(2-aminoethoxy); X9 is 2-Nal; and X10 is 2-Me-Leu.

In some embodiments, X1 is absent; X2 is 4-aminomethyl-phenylacetyl; X3 is Ser or Ala; X8 is Y(2-aminoethoxy); X9 is 2-Nal; and X10 is 2-Me-Leu.

In some embodiments, X1 is absent; X2 is 4-aminomethyl-phenylacetyl; X3 is Ser or Ala; X6 is Gln; X8 is Y(2-aminoethoxy); X9 is 2-Nal; and X10 is 2-Me-Leu.

In some embodiments, X13 is 3-(3-Pyridyl)-Ala and X14 is absent.

In some embodiments, X13 is 3-(3-Pyridyl)-Ala, X14 is absent, and R² is NHMe.

In some embodiments, Z is an amino acid sequence selected from the group consisting of a sequence listed in **Table 1-1a**.

In some embodiments, the compound is selected from a compound from **Table 1-1**, or a pharmaceutically acceptable salt or solvate thereof.

The invention further provides a composition comprising a compound as described above. The composition may be a pharmaceutical composition, and may comprise a pharmaceutically acceptable carrier, excipient or vehicle.

The invention further provides a method for the synthesis of a compound as described above. The method may comprise the steps of synthesising the peptide by solid-phase or liquid-phase methodology, and optionally isolating and/or purifying the final product, and optionally further comprising the step of forming an amide bond between the amino acid residues at position X1 or at position X2 when X1 is absent and at position X11, and optionally further comprising the step of forming an amide bond or forming two thioether bonds with a linker between the amino acid residues at positions X4 and X7.

The invention further provides a compound of the invention, or a pharmaceutical compositions comprising said compound, for use in a method of medical treatment.

The invention also provides a compound of the invention, or a pharmaceutical composition comprising said compound, for use in a method of prevention or treatment of inflammatory bowel disease (IBD), Crohn's Disease, ulcerative colitis, and psoriasis. In some embodiments, the condition is inflammatory bowel disease (IBD) and/or psoriasis.

The invention also provides use of a compound of the invention, or the pharmaceutical composition comprising said compound, in the manufacture of a medicament for the prevention or treatment of inflammatory bowel disease (IBD), Crohn's Disease, ulcerative colitis, and psoriasis. In some embodiments, the condition is inflammatory bowel disease (IBD) and/or psoriasis.

The invention also provides a method of prevention or treatment of inflammatory bowel disease (IBD), Crohn's Disease, ulcerative colitis, and psoriasis; which comprises administering to the subject an effective amount of the compound of the invention, or the pharmaceutical composition comprising said compound. In some embodiments, the condition is inflammatory bowel disease (IBD) and/or psoriasis.

Further aspects and embodiments of the present invention will become apparent from the disclosure below.

### DETAILED DESCRIPTION OF THE INVENTION

### Definitions

Unless otherwise defined herein, scientific and technical terms used herein shall have the meanings that are commonly understood by those of ordinary skill in the art. Generally, nomenclature employed herein in connection with techniques of chemistry, molecular biology, cell and cancer biology, immunology, microbiology, pharmacology, and protein and nucleic acid chemistry, described herein, is that well known and commonly used in the art.

All publications, patents and published patent applications referred to in this application are specifically incorporated by reference herein. In case of conflict, the present specification, including its specific definitions, will control.

Throughout this specification, the word "comprise" or variations such as "comprises" or "comprising" will be understood to imply the inclusion of a stated integer or component, or of a stated group of integers or components, but not the exclusion of any other integer or component or group of integers or components.

The singular forms "a", "an", and "the" include the plurals unless the context clearly dictates otherwise.

The term "including" is used to mean "including but not limited to". "Including" and "including but not limited to" are used interchangeably.

The terms "patient", "subject", and "individual" may be used interchangeably and may refer to either a human or a non-human animal. Subjects are typically mammals, including humans, non-human primates (including great apes, Old World monkeys and New World monkeys), livestock animals (e.g., bovines, porcines), companion animals (e.g., canines, felines) and rodents (e.g., mice and rats).

As used herein, the term "pharmaceutically acceptable salt" is intended to indicate a salt which is not harmful to a patient or subject to which the salt in question is administered. It may suitably be a salt chosen, e.g., among acid addition salts and basic salts. Examples of acid addition salts include chloride salts, citrate salts and acetate salts. Examples of basic salts include salts where the cation is selected among alkali metal cations, such as sodium or potassium ions, alkaline earth metal cations, such as calcium or magnesium ions, as well as substituted ammonium ions, such as ions of the type N(R¹)(R²)(R³)(R⁴)⁺, where R¹, R², R³ and R⁴ independently will typically designate hydrogen, optionally substituted C₁₋₆-alkyl or optionally substituted C₂₋₆-alkenyl. Examples of relevant C₁₋₆-alkyl groups include methyl, ethyl, 1-propyl and 2-propyl groups. Examples of C₂₋₆-alkenyl groups of possible relevance include ethenyl, 1-propenyl and 2-propenyl. Other examples of pharmaceutically acceptable salts are described in "Remington's Pharmaceutical Sciences", 17th edition, Alfonso R. Gennaro (Ed.), Mark Publishing Company, Easton, PA, USA, 1985 (and more recent editions thereof), in the "Encyclopaedia of Pharmaceutical Technology", 3rd edition, James Swarbrick (Ed.), Informa Healthcare USA (Inc.), NY, USA, 2007, and in J. Pharm. Sci. 66: 2 (1977).

The term "solvate" in the context of the present invention refers to a complex of defined stoichiometry formed between a solute (*in casu*, a peptide or pharmaceutically acceptable salt thereof according to the invention) and a solvent. The solvent in this connection may, for example, be water, ethanol or another pharmaceutically acceptable - typically small-molecular - organic species, such as, but not limited to, acetic acid or lactic acid. When the solvent in question is water, such a solvate is normally referred to as a hydrate.

The term "antagonist" as employed in the context of the invention refers to a substance that inhibits the receptor type in question, typically by binding to it (i.e. as a ligand) and blocking it.

Each embodiment of the invention described herein may be taken alone or in combination with one or more other embodiments of the invention.

The term "therapeutically effective amount" or "effective amount" as used herein in the context of the above-described methods of treatment or other therapeutic interventions according to the invention refers to an amount that is sufficient to cure, ameliorate, alleviate or partially arrest the clinical manifestations of the particular disease, disorder or condition that is the object of the treatment or other therapeutic intervention in question e.g. as measured by established clinical endpoints or other biomarkers (established or experimental). A therapeutically relevant amount may be determined empirically by one skilled in the art based on the indication being treated or prevented and the subject to whom the therapeutically relevant amount is being administered. For example, the skilled worker may measure one or more of the clinically relevant indicators of bioactivity described herein, e.g. myeloperoxidase (MPO), interleukin-1β (IL-1β), interleukin-6 (IL-6), interleukin-22 (IL-22), interleukin-17A (IL-17A), interleukin-17F (IL-17F), lipocalin 2 (LCN2), matrix metallopeptidase 9 (MMP9), S100 calcium-binding protein A8 (S100A8), microRNA-223-3p (miR223-3p), Claudin 8 (CLDN8), and phosphorylated signal transducer and activator of transcription 3 (pSTAT3) proteins, polynucleotides encoding any of the proteins, and polynucleotides comprising a region complementary to microRNA-223-3p or any of the polynucleotides that encode any of the proteins, as described in WO 2018/089693. The skilled worker may determine a clinically relevant amount through *in vitro* or *in vivo* measurements.

An amount adequate to accomplish any or all of these effects is defined as a therapeutically effective amount. The administered amount and the method of administration can be tailored to achieve optimal efficacy. An amount effective for a given purpose will depend, *inter alia*, on the severity of the disease, disorder or condition that is the object of the particular treatment or other therapeutic intervention, on the body weight and general condition of the subject in question, on diet, on possible concurrent medication, and on other factors well known to those skilled in the medical arts. Determination of an appropriate dosage size and dosing regimen most appropriate for administration of a peptide or pharmaceutically acceptable salt or solvate thereof according to the invention to a human may be guided by the results obtained by the present invention, and may be confirmed in properly designed clinical trials. An effective dosage and treatment protocol may be determined by conventional means, starting with a low dose in laboratory animals and then increasing the dosage while monitoring the effects, and systematically varying the dosage regimen as well. Numerous factors may be taken into consideration by a clinician when determining an optimal dosage for a given subject. Such considerations are well known to the skilled person.

The terms "treatment" and grammatical variants thereof (e.g. "treated", "treating", "treat") as employed in the present context refer to an approach for obtaining beneficial or desired clinical results. For the purposes of this invention, beneficial or desired clinical results include, but are not limited to, alleviation of symptoms, diminishment of extent of disease, stabilization (i.e. not worsening) of state of disease, delay or slowing of disease progression, amelioration or palliation of the disease state, and remission (whether partial or total), whether detectable or undetectable. "Treatment" can also mean prolonging survival relative to expected survival time if not receiving treatment. A subject (e.g. a human) in need of treatment may thus be a subject already afflicted with the disease or disorder in question. The term "treatment" includes inhibition or reduction of an increase in severity of a pathological state or symptoms (e.g. inflammation) relative to the absence of treatment, and is not necessarily meant to imply complete cessation of the relevant disease, disorder or condition.

The terms "prevention" and grammatical variants thereof (e.g., "prevented", "preventing", "prevent") as employed in the present context refer to an approach for hindering or preventing the development of, or altering the pathology of, a condition, disease or disorder. Accordingly, "prevention" may refer to prophylactic or preventive measures. For the purposes of this invention, beneficial or desired clinical results include, but are not limited to, prevention or slowing of symptoms, progression or development of a disease, whether detectable or undetectable. A subject (e.g. a human) in need of "prevention" may thus be a subject not yet afflicted with the disease or disorder in question. The term "prevention" thus includes inhibiting or slowing the onset of disease relative to the absence of treatment, and is not necessarily meant to imply permanent prevention of the relevant disease, disorder or condition.

### Amino acids nomenclature

The term "amino acid" is an organic compound that contains an amino or amine group (-NH₂ or -NHR) and a carboxylic acid (-COOH) group. The carbonyl of the carboxylic acid group may be further functionalised, such as converted to -CF₃ in Gly-CF3 and D-Gly-CF3. This further functionalisation may occur before, during, or after peptide coupling with the other amino acid residues in the peptide chain.

Some amino acids described herein have the amine and carboxylic acid groups attached to the same carbon, called alpha (α) amino acids. Some amino acids described herein have the amine and carboxylic acid groups 1, 2, 3, 4, 5, or 6 carbon atoms away. For example, beta-homo-Trp and beta-homo-Leu have the amine and the carboxylic acid groups 1 carbon away from each other, such that the carbon connected to the amine group and the carbon connected to the carboxylic acid group are adjacent to one another.

Some amino acids described herein have a side chain specific to each amino acid. The side chain may also be further functionalised.

Throughout the present specification, unless naturally occurring amino acids are referred to by their full name (e.g. alanine, arginine, etc.), they are designated by their conventional three-letter or single-letter abbreviations (e.g. Ala or A for alanine, Arg or R for arginine, etc.). In the case of certain less common or non-naturally occurring amino acids (i.e. amino acids other than the 20 encoded by the standard mammalian genetic code), unless they are referred to by their full name (e.g. ornithine, etc.), frequently employed three- or four-character codes are employed for residues thereof, including 2-Nal (3-(2-naphthyl)-alanine).

Unless otherwise indicated, reference is made to the L-isomeric forms of the amino acids in question.

Amino acid residues are amino acid moieties within a peptide chain. Unnatural amino acid residues may be identified as the fragment of the unnatural amino acid defined in a peptide chain (for example, the unnatural amino acid 3-aminopropanoic acid may be identified as the unnatural amino acid residue 3-aminopropanoyl in a peptide chain).

Additional abbreviations for amino acid residues are described in **Table A**.

**Table A**

| **Amino Acid Residue** | **Structure** |
|---|---|
| 3-(3-Pyridyl)-Ala | 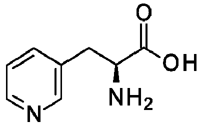 |
| also known as: | |
| 3-(3-pyridyl)-L-alanine, 3-Pal, or (2S)-2-amino-3-(pyridin-3-yl)propanoic acid | |
| 3-aminopropanoyl | 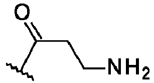 |
| 4-aminomethyl-phenylacetyl | 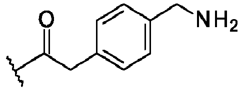 |
| N-Me-Ser | 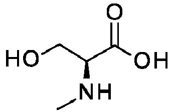 |
| also known as: | |
| N-methyl-L-serine or (2S)-3-hydroxy-2-(methylamino)propanoic acid | |
| Q(Me) | 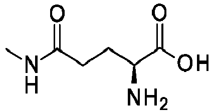 |
| also known as: | |
| (2S)-2-amino-5-(methylamino)-5-oxo-pentanoic acid | |
| Q(2Me) | 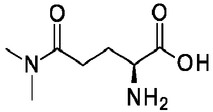 |
| also known as: | |
| (2S)-2-amino-5-(dimethylamino)-5-oxo-pentanoic acid | |
| Q(pyrrolidin) | 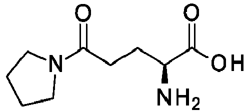 |
| also known as: | |
| (2S)-2-amino-5-oxo-5-pyrrolidin-1-yl-pentanoic acid | |
| Lys(Ac) or K(Ac) | 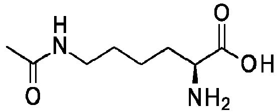 |
| also known as: | |
| acetyllysine, N(6)-acetyl-L-lysine or (2S)-6-acetamido-2-amino-hexanoic acid | |
| Dab(Ac) | 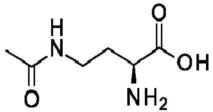 |
| also known as: | |
| (2S)-4-acetamido-2-amino-butanoic acid | |
| Cit | 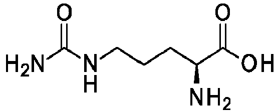 |
| also known as: | |
| L-citrulline or (2S)-2-amino-5-ureido-pentanoic acid | |
| Orn | 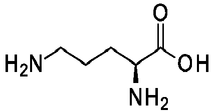 |
| also known as: | |
| L-ornithine or (2S)-2,5-diaminopentanoic acid | |
| Dab (also iso-Dab - see **Table B**) | 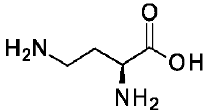 |
| also known as: | |
| (2S)-2,4-diaminobutanoic acid | |
| Y(2-aminoethoxy) | 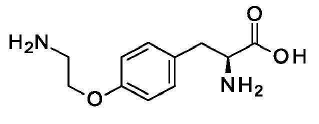 |
| also known as: | |
| (2S)-2-amino-3-[4-(2-aminoethoxy)phenyl]propanoic acid | |
| homo-Phe or hPhe | 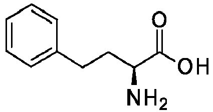 |
| also known as: | |
| L-Homophenylalanine or (2S)-2-amino-4-phenylbutanoic acid | |
| 7-AzaTrp | 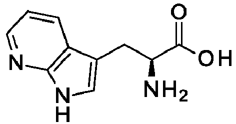 |
| also known as: | |
| (2S)-2-amino-3-(1H-pyrrolo[2,3-b]pyridin-3-yl)propanoic acid | |
| beta-homo-Trp or beta-hTrp | 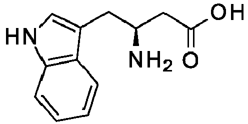 |
| also known as: | |
| L-beta-homotryptophan, L-β-homotryptophan, or (3S)-3-amino-4-(1H-indol-3-yl)butanoic acid | |
| 7-F-Trp | 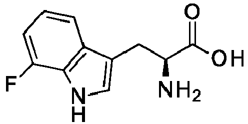 |
| also known as: | |
| (2S)-2-amino-3-(7-fluoro-1H-indol-3-yl)propanoic acid | |
| 2-Nal | 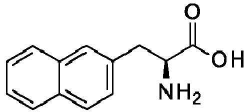 |
| also known as: | |
| 3-(2-naphthyl)-L-alanine or (2S)-2-amino-3-(2-naphthyl)propanoic acid | |
| Cyclopropyl-Ala | 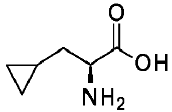 |
| also known as: | |
| cyclopropyl-L-alanine or (2S)-2-amino-3-cyclopropyl-propanoic acid | |
| 2-Me-Leu | 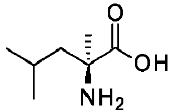 |
| also known as: | |
| 2-methyl-L-leucine, alpha-methylleucine, α-methylleucine, or (2S)-2-amino-2,4-dimethylpentanoic acid | |
| Aib | 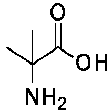 |
| also known as: | |
| 2-aminoisobutyric acid, alpha-methylalanine, α-methylalanine, or 2-methylalanine, or 2-amino-2-methyl-propanoic acid | |
| D-Arg or {d}R | 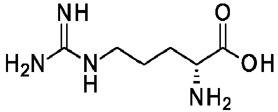 |
| also known as: | |
| D-arginine or (2R)-2-amino-5-guanidino-pentanoic acid | |
| Gly-CF3 | 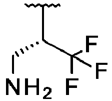 |
| D-Gly-CF3 | 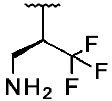 |
| Nle | 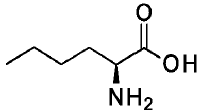 |
| also known as: | |
| L-norleucine or (2S)-2-aminohexanoic acid | |
| THP | 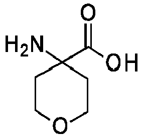 |
| also known as: | |
| 4-aminotetrahydropyran-4-carboxylic acid | |
| D-Ser or {d}S | 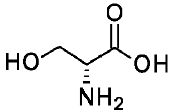 |
| also known as: | |
| D-serine or (2R)-2-amino-3-hydroxy-propanoic acid | |
| 2-Me-Ser | 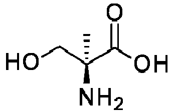 |
| also known as: | |
| 2-methyl-L-serine, alpha-methyl-L-serine (2S)-2-amino-3-hydroxy-2-methylpropanoic acid | |
| Ser(OMe) | 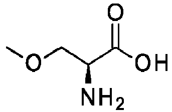 |
| also known as: | |
| O-methyl-L-serine or (2S)-2-amino-3-methoxy-propanoic acid | |
| homo-Ser or hSer | 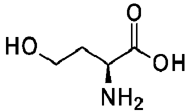 |
| also known as: | |
| L-homoserine or (2S)-2-amino-4-hydroxybutanoic acid | |
| D-3-(3-Pyridyl)-Ala or {d}3-(3-Pyridyl)-Ala | 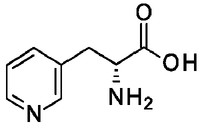 |
| also known as: | |
| 3-(3-pyridyl)-D-alanine, D-3-Pal, or (2R)-2-amino-3-(pyridin-3-yl)propanoic acid | |
| 2-Me-3-(3-Pyridyl)-Ala | 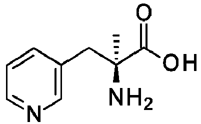 |
| also known as: | |
| 2-Me-3-Pal, or (2S)-2-amino-2-methyl-3-(3-pyridyl)propanoic acid | |
| N-Me-3-(3-Pyridyl)-Ala | 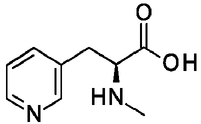 |
| also known as: | |
| N-Me-3-Pal, or (2S)-2-(methylamino)-3-(3-pyridyl)propanoic acid | |
| 3-(3,5-Pyrimidyl)-Ala | 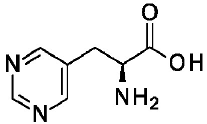 |
| also known as: | |
| (2S)-2-amino-3-pyrimidin-5-yl-propanoic acid | |
| D-His or {d}H | 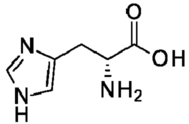 |
| also known as: | |
| D-histidine or (2R)-2-amino-3-(1H-imidazol-4-yl)propanoic acid | |
| His(Me) or H(Me) or His(1-Me) or H(1-Me) | 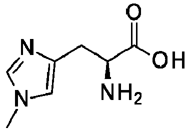 |
| also known as: | |
| 1-methyl-L-histidine or (2S)-2-amino-3-(1-methylimidazol-4-yl)propanoic acid | |
| Sar | 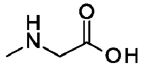 |
| also known as: | |
| sarcosine, N-methyl-glycine, or 2-(methylamino)acetic acid | |
| beta-homo-Leu or beta-hLeu | 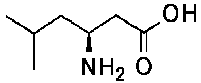 |
| also known as: | |
| L-beta-homoleucine, L-β-homoleucine, or (3S)-3-amino-5-methyl-hexanoic acid | |

Linear peptides are written from N-terminus to C-terminus, left to right.

Unnatural (or non-naturally occurring) amino acids and unnatural (or non-naturally occurring) amino acid residues are amino acids and amino acid residues that do not naturally occur in peptide chains. Unnatural amino acids may be formed as secondary metabolites in bacteria, fungi, plants, or marine organisms, or they can be synthesised chemically.

Unless otherwise stated, the peptide backbone (that is, the amide bonds "-" between X1-X2-X3-X4-X5-X6-X7-X8-X9-X10-X11-X12-X13-X14 in the peptide chain) is formed by the amino acid residues joined by amide bonds via their terminal -NH₂ and -COOH groups. That is, the "terminal -NH₂ group" is the alpha-amine group for alpha amino acids or beta-amine group for beta amino acids; and the "terminal -COOH group" is the alpha-carboxylic acid group for the alpha amino acids.

Where the amino acid residue comprises two or more amine groups (-NH₂, such as Lys and Dab) or carboxylic acid groups (-COOH, such as Glu), the peptide backbone may instead be formed using the side chain of the amino acid residue.

For example, the following nomenclature in **Table B** is used to distinguish the use of the terminal and the side chain -NH₂ and -COOH groups.

**Table B**

| **Amino Acid Residue** | **Residue in peptide chain** | **Iso-residue in peptide chain** |
|---|---|---|
| Dab / iso-Dab | Dab | iso-Dab |
| 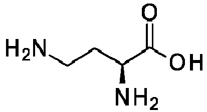 Side chain: | 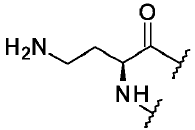 | 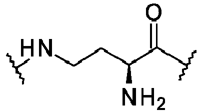 |
| -CH₂CH₂NH₂ | | |

The C-terminus of the peptide may be derivatised with an alkyl group substituted with a pyridyl group to improve gastrointestinal stability (see **Example 2**). The following C-terminal derivatives are disclosed in **Table C**:

**Table C**

| ***C*-terminal Derivatisation (R² group)** | **Structure** |
|---|---|
| NH-(4-(pyridin-3-yl)butanyl) | 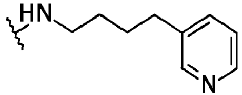 |
| NH-(2-(pyridin-3-yl)ethyl) | 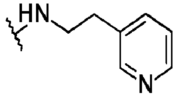 |

For the NH-(4-(pyridin-3-yl)butanyl) and NH-(2-(pyridin-3-yl)ethyl) groups, these are attached to carbonyl carbon of the carboxylic acid group of the C-terminal amino acid residue, such as at Dab. The attachment forms an amide bond.

### Lactam

A lactam is cyclic amide of formula cyclo(R-NH-C(=O)-R) wherein each R may be any other suitable functional group that joins to the other R. Each R may the the same or different.

### Dithioether

A thioether is a functional group of the formula R-S-R, wherein R may be any other suitable functional group. A dithioether is a functional group comprising two thioether groups linked together by a linker, such as R-S-L-Y-L-S-R wherein the linker is -L-Y-L-.

### Head-to-tail cyclisation

The term "head-to-tail cyclisation" is cyclisation of the N-terminal amine (or derivative thereof) and the C-terminal carboxylic acid to form a cyclic peptide. Typically, this cyclisation forms an amide bond.

### C₁₋₄ alkyl groups

C₁₋₄ alkyl groups that may be present as a group R² in the context of compounds of the present invention include, but are not limited to, C₄ alkyl groups such as butyl (n-Bu or-CH₂CH₂CH₂CH₃), or C₁₋₃ alkyl groups, such as methyl (Me or -CH₃, that is a C₁ alkyl group), ethyl (-CH₂CH₃, that is a C₂ alkyl group), 1-propyl (-CH₂CH₂CH₃, that is a C₃ alkyl group), or 2-propyl (-CH(CH₃)₂, that is a C₃ alkyl group).

### C₁₋₃ alkyl groups

C₁₋₃ alkyl groups that may be present as a group R² in the context of compounds of the present invention include methyl (Me or -CH₃, that is a C₁ alkyl group), ethyl (-CH₂CH₃, that is a C₂ alkyl group), 1-propyl (-CH₂CH₂CH₃, that is a C₃ alkyl group), and 2-propyl (-CH(CH₃)₂, that is a C₃ alkyl group).

### C₁₋₂ alkyl groups

C₁₋₂ alkyl groups that may be present as a group R² in the context of compounds of the present invention include methyl (Me or -CH₃, that is a C₁ alkyl group) and ethyl (-CH₂CH₃, that is a C₂ alkyl group).

### C₁₋₄ alkylene groups

C₁₋₄ alkylene groups that may be present as a group L of the dithioether bridge in the context of compounds of the present invention include, but are not limited to, C₁₋₂ alkylene groups, such as methylene (-CH₂-, that is a C₁ alkylene group) and ethylene (-CH₂CH₂-, that is a C₂ alkylene group).

### Bridging moieties

The sequences disclosed herein containing bridging moieties noted in the rounded brackets (e.g., (1a), (2a), etc.). These represent chemical bridges between the specific residue pairs. Each rounded bracket will appear twice in the sequence as a pair to indicate a single bridging moiety. Most of the sequences have two bridging moieties, indicated by 4 sets of rounded brackets meaning two bridging moiety pairs.

The number in the rounded bracket indicates a specific bridging moiety pair (e.g. "1" indicates a bridge between amino acid residues at positions 2 and 11, which is also indicated by the square bracket notation defining the specific chemical bridge). The letter indicates the type of chemical bridge (e.g. "a" indicates a 1,3-dithio-propan-2-one bridge).

The specific chemical bridge is defined at the end of the tables, using a square bracket (e.g. [2,11], [4,7], etc.) to indicate the amino acid residues using in the bridging moiety as compared to the original starting peptide (the I3 peptide (isomer 3) as described in Example 2 of PCT/EP2022/084077), so these may not line-up specifically with the *actual* amino acid numbering of the SEQ ID NO: (as in some of these sequences for example, the first amino acid residue had been deleted as compared to the original starting peptide).

The residue directly preceding the rounded bracket notation indicates that specific residue is used in the bridging moiety.

The "*" notation directly after the rounded bracket notation indicates that the terminal -NH₂ (if at the start of the sequence i.e. the N-terminus) or -COOH (if at the end of the sequence i.e. the C-terminus) is used to form the bridge.

### SMILES strings

The Simplified Molecular-Input Line-Entry System (SMILES) strings are provided below the structures described by the amino acid sequence for each of the compounds disclosed herein. A SMILES string is a line notation for describing the structure of chemical species using short American Standard Code for Information Interchange (ASCII) strings. SMILES strings can be imported by most molecule editors (e.g. ChemDraw^{®}, BIOVIA Draw) for conversion back into two-dimensional or three-dimensional drawings of the chemical structure. Where there is a discrepancy between the structure of the amino acid sequence and the structure provided by the SMILES string, the SMILES string prevails.

### Compounds

The invention provides compounds which are peptide inhibitors of IL-23R. These compounds exhibit a good combination of properties such as highly potent inhibition of IL-23R and/or high stability in the gastrointestinal tract (see Example 3). Furthermore, the compounds described herein may be useful in the treatment of various diseases, conditions and disorders related to IL-23R such as inflammatory bowel disease, Crohn's disease, ulcerative colitis, and psoriasis. The compounds described herein exhibit improved properties (such as more potent inhibition and/or higher gastrointestinal tract stability) as compared to the compounds disclosed in PCT/EP2022/084077.

The invention provides a compound of the formula:

Z-R²

wherein
R² is NHR³ wherein R³ is hydrogen or C₁₋₄ alkyl optionally substituted with a pyridyl ring; and
Z is an amino acid sequence of formula I:

   X1-X2-X3-X4-X5-X6-X7-X8-X9-X10-X11-X12-X13-X14 (I)

   wherein
   X1 is absent or is selected from 3-(3-Pyridyl)-Ala or 3-aminopropanoyl;
   X2 is selected from the group consisting of 4-aminomethyl-phenylacetyl, 3-(3-Pyridyl)-Ala, and 3-aminopropanoyl;
   X3 is selected from the group consisting of Ser, Ala, Phe, and N-Me-Ser;
   X4 is Glu or Cys;
   X5 is Trp;
   X6 is selected from the group consisting of Gln, Q(Me), Q(2Me), Q(pyrrolidin), Ala, Phe, Lys(Ac), Dab(Ac), Cit, and Orn;
   X7 is Dab or Cys;
   X8 is selected from the group consisting of Y(2-aminoethoxy), homo-Phe, 7-AzaTrp, beta-homo-Trp, and 7-F-Trp;
   X9 is selected from the group consisting of 2-Nal, 7-AzaTrp, beta-homo-Trp, and Cyclopropyl-Ala;
   X10 is 2-Me-Leu or Aib;
   X11 is Glu;
   X12 is selected from the group consisting of Dab, iso-Dab, D-Arg, Gly-CF3, D-Gly-CF3, Nle, Gln, His, THP, Ser, D-Ser, 2-Me-Ser, Ser(OMe), and homo-Ser;
   X13 is selected from the group consisting of 3-(3-Pyridyl)-Ala, D-3-(3-Pyridyl)-Ala, 2-Me-3-(3-Pyridyl)-Ala, N-Me-3-(3-Pyridyl)-Ala, 3-(3,5-Pyrimidyl)-Ala, His, D-His, and His(Me), or is absent; and
   X14 is absent or is selected from the group consisting of Sar, D-His, beta-homo-Leu, and 3-(3-Pyridyl)-Ala;
   wherein
      (i) the Glu at X11 forms a lactam bridge with the amino acid residue at X1 or at X2 when X1 is absent; and
      (ii) X4 and X7 are amino acid residues who together form a lactam bridge or a dithioether bridge;
      or a pharmaceutically acceptable salt or solvate thereof.

In all embodiments, the compound of the invention is not:

| | |
|---|---|
| Ref 80 | [4-Aminomethyl-phenylacetyl](1c)*SC(2a)WQC(2a)[Y(2-aminoethoxy)][2-NaI][2-Me-Leu]E(1c)[{d}R]-[NH2] |
| Ref 81 | [4-Aminomethyl-phenylacetyl](1c)*SC(2a)WQC(2a)[Y(2-aminoethoxy)][2-NaI][2-Me-Leu]E(1c)S-[NH2] |
| Ref 82 | [4-Aminomethyl-phenylacetyl](1c)*SC(2a)WQC(2a)[Y(2-aminoethoxy)][2-Nal][2-Me-Leu]E(1c)[Dab]-[NH2] |
| Ref 120 | [4-Aminomethyl-phenylacetyl](1c)*SC(2a)WQC(2a)[Y(2-aminoethoxy)][2-Nal][2-Me-Leu]E(1c)[Dab][3-(3-Pyridyl)-Ala]-[NH2] |
| Ref 122 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala]-[NH2] |
| Ref 125 | [4-Aminomethyl-phenylacetyl](1c)*SE(2c)WQ[Dab](2c)[Y(2-aminoethoxy)][2-Nal][2-Me-Leu]E(1c)[Dab]-[NH2] |
| Ref 141 | [4-Aminomethyl-phenylacetyl](1c)*[N-Me-Ser]C(2a)WQC(2a)[Y(2-aminoethoxy)][2-Nal][2-Me-Leu]E(1c)[Dab]-[NH2] |
| Ref 146 | [4-Aminomethyl-phenylacetyl](1c)*SE(2c)W[Q(pyrrolidin)][Dab](2c)[Y(2-aminoethoxy)][2-Nal][2-Me-Leu]E(1c)[Dab]-[NH2] |

and pharmaceutically acceptable salts and solvates thereof;
wherein:
* denotes that the (1c), (2a) and (2c) bridges use the peptide backbone amine or carboxylic acid at the *N*- or *C*-terminus, not the side chain amine or carboxylic acid
(1c) denotes the [2,11] lactam bridge; (2a) denotes the [4,7] 1,3-dithio-propan-2-one bridge; (2c) denotes the [4,7] lactam bridge.

Reference compounds Ref 80, Ref 81, Ref 82, Ref 120, Ref 122, Ref 125, Ref 141, and Ref 146 were disclosed in PCT/EP2022/084077. The present invention is not directed to the compounds disclosed in PCT/EP2022/084077.

In some embodiments, the compound is not when X1 is absent; X2 is 4-aminomethyl-phenylacetyl; X3 is Ser or N-Me-Ser; X6 is Gln or Q(pyrrolidin); X8 is Y(2-aminoethoxy); X9 is 2-Nal, X10 is 2-Me-Leu; X12 is Dab, D-Arg, or Ser; X13 is 3-(3-Pyridyl)-Ala or absent; X14 is absent; and R² is NH₂.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula II:

   X2-X3-X4-X5-X6-X7-X8-X9-X10-X11-X12-X13-X14 (II)

   wherein
   X2, X3, X4, X5, X6, X7, X8, X9, X10, X11, X12, X13, and X14 are as defined in formula I; and
   wherein
   (i) the Glu at X11 forms a lactam bridge with the amino acid residue at X2; and
   (ii) X4 and X7 are amino acid residues who together form a lactam bridge or a dithioether bridge;
   or a pharmaceutically acceptable salt or solvate thereof.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula III:

   X1-[4-aminomethyl-phenylacetyl]-X3-X4-X5-X6-X7-X8-X9-X10-X11-X12-X13-X14 (III)

   wherein
   X1, X3, X4, X5, X6, X7, X8, X9, X10, X11, X12, X13, and X14 are as defined in formula I; and
   wherein
   (i) the Glu at X11 forms a lactam bridge with the amino acid residue at X1 or the 4-aminomethyl-phenylacetyl residue at X2 when X1 is absent; and
   (ii) X4 and X7 are amino acid residues who together form a lactam bridge or a dithioether bridge;
   or a pharmaceutically acceptable salt or solvate thereof.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula IV:

   X1-X2-[Ser]-X4-X5-X6-X7-X8-X9-X10-X11-X12-X13-X14 (IV)

   wherein
   X1, X2, X4, X5, X6, X7, X8, X9, X10, X11, X12, X13, and X14 are as defined in formula I; and
   wherein
   (i) the Glu at X11 forms a lactam bridge with the amino acid residue at X1 or at X2 when X1 is absent; and
   (ii) X4 and X7 are amino acid residues who together form a lactam bridge or a dithioether bridge;
   or a pharmaceutically acceptable salt or solvate thereof.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula V:

   X1-X2-X3-[Glu]-X5-X6-[Dab]-X8-X9-X10-X11-X12-X13-X14 (V)

   wherein
   X1, X2, X3, X5, X6, X8, X9, X10, X11, X12, X13, and X14 are as defined in formula I; and
   wherein
   (i) the Glu at X11 forms a lactam bridge with the amino acid residue at X1 or at X2 when X1 is absent; and
   (ii) the Glu at X4 and the Dab at X7 are amino acid residues who together form a lactam bridge;
   or a pharmaceutically acceptable salt or solvate thereof.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula VI:

   X1-X2-X3-X4-X5-[Gln]-X7-X8-X9-X10-X11-X12-X13-X14 (VI)

   wherein
   X1, X2, X3, X4, X5, X7, X8, X9, X10, X11, X12, X13, and X14 are as defined in formula I; and
   wherein
   (i) the Glu at X11 forms a lactam bridge with the amino acid residue at X1 or at X2 when X1 is absent; and
   (ii) X4 and X7 are amino acid residues who together form a lactam bridge or a dithioether bridge;
   or a pharmaceutically acceptable salt or solvate thereof.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula VII:

   X1-X2-X3-X4-X5-X6-X7-[Y(2-aminoethoxy)]-X9-X10-X11-X12-X13-X14 (VII)

   wherein
   X1, X2, X3, X4, X5, X6, X7, X9, X10, X11, X12, X13, and X14 are as defined in formula I; and
   wherein
   (i) the Glu at X11 forms a lactam bridge with the amino acid residue at X1 or at X2 when X1 is absent; and
   (ii) X4 and X7 are amino acid residues who together form a lactam bridge or a dithioether bridge;
   or a pharmaceutically acceptable salt or solvate thereof.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula VIII:

   X1-X2-X3-X4-X5-X6-X7-X8-[2-Nal]-X10-X11-X12-X13-X14 (VIII)

   wherein
   X1, X2, X3, X4, X5, X6, X7, X8, X10, X11, X12, X13, and X14 are as defined in formula I; and
   wherein
   (i) the Glu at X11 forms a lactam bridge with the amino acid residue at X1 or at X2 when X1 is absent; and
   (ii) X4 and X7 are amino acid residues who together form a lactam bridge or a dithioether bridge;
   or a pharmaceutically acceptable salt or solvate thereof.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula IX:

   X1-X2-X3-X4-X5-X6-X7-X8-X9-[2-Me-Leu]-X11-X12-X13-X14 (IX)

   wherein
   X1, X2, X3, X4, X5, X6, X7, X8, X9, X11, X12, X13, and X14 are as defined in formula I; and
   wherein
   (i) the Glu at X11 forms a lactam bridge with the amino acid residue at X1 or at X2 when X1 is absent; and
   (ii) X4 and X7 are amino acid residues who together form a lactam bridge or a dithioether bridge;
   or a pharmaceutically acceptable salt or solvate thereof.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula X:

   X1-X2-X3-X4-X5-X6-X7-X8-X9-X10-X11 -[Dab]-X13-X14 (X)

   wherein
   X1, X2, X3, X4, X5, X6, X7, X8, X9, X10, X11, X13, and X14 are as defined in formula I; and
   wherein
   (i) the Glu at X11 forms a lactam bridge with the amino acid residue at X1 or at X2 when X1 is absent; and
   (ii) X4 and X7 are amino acid residues who together form a lactam bridge or a dithioether bridge;
   or a pharmaceutically acceptable salt or solvate thereof.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula XI:

   X1-X2-X3-X4-X5-X6-X7-X8-X9-X10-X11-X12-[3-(3-Pyridyl)-Ala]-X14 (XI)

   wherein
   X1, X2, X3, X4, X5, X6, X7, X8, X9, X10, X11, X12, and X14 are as defined in formula I; and
   wherein
   (i) the Glu at X11 forms a lactam bridge with the amino acid residue at X1 or at X2 when X1 is absent; and
   (ii) X4 and X7 are amino acid residues who together form a lactam bridge or a dithioether bridge;
   or a pharmaceutically acceptable salt or solvate thereof.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula XII:

   X 1-X2-X3-X4-X5-X6-X7-X8-X9-X 10-X 11-X 12-X 13 (XII)

   wherein
   X1, X2, X3, X4, X5, X6, X7, X8, X9, X10, X11, X12, and X13 are as defined in formula I; and
   wherein
   (i) the Glu at X11 forms a lactam bridge with the amino acid residue at X1 or at X2 when X1 is absent; and
   (ii) X4 and X7 are amino acid residues who together form a lactam bridge or a dithioether bridge;
   or a pharmaceutically acceptable salt or solvate thereof.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula XIII:

   X2-X3-X4-X5-X6-X7-X8-X9-X10-X11 -X 12-X 13 (XIII)

   wherein
   X2, X3, X4, X5, X6, X7, X8, X9, X10, X11, X12, and X13 are as defined in formula I; and
   wherein
   (i) the Glu at X11 forms a lactam bridge with the amino acid residue at X2; and
   (ii) X4 and X7 are amino acid residues who together form a lactam bridge or a dithioether bridge;
   or a pharmaceutically acceptable salt or solvate thereof.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula XIV:

   [4-aminomethyl-phenylacetyl]-X3-X4-X5-X6-X7-X8-X9-X10-X11-X12-X13-X14 (XIV)

   wherein
   X3, X4, X5, X6, X7, X8, X9, X10, X11, X12, X13, and X14 are as defined in formula I; and
   wherein
   (i) the Glu at X11 forms a lactam bridge with the 4-aminomethyl-phenylacetyl residue at X2; and
   (ii) X4 and X7 are amino acid residues who together form a lactam bridge or a dithioether bridge;
   or a pharmaceutically acceptable salt or solvate thereof.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula XV:

   [4-aminomethyl-phenylacetyl]-X3-X4-X5-X6-X7-[Y(2-aminoethoxy)]-[2-Nal]-[2-Me-Leu]-X11-X12-X13-X14 (XV)

   wherein
   X3, X4, X5, X6, X7, X11, X12, X13, and X14 are as defined in formula I; and
   wherein
   (i) the Glu at X11 forms a lactam bridge with the 4-aminomethyl-phenylacetyl residue at X2; and
   (ii) X4 and X7 are amino acid residues who together form a lactam bridge or a dithioether bridge;
   or a pharmaceutically acceptable salt or solvate thereof.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula XVI:

   [4-aminomethyl-phenylacetyl]-X3-X4-X5-X6-X7-[Y(2-aminoethoxy)]-[2-Nal]-[2-Me-Leu]-X11-X12-X13-X14 (XVI)

   wherein
   X3 is selected from the group consisting of Ser or Ala; and
   X4, X5, X6, X7, X11, X12, X13, and X14 are as defined in formula I;
   wherein
   (i) the Glu at X11 forms a lactam bridge with the 4-aminomethyl-phenylacetyl residue at X2; and
   (ii) X4 and X7 are amino acid residues who together form a lactam bridge or a dithioether bridge;
   or a pharmaceutically acceptable salt or solvate thereof.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula XVII:

   [4-aminomethyl-phenylacetyl]-X3-X4-X5-[Gln]-X7-[Y(2-aminoethoxy)]-[2-Nal]-[2-Me-Leu]-X11-X12-X13-X14 (XVII)

   wherein
   X3 is selected from the group consisting of Ser or Ala; and
   X4, X5, X7, X11, X12, X13, and X14 are as defined in formula I;
   wherein
   (i) the Glu at X11 forms a lactam bridge with the 4-aminomethyl-phenylacetyl residue at X2; and
   (ii) X4 and X7 are amino acid residues who together form a lactam bridge or a dithioether bridge;
   or a pharmaceutically acceptable salt or solvate thereof.

In some embodiments, the invention provides a compound of the formula:

Z-R²

wherein
R² is NHR³ wherein R³ is hydrogen or C₁₋₄ alkyl optionally substituted with a pyridyl ring; and
Z is an amino acid sequence of formula XVIII:

   X1-X2-X3-X4-X5-X6-X7-X8-X9-X10-X11-X12-[3-(3-Pyridyl)-Ala] (XVIII)

   wherein
   X1, X2, X3, X4, X5, X6, X7, X8, X9, X10, X11, and X12 are as defined in formula I; and
   wherein
   (i) the Glu at X11 forms a lactam bridge with the amino acid residue at X1 or at X2 when X1 is absent; and
   (ii) X4 and X7 are amino acid residues who together form a lactam bridge or a dithioether bridge;
   or a pharmaceutically acceptable salt or solvate thereof.

In some embodiments, the invention provides a compound of the formula:

Z-NH₂ or Z-NHMe

wherein
Z is an amino acid sequence of any one of the formulae I-XVIII as defined above;
or a pharmaceutically acceptable salt or solvate thereof.

In some embodiments, the invention provides a compound of the formula:

Z-NHMe

wherein
Z is an amino acid sequence of formula XVIII:

X1-X2-X3-X4-X5-X6-X7-X8-X9-X10-X11-X12-[3-(3-Pyridyl)-Ala] (XVIII)

wherein
X1, X2, X3, X4, X5, X6, X7, X8, X9, X10, X11, and X12 are as defined in formula I; and
wherein
(i) the Glu at X11 forms a lactam bridge with the amino acid residue at X1 or at X2 when X1 is absent; and
(ii) X4 and X7 are amino acid residues who together form a lactam bridge or a dithioether bridge;
or a pharmaceutically acceptable salt or solvate thereof.

In some embodiments, the invention provides a compound selected from a compound in **Table 1-1**, or a pharmaceutically acceptable salt or solvate thereof.

Any internal truncation, that is deletion of amino acid residues, between X2 and X11 leads to inactive compounds (see reference compounds Ref 5, Ref 6, and Ref 7 in the Examples of PCT/EP2022/084077).

It will be understood that the invention encompasses salts and solvates of the compounds. Suitable salts and solvates of peptides are known in the art.

It will also be understood any of the following references to embodiments may be applicable and are combinable with any of the formulae described herein.

### R²

R² is NHR³ wherein R³ is hydrogen or C₁₋₄ alkyl optionally substituted with a pyridyl ring.

In some embodiments, R² is NHR³ wherein R³ is hydrogen or C₁₋₄ alkyl optionally substituted with a pyrid-3-yl ring.

In some embodiments, R² is NHR³ wherein R³ is hydrogen or C₁₋₄ alkyl. In some embodiments, R² is NHR³ wherein R³ is hydrogen or C₁₋₃ alkyl. In some embodiments, R² is NHR³ wherein R³ is hydrogen or C₁₋₂ alkyl. In some embodiments, R² is NHR³ wherein R³ is hydrogen or C₁ alkyl (methyl or Me).

In some embodiments, R² is NHR³ wherein R³ is C₁₋₄ alkyl optionally substituted with a pyridyl ring. In some embodiments, R² is NHR³ wherein R³ is C₁₋₄ alkyl substituted with a pyridyl ring.

In some embodiments, R² is NHR³ wherein R³ is C₁₋₄ alkyl optionally substituted with a pyrid-3-yl ring. In some embodiments, R² is NHR³ wherein R³ is C₁₋₄ alkyl substituted with a pyrid-3-yl ring.

In some embodiments, R² is NHR³ wherein R³ is C₄ alkyl substituted with a pyridyl ring. In some embodiments, R² is NHR³ wherein R³ is C₄ alkyl substituted with a pyrid-3-yl ring.
In some embodiments, R² is NHR³ wherein R³ is n-Bu substituted with a pyrid-3-yl ring. In some embodiments, R² is NHR³ wherein R³ is -CH₂CH₂CH₂CH₂(pyrid-3-yl). That is, NHCH₂CH₂CH₂CH₂(pyrid-3-yl) or NH-(4-(pyridin-3-yl)butanyl). The group NH-(4-(pyridin-3-yl)butanyl) has the structure:

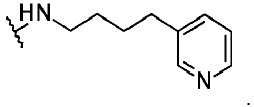

In some embodiments, R² is NHR³ wherein R³ is C₂ alkyl substituted with a pyridyl ring. In some embodiments, R² is NHR³ wherein R³ is C₂ alkyl substituted with a pyrid-3-yl ring. In some embodiments, R² is NHR³ wherein R³ is ethyl (Et) substituted with a pyrid-3-yl ring. In some embodiments, R² is NHR³ wherein R³ is -CH₂CH₂(pyrid-3-yl). That is, NHCH₂CH₂(pyrid-3-yl) or NH-(2-(pyridin-3-yl)ethyl). The group NH-(2-(pyridin-3-yl)ethyl) has the structure:

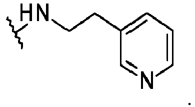

Preferably in embodiments where R² is NHR³ wherein R³ is C₁₋₄ alkyl substituted with a pyridyl ring, such as NH-(4-(pyridin-3-yl)butanyl) and NH-(2-(pyridin-3-yl)ethyl), both X13 and X14 are absent.

In some embodiments, R² is NHMe, NH₂, NHCH₂CH₂CH₂CH₂(pyrid-3-yl) [that is, NH-(4-(pyridin-3-yl)butanyl)], or NHCH₂CH₂(pyrid-3-yl) [that is, NH-(2-(pyridin-3-yl)ethyl)]..

In some embodiments, R² is NHMe. In some embodiments, R² is NH₂. In some embodiments, R² is NHCH₂CH₂CH₂CH₂(pyrid-3-yl), that is NH-(4-(pyridin-3-yl)butanyl). In some embodiments, R² is NHCH₂CH₂(pyrid-3-yl), that is NH-(2-(pyridin-3-yl)ethyl).

Preferably, R² is NHMe or NH₂. Even more preferably, R² is NHMe.

In particular, R² is NHMe is preferable because the modification improves SIF stability (see **Example 2**).

### Z

Z is an amino acid sequence of formula I:

X1-X2-X3-X4-X5-X6-X7-X8-X9-X10-X11-X12-X13-X14 (I)

wherein
X1 is absent or is selected from 3-(3-Pyridyl)-Ala or 3-aminopropanoyl;
X2 is selected from the group consisting of 4-aminomethyl-phenylacetyl, 3-(3-Pyridyl)-Ala, and 3-aminopropanoyl;
X3 is selected from the group consisting of Ser, Ala, Phe, and N-Me-Ser;
X4 is Glu or Cys;
X5 is Trp;
X6 is selected from the group consisting of Gln, Q(Me), Q(2Me), Q(pyrrolidin), Ala, Phe, Lys(Ac), Dab(Ac), Cit, and Orn;
X7 is Dab or Cys;
X8 is selected from the group consisting of Y(2-aminoethoxy), homo-Phe, 7-AzaTrp, beta-homo-Trp, and 7-F-Trp;
X9 is selected from the group consisting of 2-Nal, 7-AzaTrp, beta-homo-Trp, and Cyclopropyl-Ala;
X10 is 2-Me-Leu or Aib;
X11 is Glu;
X12 is selected from the group consisting of Dab, iso-Dab, D-Arg, Gly-CF3, D-Gly-CF3, Nle, Gln, His, THP, Ser, D-Ser, 2-Me-Ser, Ser(OMe), and homo-Ser;
X13 is selected from the group consisting of 3-(3-Pyridyl)-Ala, D-3-(3-Pyridyl)-Ala, 2-Me-3-(3-Pyridyl)-Ala, N-Me-3-(3-Pyridyl)-Ala, 3-(3,5-Pyrimidyl)-Ala, His, D-His, and His(Me), or is absent; and
X14 is absent or is selected from the group consisting of Sar, D-His, beta-homo-Leu, and 3-(3-Pyridyl)-Ala;
wherein
(i) the Glu at X11 forms a lactam bridge with the amino acid residue at X1 or at X2 when X1 is absent; and
(ii) X4 and X7 are amino acid residues who together form a lactam bridge or a dithioether bridge.

In some embodiments, Z is an amino acid sequence of formula II:

X2-X3-X4-X5-X6-X7-X8-X9-X10-X11-X12-X13-X14 (II)

wherein
X2, X3, X4, X5, X6, X7, X8, X9, X10, X11, X12, X13, and X14 are as defined in formula I; and
wherein
(i) the Glu at X11 forms a lactam bridge with the amino acid residue at X2; and
(ii) X4 and X7 are amino acid residues who together form a lactam bridge or a dithioether bridge.

In some embodiments, Z is an amino acid sequence of formula III:

X1-[4-aminomethyl-phenylacetyl]-X3-X4-X5-X6-X7-X8-X9-X10-X11-X12-X13-X14 (III)

wherein
X1, X3, X4, X5, X6, X7, X8, X9, X10, X11, X12, X13, and X14 are as defined in formula I; and
wherein
(i) the Glu at X11 forms a lactam bridge with the amino acid residue at X1 or the 4-aminomethyl-phenylacetyl residue at X2 when X1 is absent; and
(ii) X4 and X7 are amino acid residues who together form a lactam bridge or a dithioether bridge.

In some embodiments, Z is an amino acid sequence of formula IV:

X1-X2-[Ser]-X4-X5-X6-X7-X8-X9-X10-X11-X12-X13-X14 (IV)

wherein
X1, X2, X4, X5, X6, X7, X8, X9, X10, X11, X12, X13, and X14 are as defined in formula I; and
wherein
(i) the Glu at X11 forms a lactam bridge with the amino acid residue at X1 or at X2 when X1 is absent; and
(ii) X4 and X7 are amino acid residues who together form a lactam bridge or a dithioether bridge.

In some embodiments, Z is an amino acid sequence of formula V:

X1-X2-X3-[Glu]-X5-X6-[Dab]-X8-X9-X10-X11-X12-X13-X14 (V)

wherein
X1, X2, X3, X5, X6, X8, X9, X10, X11, X12, X13, and X14 are as defined in formula I; and
wherein
(i) the Glu at X11 forms a lactam bridge with the amino acid residue at X1 or at X2 when X1 is absent; and
(ii) the Glu at X4 and the Dab at X7 are amino acid residues who together form a lactam bridge.

In some embodiments, Z is an amino acid sequence of formula VI:

X1-X2-X3-X4-X5-[Gln]-X7-X8-X9-X10-X11-X12-X13-X14 (VI)

wherein
X1, X2, X3, X4, X5, X7, X8, X9, X10, X11, X12, X13, and X14 are as defined in formula I; and
wherein
(i) the Glu at X11 forms a lactam bridge with the amino acid residue at X1 or at X2 when X1 is absent; and
(ii) X4 and X7 are amino acid residues who together form a lactam bridge or a dithioether bridge.

In some embodiments, Z is an amino acid sequence of formula VII:

X1-X2-X3-X4-X5-X6-X7-[Y(2-aminoethoxy)]-X9-X10-X11-X12-X13-X14 (VII)

wherein
X1, X2, X3, X4, X5, X6, X7, X9, X10, X11, X12, X13, and X14 are as defined in formula I; and
wherein
(i) the Glu at X11 forms a lactam bridge with the amino acid residue at X1 or at X2 when X1 is absent; and
(ii) X4 and X7 are amino acid residues who together form a lactam bridge or a dithioether bridge.

In some embodiments, Z is an amino acid sequence of formula VIII:

X1-X2-X3-X4-X5-X6-X7-X8-[2-Nal]-X10-X11-X12-X13-X14 (VIII)

wherein
X1, X2, X3, X4, X5, X6, X7, X8, X10, X11, X12, X13, and X14 are as defined in formula I; and
wherein
(i) the Glu at X11 forms a lactam bridge with the amino acid residue at X1 or at X2 when X1 is absent; and
(ii) X4 and X7 are amino acid residues who together form a lactam bridge or a dithioether bridge.

In some embodiments, Z is an amino acid sequence of formula IX:

X1-X2-X3-X4-X5-X6-X7-X8-X9-[2-Me-Leu]-X11-X12-X13-X14 (IX)

wherein
X1, X2, X3, X4, X5, X6, X7, X8, X9, X11, X12, X13, and X14 are as defined in formula I; and
wherein
(i) the Glu at X11 forms a lactam bridge with the amino acid residue at X1 or at X2 when X1 is absent; and
(ii) X4 and X7 are amino acid residues who together form a lactam bridge or a dithioether bridge.

In some embodiments, Z is an amino acid sequence of formula X:

X1-X2-X3-X4-X5-X6-X7-X8-X9-X10-X11 -[Dab]-X13-X14 (X)

wherein
X1, X2, X3, X4, X5, X6, X7, X8, X9, X10, X11, X13, and X14 are as defined in formula I; and
wherein
(i) the Glu at X11 forms a lactam bridge with the amino acid residue at X1 or at X2 when X1 is absent; and
(ii) X4 and X7 are amino acid residues who together form a lactam bridge or a dithioether bridge.

In some embodiments, Z is an amino acid sequence of formula XI:

X1-X2-X3-X4-X5-X6-X7-X8-X9-X10-X11-X12-[3-(3-Pyridyl)-Ala]-X14 (XI)

wherein
X1, X2, X3, X4, X5, X6, X7, X8, X9, X10, X11, X12, and X14 are as defined in formula I; and
wherein
(i) the Glu at X11 forms a lactam bridge with the amino acid residue at X1 or at X2 when X1 is absent; and
(ii) X4 and X7 are amino acid residues who together form a lactam bridge or a dithioether bridge.

In some embodiments, Z is an amino acid sequence of formula XII:

X 1-X2-X3-X4-X5-X6-X7-X8-X9-X 10-X 11-X 12-X 13 (XII)

wherein
X1, X2, X3, X4, X5, X6, X7, X8, X9, X10, X11, X12, and X13 are as defined in formula I; and
wherein
(i) the Glu at X11 forms a lactam bridge with the amino acid residue at X1 or at X2 when X1 is absent; and
(ii) X4 and X7 are amino acid residues who together form a lactam bridge or a dithioether bridge.

In some embodiments, Z is an amino acid sequence of formula XIII:

X2-X3-X4-X5-X6-X7-X8-X9-X10-X11 -X 12-X 13 (XIII)

wherein
X2, X3, X4, X5, X6, X7, X8, X9, X10, X11, X12, and X13 are as defined in formula I; and
wherein
(i) the Glu at X11 forms a lactam bridge with the amino acid residue at X2; and
(ii) X4 and X7 are amino acid residues who together form a lactam bridge or a dithioether bridge.

In some embodiments, Z is an amino acid sequence of formula XIV:

[4-aminomethyl-phenylacetyl]-X3-X4-X5-X6-X7-X8-X9-X10-X11-X12-X13-X14 (XIV)

wherein
X3, X4, X5, X6, X7, X8, X9, X10, X11, X12, X13, and X14 are as defined in formula I; and
wherein
(i) the Glu at X11 forms a lactam bridge with the 4-aminomethyl-phenylacetyl residue at X2; and
(ii) X4 and X7 are amino acid residues who together form a lactam bridge or a dithioether bridge.

In some embodiments, Z is an amino acid sequence of formula XV:

[4-aminomethyl-phenylacetyl]-X3-X4-X5-X6-X7-[Y(2-aminoethoxy)]-[2-Nal]-[2-Me-Leu]-X11-X12-X13-X14 (XV)

wherein
X3, X4, X5, X6, X7, X11, X12, X13, and X14 are as defined in formula I; and
wherein
(i) the Glu at X11 forms a lactam bridge with the 4-aminomethyl-phenylacetyl residue at X2; and
(ii) X4 and X7 are amino acid residues who together form a lactam bridge or a dithioether bridge.

In some embodiments, Z is an amino acid sequence of formula XVI:

[4-aminomethyl-phenylacetyl]-X3-X4-X5-X6-X7-[Y(2-aminoethoxy)]-[2-Nal]-[2-Me-Leu]-X11-X12-X13-X14 (XVI)

wherein
X3 is selected from the group consisting of Ser or Ala; and
X4, X5, X6, X7, X11, X12, X13, and X14 are as defined in formula I;
wherein
(i) the Glu at X11 forms a lactam bridge with the 4-aminomethyl-phenylacetyl residue at X2; and
(ii) X4 and X7 are amino acid residues who together form a lactam bridge or a dithioether bridge.

In some embodiments, Z is an amino acid sequence of formula XVII:

[4-aminomethyl-phenylacetyl]-X3-X4-X5-[Gln]-X7-[Y(2-aminoethoxy)]-[2-Nal]-[2-Me-Leu]-X11-X12-X13-X14 (XVII)

wherein
X3 is selected from the group consisting of Ser or Ala; and
X4, X5, X7, X11, X12, X13, and X14 are as defined in formula I;
wherein
(i) the Glu at X11 forms a lactam bridge with the 4-aminomethyl-phenylacetyl residue at X2; and
(ii) X4 and X7 are amino acid residues who together form a lactam bridge or a dithioether bridge.

In some embodiments, Z is an amino acid sequence of formula XVIII:

X1-X2-X3-X4-X5-X6-X7-X8-X9-X10-X11-X12-[3-(3-Pyridyl)-Ala] (XVIII)

wherein
X1, X2, X3, X4, X5, X6, X7, X8, X9, X10, X11, and X12 are as defined in formula I; and
wherein
(i) the Glu at X11 forms a lactam bridge with the amino acid residue at X1 or at X2 when X1 is absent; and
(ii) X4 and X7 are amino acid residues who together form a lactam bridge or a dithioether bridge.

In some embodiments, Z is an amino acid sequence selected from the group consisting of a sequence listed in **Table 1-1a**.

### X1

X1 is absent or is selected from the group consisting of 3-(3-Pyridyl)-Ala or 3-aminopropanoyl.

In some embodiments, X1 is absent. When X1 is absent, the amino acid residue at X2 forms a lactam bridge with the Glu residue at X11.

In some embodiments, X1 is 3-(3-Pyridyl)-Ala or 3-aminopropanoyl. In some embodiments, X1 is 3-(3-Pyridyl)-Ala. In some embodiments, X1 is 3-aminopropanoyl.

When X1 is present, that is when X1 is 3-(3-Pyridyl)-Ala or 3-aminopropanoyl, X1 may form a lactam bridge with the Gly residue at X11. In some embodiments when X1 is present, that is when X1 is 3-(3-Pyridyl)-Ala or 3-aminopropanoyl, X1 forms a lactam bridge with the Glu residue at X11.

Preferably, X1 is absent.

### X2

X2 is selected from the group consisting of 4-aminomethyl-phenylacetyl, 3-(3-Pyridyl)-Ala, and 3-aminopropanoyl.

The amino acid residue at X2 may form a lactam bridge with the Glu residue at X11. When X1 is absent, the amino acid residue at X2 forms a lactam bridge with the Glu residue at X11.

In some embodiments, X2 is 4-aminomethyl-phenylacetyl. In some embodiments, X2 is 3-(3-Pyridyl)-Ala. In some embodiments, X2 is 3-aminopropanoyl.

Preferably, X2 is 4-aminomethyl-phenylacetyl.

### X3

X3 is selected from the group consisting of Ser, Ala, Phe, and N-Me-Ser.

In some embodiments, X3 is Ser or Ala.

In some embodiments, X3 is Ser. In some embodiments, X3 is Ala. In some embodiments, X3 is Phe. In some embodiments, X3 is N-Me-Ser.

Preferably, X3 is Ser.

### X4

X4 is Glu or Cys. The amino acid residue at X4 forms a lactam bridge or a dithioether bridge with the amino acid residue at X7.

In some embodiments, X4 is Glu. When X4 is Glu, the Glu at X4 forms a lactam bridge with the amino acid residue at X7.

In some embodiments, X4 is Cys. When X4 is Cys, the Cys at X4 forms a dithioether bridge with the amino acid residue at X7.

Preferably, X4 is Glu.

### X5

X5 is Trp.

### X6

X6 is selected from the group consisting of Gln, Q(Me), Q(2Me), Q(pyrrolidin), Ala, Phe, Lys(Ac), Dab(Ac), Cit, and Orn.

In some embodiments, X6 is Gln. In some embodiments, X6 is Q(Me). In some embodiments, X6 is Q(2Me). In some embodiments, X6 is Q(pyrrolidin). In some embodiments, X6 is Ala. In some embodiments, X6 is Phe. In some embodiments, X6 is Lys(Ac). In some embodiments, X6 is Dab(Ac). In some embodiments, X6 is Cit. In some embodiments, X6 is Orn.

Preferably, X6 is Gln.

### X7

X7 is Dab or Cys. The amino acid residue at X7 forms a lactam bridge or a dithioether bridge with the amino acid residue at X4.

In some embodiments, X7 is Dab. When X7 is Dab, the Dab at X7 forms a lactam bridge with the amino acid residue at X4.

In some embodiments, X7 is Cys. When X7 is Cys, the Cys at X7 forms a dithioether bridge with the amino acid residue at X4.

Preferably, X7 is Dab.

### X8

X8 is selected from the group consisting of Y(2-aminoethoxy), homo-Phe, 7-AzaTrp, beta-homo-Trp, and 7-F-Trp.

In some embodiments, X8 is Y(2-aminoethoxy). In some embodiments, X8 is homo-Phe. In some embodiments, X8 is 7-AzaTrp. In some embodiments, X8 is beta-homo-Trp. In some embodiments, X8 is 7-F-Trp.

Preferably, X8 is Y(2-aminoethoxy).

### X9

X9 is selected from the group consisting of 2-Nal, 7-AzaTrp, beta-homo-Trp, and Cyclopropyl-Ala.

In some embodiments, X9 is 2-Nal. In some embodiments, X9 is 7-AzaTrp. In some embodiments, X9 is beta-homo-Trp. In some embodiments, X9 is Cyclopropyl-Ala.

Preferably, X9 is 2-Nal.

### X10

X10 is selected from the group consisting of 2-Me-Leu or Aib.

In some embodiments, X10 is 2-Me-Leu. In some embodiments, X10 is Aib.

Preferably, X10 is 2-Me-Leu.

The inventors have observed that having an amino acid addressing SIF stability (that is, gastrointestinal stability) at position 10 (X10) is necessary. The amino acids 2-Me-Leu and Aib, particularly 2-Me-Leu, address SIF stability. If, for example, the amino acid residue is Gly in position 10 (X10), the inventors observed that the SIF stability of the compound is significantly decreased.

### X11

X11 is Glu.

The Glu residue at X11 forms a lactam bridge with the amino acid residue at either X1 or X2 when X1 is absent. In some embodiments, the Glu residue at X11 forms a lactam bridge with the amino acid residue at X1. In some embodiments, the Glu residue at X11 forms a lactam bridge with the amino acid residue at X2.

When X1 is absent, the Glu residue at X11 forms a lactam bridge with the amino acid residue at X2.

### X12

X12 is selected from the group consisting of Dab, iso-Dab, D-Arg, Gly-CF3, D-Gly-CF3, Nle, Gln, His, THP, Ser, D-Ser, 2-Me-Ser, Ser(OMe), and homo-Ser.

In some embodiments, X12 is selected from the group consisting of Dab, D-Arg, and Ser. In some embodiments, X12 is Dab or Ser. In some embodiments, X12 is Dab or D-Arg.

In some embodiments, X12 is Dab. In some embodiments, X12 is iso-Dab. In some embodiments, X12 is D-Arg. In some embodiments, X12 is Gly-CF3. In some embodiments, X12 is D-Gly-CF3. In some embodiments, X12 is Nle. In some embodiments, X12 is Gln. In some embodiments, X12 is His. In some embodiments, X12 is THP. In some embodiments, X12 is Ser. In some embodiments, X12 is D-Ser. In some embodiments, X12 is 2-Me-Ser. In some embodiments, X12 is Ser(OMe). In some embodiments, X12 is homo-Ser.

Preferably, X12 is Dab.

### X13

X13 is selected from the group consisting of 3-(3-Pyridyl)-Ala, D-3-(3-Pyridyl)-Ala, 2-Me-3-(3-Pyridyl)-Ala, N-Me-3-(3-Pyridyl)-Ala, 3-(3,5-Pyrimidyl)-Ala, His, D-His, and His(Me), or is absent.

In some embodiments, X13 is 3-(3-Pyridyl)-Ala or is absent. When X13 is absent, X14 is also absent.

In some embodiments, X13 is 3-(3-Pyridyl)-Ala. In some embodiments, X13 is D-3-(3-Pyridyl)-Ala. In some embodiments, X13 is 2-Me-3-(3-Pyridyl)-Ala. In some embodiments, X13 is N-Me-3-(3-Pyridyl)-Ala. In some embodiments, X13 is 3-(3,5-Pyrimidyl)-Ala. In some embodiments, X13 is His. In some embodiments, X13 is D-His. In some embodiments, X13 is His(Me). In some embodiments, X13 is absent.

Preferably, X13 is 3-(3-Pyridyl)-Ala or is absent. Even more preferably, X13 is 3-(3-Pyridyl)-Ala.

### X14

X14 is absent or is selected from the group consisting of Sar, D-His, beta-homo-Leu, and 3-(3-Pyridyl)-Ala.

In some embodiments, X14 is absent. In such embodiments where X14 is absent, X13 may preferably be 3-(3-Pyridyl)-Ala.

In some embodiments, X14 is Sar. In some embodiments, X14 is D-His. In some embodiments, X14 is beta-homo-Leu. In some embodiments, X14 is 3-(3-Pyridyl)-Ala. When X14 is present, that is when X14 is selected from the group consisting of Sar, D-His, beta-homo-Leu, and 3-(3-Pyridyl)-Ala; X13 may be 3-(3-Pyridyl)-Ala or His.

Preferably, X14 is absent.

### Lactam bridge

A lactam bridge is formed of one amino acid residue comprising an amine group and another amino acid residue comprising a carboxylic acid group. The amine and/or carboxylic acid group of the amino acid residue may be on the side chain of the amino acid residue, such as Dab and Glu. Alternatively, the amine and/or carboxylic acid group of the amino acid residue may be the /V- or C-terminus of the peptide chain, such as the amine or carboxylic acid of the peptide backbone of any amino acid, such as 4-aminomethyl-phenylacetyl, 3-(3-Pyridyl)-Ala, and 3-aminopropanoyl.

One of the amino acid residues comprise an amine group and the other amino acid residue comprises a carboxylic acid group, wherein a lactam (cyclic amide) is formed between the amine and carboxylic acid groups.

For simplicity, the amino acid residues who together form a lactam bridge will be discussed by reference to the residues nominally present before lactam formation.

Suitable amino acid residues who together form a lactam bridge may be selected from:
Amino acid residues comprising an amine group: 4-aminomethyl-phenylacetyl, 3-(3-Pyridyl)-Ala, 3-aminopropanoyl, and Dab.
Amino acid residues comprising a carboxylic acid group: Glu.

The inventors have found that replacing a dithioether bridge with a lactam bridge can lead to increased potency of the IL-23R peptide inhibitor, as previously described in Example 2 of PCT/EP2022/084077.

### First lactam bridge - X1/X2 and X11

A first lactam bridge is formed between the Glu at X11 with the amino acid residue at X1 or at X2 when X1 is absent. In some embodiments, the first lactam bridge is formed between the Glu at X11 with the amino acid residue at X1. In some embodiments, the first lactam bridge is formed between the Glu at X11 with the amino acid residue at X2. When X1 is absent, the first lactam bridge is formed between the Glu at X11 with the amino acid residue at X2. Preferably, the first lactam bridge is formed between the Glu at X11 with the amino acid residue at X2.

In embodiments where the first lactam bridge is formed between the Glu at X11 with the amino acid residue at X1, X1 is 3-(3-Pyridyl)-Ala or 3-aminopropanoyl. That is, the following combinations of X1 and X11 form a lactam bridge:
X1 is 3-(3-Pyridyl)-Ala and X11 is Glu; and
X1 is 3-aminopropanoyl and X11 is Glu.

In embodiments where the first lactam bridge is formed between the Glu at X11 with the amino acid residue at X2, X2 is 4-aminomethyl-phenylacetyl, 3-(3-Pyridyl)-Ala, and 3-aminopropanoyl. That is, the following combinations of X2 and X11 form a lactam bridge:
X2 is 4-aminomethyl-phenylacetyl and X11 is Glu;
X2 is 3-(3-Pyridyl)-Ala and X11 is Glu; and
X2 is 3-aminopropanoyl and X11 is Glu.
In such combinations, X1 is absent.

### Second lactam bridge - X4 and X7

A second lactam bridge may be formed between the amino acid residues at X4 and X7. In such embodiments, X4 is Glu and X7 is Dab.

Preferably, the bridge between the amino acid residues at X4 and X7 is a lactam bridge.

### Dithioether bridge

A dithioether bridge is formed of two amino acid residues comprising sulfur moieties, such as -SH. Preferably, the sulfur moiety of the amino acid residue is on the side chain of the amino acid residue, such as Cys.

For simplicity, the amino acid residues who together form a dithioether bridge will be discussed by reference to the residues nominally present before dithioether formation.

Suitable amino acid residues who together form a dithioether bridge may be Cys.

The inventors have found that a linker is required between the thiol side chains of linked [4,7] cysteine residues in the compounds to retain IL-23R inhibitory activity. Replacing the dithioether bridges with disulfide bridges resulted in loss of inhibitory activity (see Example 2 of PCT/EP2022/084077).

### Dithioether formula

In some embodiments, the dithioether bridge is of the formula -S-L-Y-L-S-, wherein:
each S is a sulfur atom and is part of one of the amino acid residues forming the dithioether bridge;
each L is independently C₁₋₄ alkylene; and
Y is either absent or C(=O).

### L

Each L is independently C₁₋₄ alkylene.

In some embodiments, L is C₁₋₂ alkylene. In some embodiments, L is C₁ alkylene (methylene or -CH₂-). In some embodiments, L is C₂ alkylene (ethylene or -CH₂CH₂-).

### Y

Y is either absent or C(=O).

In some embodiments, Y is absent. In some embodiments, Y is C(=O). Preferably, Y is C(=O).

### Preferred dithioether bridges

In some embodiments, the dithioether bridge is of the formula -SCH₂C(=O)CH₂S-, wherein each S is a sulfur atom and is part of one of the amino acid residues forming the dithioether bridge.

### Dithioether bridge - X4 and X7

A dithioether bridge may be formed between the amino acid residues at X4 and X7. In such embodiments, X4 is Cys and X7 is Cys.

In embodiments where a dithioether bridge is formed between the amino acid residues at X4 and X7, preferably the dithioether bridge between X4 and X7 is of the formula -S-L-Y-L-S-, wherein:
each S is a sulfur atom and is part of the amino acid residue at X4 and X7;
each L is independently C₁₋₄ alkylene; and
Y is either absent or C(=O).

In embodiments where a dithioether bridge is formed between the amino acid residues at X4 and X7, even more preferably the bridge is of the formula -SCH₂C(=O)CH₂S-, wherein each S is a sulfur atom and is part of the amino acid residue at X4 and X7. That is, each L is -CH₂- and Y is C(=O).

### Bridge length

The length of the bridge is counted as the number of atoms in a linear chain from the first atom attached to the atom (carbon) adjacent to the carboxylic acid moiety of the amino acid of the first residue (X1/X2 for the bridge between X1/X2 and X11; or X4 for the bridge between X4 and X7), i.e. attached to the alpha carbon of the relevant residue for most amino acids, up to the first atom attached to the atom (carbon) adjacent to the carboxylic acid moiety of the amino acid of the second residue (X11 for the bridge between X1/X2 and X11; or X7 for the bridge between X4 and X7).

The contribution to the length of the bridge for amino acid residues and the type of bridge is described below.

In some embodiments, the length of the bridge between X1/X2 and X11 is at least 4 atoms long. In some embodiments, the length of the bridge between X1/X2 and X11 is no longer than 10 atoms long. In some embodiments, the length of the bridge between X1/X2 and X11 is 4 to 10 atoms long, such as 4, 5, 6, 7, 8, 9, or 10 atoms long. In some embodiments, the length of the bridge between X1/X2 and X11 is 4 to 9 atoms long, such as 4, 5, 6, 7, 8, or 9 atoms long. In some embodiments, the length of the bridge between X1/X2 and X11 is either 4, 5, or 9 atoms long. In some embodiments, the length of the bridge between X1/X2 and X11 is 4 atoms long. In some embodiments, the length of the bridge between X1/X2 and X11 is either 5 atoms long. In some embodiments, the length of the bridge between X1/X2 and X11 is 9 atoms long.

In some embodiments, the length of the bridge between X4 and X7 is at least 4 atoms long. In some embodiments, the length of the bridge between X4 and X7 is no longer than 10 atoms long. In some embodiments, the length of the bridge between X4 and X7 is 4 to 10 atoms long, such as 4, 5, 6, 7, 8, 9, or 10 atoms long. In some embodiments, the length of the bridge between X4 and X7 is 4 to 9 atoms long, such as 4, 5, 6, 7, 8, or 9 atoms long. In some embodiments, the length of the bridge between X4 and X7 is 6 or 7 atoms long. In some embodiments, the length of the bridge between X4 and X7 is 6 atoms long. In some embodiments, the length of the bridge between X4 and X7 is 7 atoms long.

### Lactam bridge

The contribution of the side chain to the length of the lactam bridge is counted as the number of atoms in a linear chain from the first atom of the side chain (which is bonded to an atom of the peptide backbone, i.e. to the alpha carbon of the relevant residue for most amino acids) up to and including the atom which participates in the amide bond of the lactam bridge (i.e. the carbon atom of the carboxylic acid functional group or the nitrogen atom of the amine group).

Thus common acid- and amine-containing side chains are considered to have the following side chain lengths:

### Amine-containing side chains:

| | | |
|---|---|---|
| Dab / iso-Dab | 3 atoms | 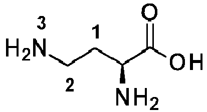 |

### Carboxylic acid-containing side chains:

| | | |
|---|---|---|
| Glu | 3 atoms | 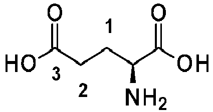 |

Similarly, the contribution of the length of the /V- or C-terminus amino acid residue to the length of the lactam bridge is counted as the number of atoms in a linear chain from the first atom attached to the atom (carbon) adjacent to the carboxylic acid moiety of the amino acid residue (i.e. the first atom attached to the alpha carbon of the relevant residue for most amino acids), up to and including the atoms which participate in the amide bond of the lactam bridge (i.e. the carbon atom of the carboxylic acid functional group or the nitrogen atom of the amine group).

Thus the following amino acid residues are considered to have the following lengths:

### N-terminus:

| | | |
|---|---|---|
| 3-(3-Pyridyl)-Ala | 1 atom | 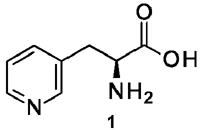 |
| 3-aminopropanoyl | 2 atoms | 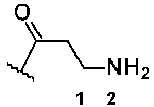 |
| 4-aminomethyl-phenylacetyl | 6 atoms | 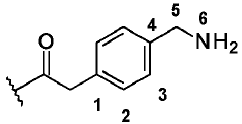 |

The location of the amide bond in the lactam bridge may affect the potency of the compound. The inventors observed that the compound was more active when the amide bond was closer to position 11 (X11) and less active when closer to position 2 (X2) (see Example 2 and Table 2-3b of PCT/EP2022/084077).

Suitable pairings of residues at positions X1/X2 and X11 in which the location of the amide bond in the lactam bridge is closer to position X11 than position X1/X2 once formed include:
X2 is 4-aminomethyl-phenylacetyl and X11 is Glu.

Alternatively, suitable pairings of residues at positions X1/X2 and X11 in which location of the amide bond in the lactam bridge is closer to position X1/X2 than position X11 include:
X1 is 3-(3-Pyridyl)-Ala and X11 is Glu;
X1 is 3-aminopropanoyl and X11 is Glu;
X2 is 3-(3-Pyridyl)-Ala and X11 is Glu; and
X2 is 3-aminopropanoyl and X11 is Glu.

The location of the amide bond in the lactam bridge between position 4 (X4) and position 7 (X7) may be equal distance between X4 and X7, such as when X4 is Glu and X7 is Dab.

Desirably, the length of the lactam bridge after formation of the amide bond (not including any atoms in the peptide backbone) is 4, 5, 6, 7, 8, 9, or 10 atoms; such as 4, 5, 6, or 9 atoms. For the lactam bridge between the amino acid residues at positions X1/X2 and X11, the length of the lactam bridge after formation of the amide bond may be 4, 5, or 9 atoms long. For the lactam bridge between the amino acid residues at positions X4 and X7, the length of the lactam bridge after formation of the amide bond may be 6 atoms long.

In some embodiments, the length of the lactam bridge after formation of the amide bond (not including any atoms in the peptide backbone) is 4 atoms. A suitable pairing of residues at positions X1/X2 and X11 in which the lactam bridge has a length of 4 atoms include: 3-(3-Pyridyl)-Ala and Glu.

In some embodiments, the length of the lactam bridge after formation of the amide bond (not including any atoms in the peptide backbone) is 5 atoms. A suitable pairing of residues at positions X1/X2 and X11 in which the lactam bridge has a length of 5 atoms include: 3-aminopropanoyl and Glu.

In some embodiments, the length of the lactam bridge after formation of the amide bond (not including any atoms in the peptide backbone) is 6 atoms. A suitable pairing of residues at positions X4 and X7 in which the lactam bridge has a length of 6 atoms include: Glu and Dab.

In some embodiments, the length of the lactam bridge after formation of the amide bond (not including any atoms in the peptide backbone) is 7 atoms.

Preferably, the length of the lactam bridge after formation of the amide bond (not including any atoms in the peptide backbone) is 8 atoms.

In some embodiments, the length of the lactam bridge after formation of the amide bond (not including any atoms in the peptide backbone) is 9 atoms. A suitable pairing of residues at positions X1/X2 and X11 in which the lactam bridge has a length of 9 atoms include: 4-aminomethyl-phenylacetyl and Glu.

In some embodiments, the length of the lactam bridge after formation of the amide bond (not including any atoms in the peptide backbone) is 10 atoms.

### Dithioether bridge

The contribution of the side chain to the length of the dithioether bridge is counted as the number of atoms in a linear chain from the first atom of the side chain (which is bonded to an atom of the peptide backbone, i.e. to the alpha carbon of the relevant residue for most amino acids) up to and including the atom which participates in the dithioether bond of the bridge (i.e. the sulfur atom).

Thus common sulfur-containing side chains are considered to have the following side chain lengths:

| | | |
|---|---|---|
| Cys | 2 atoms | 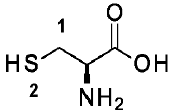 |

Preferably, X4 is Cys and X7 is Cys.

The length of the side chains of X4 and X7 are then added to the contribution of the linker between the sulfur moieties of the amino acid residues at positions X4 and X7 to determine the length of the bridge. For embodiments where the dithioether bridge is of the formula -S-L-Y-L-S-, the contribution of the linker is counted as the number of atoms in -L-Y-L-, as the sulfur atoms are already counted in the side chain length.

Desirably, the length of the dithioether bridge after the formation of the dithioether bonds (not including any atoms in the peptide backbone) is 5, 6, 7, 8, 9, or 10 atoms, such as 6, 7, 8, or 9 atoms; such as 7, 8, or 9 atoms, such as 7 or 8 atoms.

In some embodiments, the length of the dithioether bridge is 5 atoms long. In some embodiments, the length of the dithioether bridge is 6 atoms long.

In some embodiments, the length of the dithioether bridge is 7 atoms long, such as for the bridge -SCH₂C(=O)CH₂S-.

In some embodiments, the length of the dithioether bridge is 8 atoms long. In some embodiments, the length of the dithioether bridge is 9 atoms long. In some embodiments, the length of the dithioether bridge is 10 atoms long.

Preferably, the dithioether bridge is 7 atoms long.

### Synthesis of the compounds

The invention further provides a method of synthesis of a compound of the invention. The compounds (which may also be referred to as peptides) may suitably be manufactured by standard synthetic methods. Thus, the peptides may be synthesized by, e.g., methods comprising synthesizing the peptide by standard solid-phase or liquid-phase methodology, either stepwise or by fragment assembly, and optionally isolating and purifying the final peptide product. In this context, reference may be made to WO 98/11125 or, *inter alia,* Fields, G.B. et al., "Principles and Practice of Solid-Phase Peptide Synthesis"; in: Synthetic Peptides, Gregory A. Grant (ed.), Oxford University Press (2nd edition, 2002) and the synthesis examples herein. The method typically further comprises the step of forming an amide bond between the amino acid residues at position 1 (X1) or at position 2 (X2) when the amino acid residue at position 1 is absent and at position 11 (X11), and optionally further comprising the step of forming an amide bond or forming two thioether bonds with a linker between the amino acid residues at positions 4 (X4) and 7 (X7), e.g. as described below. In the case of solid phase synthesis, cyclisation may be performed in situ on the solid phase (e.g. resin), i.e. before removal of the peptide from the solid phase.

The synthesis of some example compounds of the invention are provided in Example 1. Generally, the method for the synthesis of said compound comprises synthesising the compound by solid-phase or liquid-phase peptide synthesis methodology, optionally isolating and/or purifying the final product, and optionally further comprising the step of forming an amide bond between the amino acid residues at position 1 or at position 2 when the amino acid residue at position 1 is absent and at position 11, and optionally further comprising the step of forming an amide bond or forming two thioether bonds with a linker between the amino acid residues at positions 4 and 7.

The order of the steps in the synthesis of the compounds are not necessarily in the order mentioned above.

For example, the order of the formation of the bridges (lactam bridge/amide bond; dithioether bridge/two thioether bonds) may be in any order. In some embodiments, the bridge between the amino acid residues at position 1 or at position 2 when the amino acid residue at position 1 is absent and at position 11 is formed first, then the bridge between the amino acid residues at positions 4 and 7 is formed second. In other embodiments, the bridge between the amino acid residues at positions 4 and 7 is formed first, then the bridge between the amino acid residues at position 1 or at position 2 when the amino acid residue at position 1 is absent and at position 11 is formed second.

### Efficacy of the compounds

The compounds of the invention are interleukin-23 receptor (IL-23R) inhibitors, i.e. they are capable of binding to, and blocking signalling by, one or more receptors or receptor complexes regarded as physiological receptors for interleukin-23 (IL-23).

Comparative activity may be measured by any suitable means, such as via determination of IC₅₀ values as described below.

Compounds of the present invention may exhibit a number of advantageous properties in relation to other peptide IL-23R inhibitors thereof, such as analogues described in WO 2016/011208, WO 2018/022937, WO 2018/136646, WO 2020/014646, WO 2021/146441, WO 2021/146458, WO 2023/288017, WO 2023/288019, WO 2023/288028, Heinis et al., 2020, and PCT/EP2022/084077. As compared to any of these analogues, compounds of the invention may, for example, exhibit improved effects, e.g., in the form of improved *in vitro* potency at IL-23R.

Additionally or alternatively, compounds of the invention may exhibit improved gastrointestinal (GI) stability as compared to any of the peptide inhibitors of IL-23R described in the art.

The skilled person will be aware of suitable assay formats, and examples are provided below. For example, the assays may make use of employing measurements on the human IL-23R (see the examples below). Where sequences of precursor proteins are referred to, it should be understood that assays may make use of the mature protein, lacking the signal sequence.

K_{d} values may be used as a numerical measure of the binding affinity at a given receptor. A K_{d} value, also termed the equilibrium dissociation constant, is a measure of how tightly a compound binds to a receptor in a particular assay. A small K_{d} indicates the compound binds tighter with higher affinity to the receptor as compared to a compound with a higher K_{d} value. Thus, for example, a compound having a K_{d} [IL-23R] value lower than the K_{d} [IL-23R] value of another compound inhibitor of IL-23R in a particular assay may be considered to have a stronger binding affinity (or binds more tightly) to IL-23R than that of the other compound inhibitor of IL-23R.

In absence of an experimental method to directly determine the K_{d} of a compound for a receptor, the binding affinities for compounds may be estimated by means of determining the IC₅₀ of a compound. The IC₅₀ is determined by the compound's ability to compete with a labelled compound for the receptor. In such a competition assay, the concentration for which half of the labelled compound is displaced from the receptor by the unlabelled compound is termed the IC₅₀ value. The IC₅₀ value is proportional to the affinity of the compound for the receptor (that is, its K_{d} value), and is assay system dependent as it depends on factors such as the concentration of the labelled compound used, the affinity of the labelled compound for the receptor, and assay incubation times.

In a binding assay format an IC₅₀ value may be used as a numerical format to measure how tightly a compound binds to a receptor in a particular assay. A small IC₅₀ indicates the compound binds tighter with higher affinity to the receptor as compared to a compound with a higher IC₅₀ value. Thus, for example, a compound having a IC₅₀ [IL-23R] value lower than the IC₅₀ [IL-23R] value of another compound inhibitor of IL-23R in a particular assay may be considered to have a stronger binding affinity (or binds more tightly) to IL-23R than that of the other compound inhibitor of IL-23R.

In some embodiments of compounds of the present invention, the IC₅₀ towards IL-23R is below 1000 nM (e.g. 0.0001 to 1000 nM).

In some embodiments of compounds of the present invention, the IC₅₀ towards IL-23R is below 500 nM (e.g. 0.0001 to 500 nM).

In some embodiments of compounds of the present invention, the IC₅₀ towards IL-23R is below 100 nM (e.g. 0.0001 to 100 nM).

In some embodiments of compounds of the present invention, the IC₅₀ towards IL-23R is below 50 nM (e.g. 0.0001 to 50 nM).

In some embodiments of compounds of the present invention, the IC₅₀ towards IL-23R is below 30 nM (e.g. 0.0001 to 30 nM).

In some embodiments of compounds of the present invention, the IC₅₀ towards IL-23R is below 20 nM (e.g. 0.0001 to 20 nM).

In some embodiments of compounds of the present invention, the IC₅₀ towards IL-23R is below 10 nM (e.g. 0.0001 to 10 nM).

In some embodiments of compounds of the present invention, the IC₅₀ towards IL-23R is below 5 nM (e.g. 0.0001 to 5 nM).

In some embodiments of compounds of the present invention, the IC₅₀ towards IL-23R is below 1 nM (e.g. 0.0001 to 1 nM).

In some embodiments of compounds of the present invention, the IC₅₀ towards IL-23R is below 0.5 nM (e.g. 0.0001 to 0.5 nM).

In a functional assay format, measuring the ability of compounds to inhibit IL-23 mediated signalling in a cell-based assay, IC₅₀ values may be used as a numerical measure of inhibitor potency. An IC₅₀ value is a measure of the concentration of a compound required to achieve half of that compound's maximal activity in a particular assay. Thus, for example, a compound having an IC₅₀ [IL-23R] value lower than that of another compound inhibitor of IL-23R in a particular assay may be considered to have a stronger inhibitory potency, presumably by better blocking of the IL-23 mediated signalling, than that of the other peptide inhibitor of IL-23R.

In some embodiments of compounds of the present invention, the IC₅₀ towards IL-23 mediated signalling is below 1000 nM (e.g. 0.0001 to 1000 nM).

In some embodiments of compounds of the present invention, the IC₅₀ towards IL-23 mediated signalling is below 500 nM (e.g. 0.0001 to 500 nM).

In some embodiments of compounds of the present invention, the IC₅₀ towards IL-23 mediated signalling is below 100 nM (e.g. 0.0001 to 100 nM).

In some embodiments of compounds of the present invention, the IC₅₀ towards IL-23 mediated signalling is below 50 nM (e.g. 0.0001 to 50 nM).

In some embodiments of compounds of the present invention, the IC₅₀ towards IL-23 mediated signalling is below 30 nM (e.g. 0.0001 to 30 nM).

In some embodiments of compounds of the present invention, the IC₅₀ towards IL-23 mediated signalling is below 20 nM (e.g. 0.0001 to 20 nM).

In some embodiments of compounds of the present invention, the IC₅₀ towards IL-23 mediated signalling is below 10 nM (e.g. 0.0001 to 10 nM).

In some embodiments of compounds of the present invention, the IC₅₀ towards IL-23 mediated signalling is below 5 nM (e.g. 0.0001 to 5 nM).

In some embodiments of compounds of the present invention, the IC₅₀ towards IL-23 mediated signalling is below 1 nM (e.g. 0.0001 to 1 nM).

In some embodiments of compounds of the present invention, the IC₅₀ towards IL-23 mediated signalling is below 0.5 nM (e.g. 0.0001 to 0.5 nM).

Such assays may be performed under the conditions described in Examples 3-1 and 3-2 below.

Additionally or alternatively, compounds of the invention may show gastrointestinal (GI) stability, i.e. resistance to degradation in the gastrointestinal tract. This can be measured using a simulated intestinal fluid (SIF) assay and/or a simulated gastric fluid (SGF) assay. For example, the compounds of the invention may retain at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 99% of the remaining compound or peptide after incubation for 1 hour, or for 4 hours, e.g. under the conditions described in **Examples 3-3 and 3-4.** Preferably, the compounds retain at least 70% (or more) of the compound after incubation for 1 hour, or 4 hours, under the SIF and/or SGF assay.

### Pharmaceutical compositions

The invention also extends to compositions, such as pharmaceutical compositions, comprising the compounds of the invention. As with all aspects of the invention, it is to be understood that reference to a compound of the invention encompasses reference to pharmaceutically acceptable salts and solvates.

The compounds of the present invention may be formulated as pharmaceutical compositions which are suited for administration with or without storage, and which typically comprise a therapeutically effective amount of at least one peptide of the invention, together with a pharmaceutically acceptable carrier, excipient or vehicle.

The term "pharmaceutically acceptable carrier" includes any of the standard pharmaceutical carriers. Pharmaceutically acceptable carriers for therapeutic use are well known in the pharmaceutical art and are described, for example, in "Remington's Pharmaceutical Sciences", 17th edition, Alfonso R. Gennaro (Ed.), Mark Publishing Company, Easton, PA, USA, 1985.

### Therapeutic uses

The compounds of the invention, and pharmaceutical compositions comprising said compounds, are useful in a method of prevention or treatment of various conditions.

The method of prevention or treatment comprises administering to the subject an effective amount of the compound of the invention, or the pharmaceutical composition comprising said compound.

In some embodiments, the conditions may be selected from inflammatory bowel disease (IBD), Crohn's Disease, ulcerative colitis, psoriatic arthritis, and psoriasis.

In some embodiments, the conditions may be selected from inflammatory bowel disease (IBD), Crohn's Disease, ulcerative colitis, and psoriasis.

In some embodiments, the conditions may be selected from inflammatory bowel disease (IBD) and psoriasis.

The subject or patient may be an animal subject or patient. The subject or patient may be a human subject or patient. Preferably, the subject is a human subject or patient.

### Dosages

A typical dosage of a compound as employed in the context of the present invention may be in the range from about 0.0001 to about 100 mg/kg body weight per day, such as from about 0.0005 to about 50 mg/kg body weight per day, such as from about 0.001 to about 10 mg/kg body weight per day, e.g. from about 0.01 to about 1 mg/kg body weight per day, administered in one or more doses, such as from one to three doses. The exact dosage employed will depend, *inter alia,* on: the nature and severity of the disease or disorder to be treated, on the sex, age, body weight and general condition of the subject to be treated, on possible other, concomitant, disease or disorder that is undergoing or is to undergo treatment, as well as on other factors that will be known to a medical practitioner of skill in the art.

### EXAMPLES

The following examples demonstrate certain specific embodiments of the present invention. The following examples were carried out using standard techniques that are well known and routine to those of skill in the art, except where otherwise described in detail. It is to be understood that these examples are for illustrative purposes only and do not purport to be wholly definitive as to conditions or scope of the invention. As such, they should not be construed as limiting the scope of the present invention in any way.

Abbreviations employed for the amino acids and particular R² groups may be found in **Tables A-C** in the definitions. Other abbreviations employed in the examples include:

| | |
|---|---|
| ^{t}BuOH | tert-Butanol |
| DODT | 2,2'-(Ethylenedioxy)diethanethiol |
| Pd(PPh₃)₄ | tetrakis(triphenylphosphine)palladium(0)) |
| PhSiH₃ | phenylsilane |
| PyBOP | benzotriazol-1-yloxytripyrrolidinophosphonium hexafluorophosphate) |
| equiv. | equivalents |
| r.t. | room temperature |
| aq. | Aqueous |
| IL-23R | interleukin-23 receptor |
| hIL-23R | human interleukin-23 receptor |
| GI | gastrointestinal |
| SIF | simulated intestinal fluid |
| SGF | simulated gastric fluid |
| Nluc | NanoBRET luciferase assay |
| SD | standard deviation |
| %Eff | percentage efficacy |
| pSTAT3 | phosphorylated signal transducer and activator of transcription 3 |
| BRET | Bioluminescence Resonance Energy Transfer |
| TAMRA | 5'-tetramethylrhodamine-5-carboxamide |

The following examples are provided to illustrate certain embodiments of the invention and are not intended to limit the scope of the invention.

### Example 1: Synthesis of compounds

The following compounds were synthesised in **Table 1-1** below.

**Table 1-1: Synthesised compounds**

| **Compound** | **Structure** |
|---|---|
| 1 | [3-Aminopropanoyl](4c)*[3-(3-Pyridyl)-Ala]SC(2a)WQC(2a)[Y(2-aminoethoxy)][2-Nal][2-Me-Leu]E(4c)[{d}R]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CSCC(=O)CSC[C@H](NC(=O)[C@H](CO)NC(= O)[C@H](Cc3cccnc3)NC(=O)CCNC(=O)CC[C@@H](C(=O)N[C@H](CCCNC (=N)N)C(N)=O)NC1=O)C(=O)N[C@@H](Cc1c[nH]c3ccccc13)C(=O)N[C@@ H](CCC(N)=O)C(=O)N2 |
| 2 | [3-(3-Pyridyl)-Ala](4c)*[3-Aminopropanoyl]SC(2a)WQC(2a)[Y(2-aminoethoxy)][2-Nal][2-Me-Leu]E(4c)[{d}R]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CSCC(=O)CSC[C@H](NC(=O)[C@H](CO)NC(= O)CCNC(=O)[C@H](Cc3cccnc3)NC(=O)CC[C@@H](C(=O)N[C@H](CCCNC (=N)N)C(N)=O)NC1=O)C(=O)N[C@@H](Cc1c[nH]c3ccccc13)C(=O)N[C@@ H](CCC(N)=O)C(=O)N2 |
| 3 | [4-Aminomethyl-phenylacetyl](1c)*SE(2c)WQ[Dab](2c)[Y(2-aminoethoxy)][2-Nal][2-Me-Leu]E(1c)[Dab][2-Me-3-(3-Pyridyl)-Ala]-[NH2] |
| | CC(C)C[C@]1 (C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)N[C@@](C)( Cc3cccnc3)C(N)=O)NC1=O)C(=O)N[C@@H](Cc1c[nH]c3ccccc13)C(=O)N[C @@H](CCC(N)=O)C(=O)N2 |
| 4 | [4-Aminomethyl-phenylacetyl](1c)*SE(2c)WQ[Dab](2c)[Y(2-aminoethoxy)][2-Nal][2-Me-Leu]E(1c)[Dab][N-Me-3-(3-Pyridyl)-Ala]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)N(C)[C@@ H](Cc3cccnc3)C(N)=O)NC1=O)C(=O)N[C@@H](Cc1c[nH]c3ccccc13)C(=O) N[C@@H](CCC(N)=O)C(=O)N2 |
| 5 | [4-Aminomethyl-phenylacetyl](1c)*SE(2c)WQ[Dab](2c)[Y(2-aminoethoxy)][2-Nal][2-Me-Leu]E(1c)[Dab][{d}3-(3-Pyridyl)-Ala]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)N[C@H](Cc 3cccnc3)C(N)=O)NC1=O)C(=O)N[C@@H](Cc1c[nH]c3ccccc13)C(=O)N[C@ @H](CCC(N)=O)C(=O)N2 |
| 6 | [4-Aminomethyl-phenylacetyl](1c)*SE(2c)WQ[Dab](2c)[Y(2-aminoethoxy)][2-Nal][2-Me-Leu]E(1c)[Dab]-[NH-(4-(pyridin-3-yl)butanyl)] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)NCCCCc3cc cnc3)NC1=O)C(=O)N[C@@H](Cc1c[nH]c3ccccc13)C(=O)N[C@@H](CCC(N )=O)C(=O)N2 |
| 7 | [4-Aminomethyl-phenylacetyl](1c)*SE(2c)WQ[Dab](2c)[Y(2-aminoethoxy)][2-Nal][2-Me-Leu]E(1c)[Dab]-[NH-(2-(pyridin-3-yl)ethyl)] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)NCCc3cccn c3)NC1 =O)C(=O)N[C@@H](Cc1c[nH]c3ccccc13)C(=O)N[C@@H](CCC(N)= O)C(=O)N2 |
| 8 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](CCC(N)=O)NC(=O)[C@H](Cc3c[nH]c4ccccc34)NC2=O)C(=O) N[C@@H](Cc2ccc(OCCN)cc2)C(=O)N[C@@H](Cc2ccc3ccccc3c2)C(=O)N[ C@@](C)(CC(C)C)C(=O)N1 |
| 9 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala][Sar]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)N[C@@H]( Cc3cccnc3)C(=O)N(C)CC(N)=O)NC1=O)C(=O)N[C@@H](Cc1c[nH]c3ccccc 13)C(=O)N[C@@H](CCC(N)=O)C(=O)N2 |
| 10 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala][{d}H]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)N[C@@H]( Cc3cccnc3)C(=O)N[C@H](Cc3cnc[nH]3)C(N)=O)NC1=O)C(=O)N[C@@H](C c1c[nH]c3ccccc13)C(=O)N[C@@H](CCC(N)=O)C(=O)N2 |
| 11 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala][beta-hLeu]-[NH2] |
| | CC(C)C[C@@H](CC(N)=O)NC(=O)[C@H](Cc1cccnc1)NC(=O)[C@H](CCN) NC(=O)[C@@H]1CCC(=O)NCc2ccc(cc2)CC(=O)N[C@@H](CO)C(=O)N[C@ H]2CCC(=O)NCC[C@H](NC(=O)[C@H](CCC(N)=O)NC(=O)[C@H](Cc3c[nH] c4ccccc34)NC2=O)C(=O)N[C@@H](Cc2ccc(OCCN)cc2)C(=O)N[C@@H](C c2ccc3ccccc3c2)C(=O)N[C@@](C)(CC(C)C)C(=O)N1 |
| 12 | [4-Aminomethyl-phenylacetyl](1c)*SE(2c)WQ[Dab](2c)[Y(2-aminoethoxy)][2-Nal][2-Me-Leu]E(1c)[Dab]H[3-(3-Pyridyl)-Ala]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)N[C@@H]( Cc3cnc[nH]3)C(=O)N[C@@H](Cc3cccnc3)C(N)=O)NC1=O)C(=O)N[C@@H] (Cc1c[nH]c3ccccc13)C(=O)N[C@@H](CCC(N)=O)C(=O)N2 |
| 13 | [4-Aminomethyl-phenylacetyl](1c)*SE(2c)WQ[Dab](2c)[Y(2-aminoethoxy)][2-Nal][2-Me-Leu]E(1c)[Dab][{d}H]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)N[C@H](Cc 3cnc[nH]3)C(N)=O)NC1=O)C(=O)N[C@@H](Cc1c[nH]c3ccccc13)C(=O)N[C @@H](CCC(N)=O)C(=O)N2 |
| 14 | [4-Aminomethyl-phenylacetyl](1c)*SE(2c)WQ[Dab](2c)[Y(2-aminoethoxy)][2-Nal][2-Me-Leu]E(1c)[Dab][H(Me)]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)N[C@@H]( Cc3cn(C)cn3)C(N)=O)NC1=O)C(=O)N[C@@H](Cc1c[nH]c3ccccc13)C(=O)N [C@@H](CCC(N)=O)C(=O)N2 |
| 15 | [4-Aminomethyl-phenylacetyl](1c)*SE(2c)WQ[Dab](2c)[Y(2-aminoethoxy)][2-Nal][2-Me-Leu]E(1c)[Iso-Dab][2-Me-3-(3-Pyridyl)-Ala]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)NCC[C@H](N)C(=O)N[C@@](C)(C c3cccnc3)C(N)=O)NC1=O)C(=O)N[C@@H](Cc1c[nH]c3ccccc13)C(=O)N[C @@H](CCC(N)=O)C(=O)N2 |
| 16 | [4-Aminomethyl-phenylacetyl](1c)*SE(2c)WQ[Dab](2c)[Y(2-aminoethoxy)][2-Nal][2-Me-Leu]E(1c)[{d}Gly-CF3][3-(3-Pyridyl)-Ala]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)NC[C@@H](N[C@@H](Cc3cccnc3 )C(N)=O)C(F)(F)F)NC1=O)C(=O)N[C@@H](Cc1c[nH]c3ccccc13)C(=O)N[C @@H](CCC(N)=O)C(=O)N2 |
| 17 | [4-Aminomethyl-phenylacetyl](1c)*SE(2c)WQ[Dab](2c)[Y(2-aminoethoxy)][2-Nal][2-Me-Leu]E(1c)[Gly-CF3][3-(3-Pyridyl)-Ala]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)NC[C@H](N[C@@H](Cc3cccnc3)C (N)=O)C(F)(F)F)NC1=O)C(=O)N[C@@H](Cc1c[nH]c3ccccc13)C(=O)N[C@ @H](CCC(N)=O)C(=O)N2 |
| 18 | [4-Aminomethyl-phenylacetyl](1c)*[N-Me-Ser]E(2c)WQ[Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N(C)[C@@H](CO)C(=O)N[C@H]2CCC(=O)NCC[C@H ](NC(=O)[C@H](CCC(N)=O)NC(=O)[C@H](Cc3c[nH]c4ccccc34)NC2=O)C(= O)N[C@@H](Cc2ccc(OCCN)cc2)C(=O)N[C@@H](Cc2ccc3ccccc3c2)C(=O) N[C@@](C)(CC(C)C)C(=O)N1 |
| 19 | [4-Aminomethyl-phenylacetyl](1c)*SE(2c)WQ[Dab](2c)[Y(2-aminoethoxy)][2-Nal][2-Me-Leu]E(1c)[Dab][3-(3,5-Pyrimidyl)-Ala]-[NHMe] |
| | CNC(=O)[C@H](Cc1cncnc1)NC(=O)[C@H](CCN)NC(=O)[C@@H]1CCC(=O )NCc2ccc(cc2)CC(=O)N[C@@H](CO)C(=O)N[C@H]2CCC(=O)NCC[C@H]( NC(=O)[C@H](CCC(N)=O)NC(=O)[C@H](Cc3c[nH]c4ccccc34)NC2=O)C(=O )N[C@@H](Cc2ccc(OCCN)cc2)C(=O)N[C@@H](Cc2ccc3ccccc3c2)C(=O)N[ C@@](C)(CC(C)C)C(=O)N1 |
| 20 | [4-Aminomethyl-phenylacetyl](1c)*AE(2c)WQ[Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](Cc2ccc(OCCN)cc2)C(=O)N[C@@H](Cc2ccc3ccccc3c2)C(=O)N[C @@](C)(CC(C)C)C(=O)N1 |
| 21 | [4-Aminomethyl-phenylacetyl](1c)*FE(2c)WQ[Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](Cc2ccccc2)C(=O)N[C@H]2CCC(=O)NCC[C @H](NC(=O)[C@H](CCC(N)=O)NC(=O)[C@H](Cc3c[nH]c4ccccc34)NC2=O) C(=O)N[C@@H](Cc2ccc(OCCN)cc2)C(=O)N[C@@H](Cc2ccc3ccccc3c2)C( =O)N[C@@](C)(CC(C)C)C(=O)N1 |
| 22 | [4-Aminomethyl-phenylacetyl](1c)*SE(2c)WA[Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](C)NC(=O)[C@H](Cc3c[nH]c4ccccc34)NC2=O)C(=O)N[C@@H] (Cc2ccc(OCCN)cc2)C(=O)N[C@@H](Cc2ccc3ccccc3c2)C(=O)N[C@@](C)( CC(C)C)C(=O)N1 |
| 23 | [4-Aminomethyl-phenylacetyl](1c)*SE(2c)WF[Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](Cc3ccccc3)NC(=O)[C@H](Cc3c[nH]c4ccccc34)NC2=O)C(=O) N[C@@H](Cc2ccc(OCCN)cc2)C(=O)N[C@@H](Cc2ccc3ccccc3c2)C(=O)N[ C@@](C)(CC(C)C)C(=O)N1 |
| 24 | [4-Aminomethyl-phenylacetyl](1c)*SE(2c)W[K(Ac)][Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](CCCCNC(C)=O)NC(=O)[C@H](Cc3c[nH]c4ccccc34)NC2=O)C( =O)N[C@@H](Cc2ccc(OCCN)cc2)C(=O)N[C@@H](Cc2ccc3ccccc3c2)C(=O )N[C@@](C)(CC(C)C)C(=O)N1 |
| 25 | [4-Aminomethyl-phenylacetyl](1c)*SE(2c)W[Dab(Ac)][Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](CCNC(C)=O)NC(=O)[C@H](Cc3c[nH]c4ccccc34)NC2=O)C(=O )N[C@@H](Cc2ccc(OCCN)cc2)C(=O)N[C@@H](Cc2ccc3ccccc3c2)C(=O)N[ C@@](C)(CC(C)C)C(=O)N1 |
| 26 | [4-Aminomethyl-phenylacetyl](1c)*SE(2c)W[Cit][Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](CCCNC(N)=O)NC(=O)[C@H](Cc3c[nH]c4ccccc34)NC2=O)C(= O)N[C@@H](Cc2ccc(OCCN)cc2)C(=O)N[C@@H](Cc2ccc3ccccc3c2)C(=O) N[C@@](C)(CC(C)C)C(=O)N1 |
| 27 | [4-Aminomethyl-phenylacetyl](1c)*SE(2c)W[Q(pyrrolidin)][Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](CCC(=O)N3CCCC3)NC(=O)[C@H](Cc3c[nH]c4ccccc34)NC2= O)C(=O)N[C@@H](Cc2ccc(OCCN)cc2)C(=O)N[C@@H](Cc2ccc3ccccc3c2) C(=O)N[C@@](C)(CC(C)C)C(=O)N1 |
| 28 | [4-Aminomethyl-phenylacetyl](1c)*SE(2c)W[Orn][Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](CCCN)NC(=O)[C@H](Cc3c[nH]c4ccccc34)NC2=O)C(=O)N[C @@H](Cc2ccc(OCCN)cc2)C(=O)N[C@@H](Cc2ccc3ccccc3c2)C(=O)N[C@ @](C)(CC(C)C)C(=O)N1 |
| 29 | [4-Aminomethyl-phenylacetyl](1c)*SE(2c)W[Q(Me)][Dab](2c)[Y(2-aminoethoxy)][2-Nal][2-Me-Leu]E(1c)[Dab][3-(3-Pyridyl)-Ala]-[NHMe] |
| | CNC(=O)CC[C@@H]1NC(=O)[C@H](Cc2c[nH]c3ccccc23)NC(=O)[C@@H]2 CCC(=O)NCC[C@H](NC1=O)C(=O)N[C@@H](Cc1ccc(OCCN)cc1)C(=O)N[ C@@H](Cc1ccc3ccccc3c1)C(=O)N[C@@](C)(CC(C)C)C(=O)N[C@H](C(=O) N[C@@H](CCN)C(=O)N[C@@H](Cc1cccnc1)C(=O)NC)CCC(=O)NCc1ccc(c c1)CC(=O)N[C@@H](CO)C(=O)N2 |
| 30 | [4-Aminomethyl-phenylacetyl](1c)*SE(2c)W[Q(2Me)][Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](CCC(=O)N(C)C)NC(=O)[C@H](Cc3c[nH]c4ccccc34)NC2=O)C( =O)N[C@@H](Cc2ccc(OCCN)cc2)C(=O)N[C@@H](Cc2ccc3ccccc3c2)C(=O )N[C@@](C)(CC(C)C)C(=O)N1 |
| 31 | [4-Aminomethyl-phenylacetyl](1c)*SE(2c)WQ[Dab](2c)[Y(2-aminoethoxy)][2-Nal][2-Me-Leu]E(1c)S[3-(3-Pyridyl)-Ala]-[NHMe] |
| | CNC(=O)[C@H](Cc1cccnc1)NC(=O)[C@H](CO)NC(=O)[C@@H]1CCC(=O) NCc2ccc(cc2)CC(=O)N[C@@H](CO)C(=O)N[C@H]2CCC(=O)NCC[C@H](N C(=O)[C@H](CCC(N)=O)NC(=O)[C@H](Cc3c[nH]c4ccccc34)NC2=O)C(=O) N[C@@H](Cc2ccc(OCCN)cc2)C(=O)N[C@@H](Cc2ccc3ccccc3c2)C(=O)N[ C@@](C)(CC(C)C)C(=O)N1 |
| 32 | [4-Aminomethyl-phenylacetyl](1c)*SE(2c)WQ[Dab](2c)[Y(2-aminoethoxy)][2-Nal][2-Me-Leu]E(1c)[Nle][3-(3-Pyridyl)-Ala]-[NHMe] |
| | CCCC[C@H](NC(=O)[C@@H]1CCC(=O)NCc2ccc(cc2)CC(=O)N[C@@H](C O)C(=O)N[C@H]2CCC(=O)NCC[C@H](NC(=O)[C@H](CCC(N)=O)NC(=O)[ C@H](Cc3c[nH]c4ccccc34)NC2=O)C(=O)N[C@@H](Cc2ccc(OCCN)cc2)C(= O)N[C@@H](Cc2ccc3ccccc3c2)C(=O)N[C@@](C)(CC(C)C)C(=O)N1)C(=O) N[C@@H](Cc1cccnc1)C(=O)NC |
| 33 | [4-Aminomethyl-phenylacetyl](1c)*SE(2c)WQ[Dab](2c)[Y(2-aminoethoxy)][2-Nal][2-Me-Leu]E(1c)Q[3-(3-Pyridyl)-Ala]-[NHMe] |
| | CNC(=O)[C@H](Cc1cccnc1)NC(=O)[C@H](CCC(N)=O)NC(=O)[C@@H]1CC C(=O)NCc2ccc(cc2)CC(=O)N[C@@H](CO)C(=O)N[C@H]2CCC(=O)NCC[C @H](NC(=O)[C@H](CCC(N)=O)NC(=O)[C@H](Cc3c[nH]c4ccccc34)NC2=O) C(=O)N[C@@H](Cc2ccc(OCCN)cc2)C(=O)N[C@@H](Cc2ccc3ccccc3c2)C( =O)N[C@@](C)(CC(C)C)C(=O)N1 |
| 34 | [4-Aminomethyl-phenylacetyl](1c)*SE(2c)WQ[Dab](2c)[Y(2-aminoethoxy)][2-Nal][2-Me-Leu]E(1c)H[3-(3-Pyridyl)-Ala]-[NHMe] |
| | CNC(=O)[C@H](Cc1cccnc1)NC(=O)[C@H](Cc1cnc[nH]1)NC(=O)[C@@H]1 CCC(=O)NCc2ccc(cc2)CC(=O)N[C@@H](CO)C(=O)N[C@H]2CCC(=O)NCC [C@H](NC(=O)[C@H](CCC(N)=O)NC(=O)[C@H](Cc3c[nH]c4ccccc34)NC2= O)C(=O)N[C@@H](Cc2ccc(OCCN)cc2)C(=O)N[C@@H](Cc2ccc3ccccc3c2) C(=O)N[C@@](C)(CC(C)C)C(=O)N1 |
| 35 | [4-Aminomethyl-phenylacetyl](1c)*SE(2c)WQ[Dab](2c)[Y(2-aminoethoxy)][2-Nal][2-Me-Leu]E(1c)[THP][3-(3-Pyridyl)-Ala]-[NHMe] |
| | CNC(=O)[C@H](Cc1cccnc1)NC(=O)C1(NC(=O)[C@@H]2CCC(=O)NCc3ccc (cc3)CC(=O)N[C@@H](CO)C(=O)N[C@H]3CCC(=O)NCC[C@H](NC(=O)[C @H](CCC(N)=O)NC(=O)[C@H](Cc4c[nH]c5ccccc45)NC3=O)C(=O)N[C@@ H](Cc3ccc(OCCN)cc3)C(=O)N[C@@H](Cc3ccc4ccccc4c3)C(=O)N[C@@](C )(CC(C)C)C(=O)N2)CCOCC1 |
| 36 | [4-Aminomethyl-phenylacetyl](1c)*SE(2c)WQ[Dab](2c)[Y(2-aminoethoxy)][2-Nal][2-Me-Leu]E(1c)[hSer][3-(3-Pyridyl)-Ala]-[NHMe] |
| | CNC(=O)[C@H](Cc1cccnc1)NC(=O)[C@H](CCO)NC(=O)[C@@H]1CCC(=O )NCc2ccc(cc2)CC(=O)N[C@@H](CO)C(=O)N[C@H]2CCC(=O)NCC[C@H]( NC(=O)[C@H](CCC(N)=O)NC(=O)[C@H](Cc3c[nH]c4ccccc34)NC2=O)C(=O )N[C@@H](Cc2ccc(OCCN)cc2)C(=O)N[C@@H](Cc2ccc3ccccc3c2)C(=O)N[ C@@](C)(CC(C)C)C(=O)N1 |
| 37 | [4-Aminomethyl-phenylacetyl](1c)*SE(2c)WQ[Dab](2c)[Y(2-aminoethoxy)][2-Nal][2-Me-Leu]E(1 c)[Ser(OMe)][3-(3-Pyridyl)-Ala]-[NHMe] |
| | CNC(=O)[C@H](Cc1cccnc1)NC(=O)[C@H](COC)NC(=O)[C@@H]1CCC(=O )NCc2ccc(cc2)CC(=O)N[C@@H](CO)C(=O)N[C@H]2CCC(=O)NCC[C@H]( NC(=O)[C@H](CCC(N)=O)NC(=O)[C@H](Cc3c[nH]c4ccccc34)NC2=O)C(=O )N[C@@H](Cc2ccc(OCCN)cc2)C(=O)N[C@@H](Cc2ccc3ccccc3c2)C(=O)N[ C@@](C)(CC(C)C)C(=O)N1 |
| 38 | [4-Aminomethyl-phenylacetyl](1c)*SE(2c)WQ[Dab](2c)[Y(2-aminoethoxy)][2-Nal][2-Me-Leu]E(1c)[{d}S][3-(3-Pyridyl)-Ala]-[NHMe] |
| | CNC(=O)[C@H](Cc1cccnc1)NC(=O)[C@@H](CO)NC(=O)[C@@H]1CCC(=O )NCc2ccc(cc2)CC(=O)N[C@@H](CO)C(=O)N[C@H]2CCC(=O)NCC[C@H]( NC(=O)[C@H](CCC(N)=O)NC(=O)[C@H](Cc3c[nH]c4ccccc34)NC2=O)C(=O )N[C@@H](Cc2ccc(OCCN)cc2)C(=O)N[C@@H](Cc2ccc3ccccc3c2)C(=O)N[ C@@1(C)(CC(C)C)C(=O)N1 |
| 39 | [4-Aminomethyl-phenylacetyl](1c)*SE(2c)WQ[Dab](2c)[Y(2-aminoethoxy)][2-Nal][2-Me-Leu]E(1c)[2-Me-Ser][3-(3-Pyridyl)-Ala]-[NHMe] |
| | CNC(=O)[C@H](Cc1cccnc1)NC(=O)[C@](C)(CO)NC(=O)[C@@H]1CCC(=O) NCc2ccc(cc2)CC(=O)N[C@@H](CO)C(=O)N[C@H]2CCC(=O)NCC[C@H](N C(=O)[C@H](CCC(N)=O)NC(=O)[C@H](Cc3c[nH]c4ccccc34)NC2=O)C(=O) N[C@@H](Cc2ccc(OCCN)cc2)C(=O)N[C@@H](Cc2ccc3ccccc3c2)C(=O)N[ C@@](C)(CC(C)C)C(=O)N1 |
| 40 | [4-Aminomethyl-phenylacetyl](1c)*AE(2c)WQ[Dab](2c)[Y(2-aminoethoxy)][2-Nal][2-Me-Leu]E(1c)S[3-(3-Pyridyl)-Ala]-[NHMe] |
| | CNC(=O)[C@H](Cc1cccnc1)NC(=O)[C@H](CO)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](Cc2ccc(OCCN)cc2)C(=O)N[C@@H](Cc2ccc3ccccc3c2)C(=O)N[C @@](C)(CC(C)C)C(=0)N1 |
| 41 | [4-Aminomethyl-phenylacetyl](1c)*AE(2c)WQ[Dab](2c)[hPhe][2-Nal][Aib]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](CCc2ccccc2)C(=O)N[C@@H](Cc2ccc3ccccc3c2)C(=O)NC(C)(C)C( =O)N1 |
| 42 | [4-Aminomethyl-phenylacetyl](1c)*AE(2c)WQ[Dab](2c)[Y(2-aminoethoxy)][Cyclopropyl-Ala][Aib]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](Cc2ccc(OCCN)cc2)C(=O)N[C@@H](CC2CC2)C(=O)NC(C)(C)C(= O)N1 |
| 43 | [4-Aminomethyl-phenylacetyl](1c)*AE(2c)WQ[Dab](2c)[7-AzaTrp][2-Nal][Aib]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](Cc2c[nH]c3ncccc23)C(=O)N[C@@H](Cc2ccc3ccccc3c2)C(=O)NC( C)(C)C(=O)N1 |
| 44 | [4-Aminomethyl-phenylacetyl](1c)*AE(2c)WQ[Dab](2c)[Y(2-aminoethoxy)][7-AzaTrp][Aib]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](Cc2ccc(OCCN)cc2)C(=O)N[C@@H](Cc2c[nH]c3ncccc23)C(=O)NC (C)(C)C(=O)N1 |
| 45 | [4-Aminomethyl-phenylacetyl](1c)*AE(2c)WQ[Dab](2c)[beta-hTrp][2-Nal][Aib]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](Cc2c[nH]c3ccccc23)CC(=O)N[C@@H](Cc2ccc3ccccc3c2)C(=O)NC (C)(C)C(=O)N1 |
| 46 | [4-Aminomethyl-phenylacetyl](1c)*AE(2c)WQ[Dab](2c)[Y(2-aminoethoxy)][beta-hTrp][Aib]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](Cc2ccc(OCCN)cc2)C(=O)N[C@@H](Cc2c[nH]c3ccccc23)CC(=O)N C(C)(C)C(=O)N1 |
| 47 | [4-Aminomethyl-phenylacetyl](1c)*AE(2c)WQ[Dab](2c)[7-F-Trp][2-Nal][Aib]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](Cc2c[nH]c3c(F)cccc23)C(=O)N[C@@H](Cc2ccc3ccccc3c2)C(=O)N C(C)(C)C(=O)N1 |

The bridging amino acid residue is the amino acid residue directly preceding the parentheses below. The square brackets indicate the bridging amino acid residues. For example, [2,11] is a bridge between amino acid residues 2 and 11. Similarly, [4,7] is a bridge between amino acid residues 4 and 7, and [1,11] is a bridge between amino acid residues 1 and 11.
* denotes that the (1c), (2a), (2c), and (4c) bridges use the peptide backbone amine or carboxylic acid at the /V- or C-terminus, not the side chain amine or carboxylic acid
(1c) denotes the [2,11] lactam bridge; (2a) denotes the [4,7] 1,3-dithio-propan-2-one bridge; (2c) denotes the [4,7] lactam bridge; (4c) denotes the [1,11] lactam bridge.

The compound numbering of **Table 1-1** corresponds to the same number as the SEQ ID NO., as shown in **Table 1-1a** below.

**Table 1-1a: Sequence ID Numbers of synthesised compounds from Table 1-1**

| **SEQ ID NO:** | **Sequence** |
|---|---|
| SEQ ID NO: 1 | [3-Aminopropanoyl](4c)*[3-(3-Pyridyl)-Ala]SC(2a)WQC(2a)[Y(2-aminoethoxy)] [2-Nal][2-Me-Leu] E(4c)[{d}R] |
| SEQ ID NO: 2 | [3-(3-Pyridyl)-Ala](4c)*[3-Aminopropanoyl]SC(2a)WQC(2a)[Y(2-aminoethoxy)][2-Nal][2-Me-Leu]E(4c)[{d}R] |
| SEQ ID NO: 3 | [4-Aminomethyl-phenylacetyl](1c)*SE(2c)WQ[Dab](2c)[Y(2-aminoethoxy)][2-Nal][2-Me-Leu]E(1c)[Dab][2-Me-3-(3-Pyridyl)-Ala] |
| SEQ ID NO: 4 | [4-Aminomethyl-phenylacetyl](1c)*SE(2c)WQ[Dab](2c)[Y(2-aminoethoxy)][2-Nal][2-Me-Leu]E(1c)[Dab][N-Me-3-(3-Pyridyl)-Ala] |
| SEQ ID NO: 5 | [4-Aminomethyl-phenylacetyl](1c)*SE(2c)WQ[Dab](2c)[Y(2-aminoethoxy)][2-Nal][2-Me-Leu]E(1c)[Dab][{d}3-(3-Pyridyl)-Ala] |
| SEQ ID NO: 6 | [4-Aminomethyl-phenylacetyl](1c)*SE(2c)WQ[Dab](2c)[Y(2-aminoethoxy)][2-Nal][2-Me-Leu]E(1c)[Dab] |
| SEQ ID NO: 7 | [4-Aminomethyl-phenylacetyl](1c)*SE(2c)WQ[Dab](2c)[Y(2-aminoethoxy)][2-Nal][2-Me-Leu]E(1c)[Dab] |
| SEQ ID NO: 8 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala] |
| SEQ ID NO: 9 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala][Sar] |
| SEQ ID NO: 10 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala][{d}H] |
| SEQ ID NO: 11 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala][beta-hLeu] |
| SEQ ID NO: 12 | [4-Aminomethyl-phenylacetyl](1c)*SE(2c)WQ[Dab](2c)[Y(2-aminoethoxy)][2-Nal][2-Me-Leu]E(1c)[Dab]H[3-(3-Pyridyl)-Ala] |
| SEQ ID NO: 13 | [4-Aminomethyl-phenylacetyl](1c)*SE(2c)WQ[Dab](2c)[Y(2-aminoethoxy)][2-Nal][2-Me-Leu]E(1c)[Dab][{d}H] |
| SEQ ID NO: 14 | [4-Aminomethyl-phenylacetyl](1c)*SE(2c)WQ[Dab](2c)[Y(2-aminoethoxy)][2-Nal][2-Me-Leu]E(1c)[Dab][H(Me)] |
| SEQ ID NO: 15 | [4-Aminomethyl-phenylacetyl](1c)*SE(2c)WQ[Dab](2c)[Y(2-aminoethoxy)][2-Nal][2-Me-Leu]E(1c)[Iso-Dab][2-Me-3-(3-Pyridyl)-Ala] |
| SEQ ID NO: 16 | [4-Aminomethyl-phenylacetyl](1c)*SE(2c)WQ[Dab](2c)[Y(2-aminoethoxy)][2-Nal][2-Me-Leu]E(1c)[{d}Gly-CF3][3-(3-Pyridyl)-Ala] |
| SEQ ID NO: 17 | [4-Aminomethyl-phenylacetyl](1c)*SE(2c)WQ[Dab](2c)[Y(2-aminoethoxy)][2-Nal][2-Me-Leu]E(1c)[Gly-CF3][3-(3-Pyridyl)-Ala] |
| SEQ ID NO: 18 | [4-Aminomethyl-phenylacetyl](1c)*[N-Me-Ser]E(2c)WQ[Dab](2c)[Y(2-aminoethoxy)][2-Nal][2-Me-Leu]E(1c)[Dab][3-(3-Pyridyl)-Ala] |
| SEQ ID NO: 19 | [4-Aminomethyl-phenylacetyl](1c)*SE(2c)WQ[Dab](2c)[Y(2-aminoethoxy)][2-Nal][2-Me-Leu]E(1c)[Dab][3-(3,5-Pyrimidyl)-Ala] |
| SEQ ID NO: 20 | [4-Aminomethyl-phenylacetyl](1c)*AE(2c)WQ[Dab](2c)[Y(2-aminoethoxy)][2-Nal][2-Me-Leu]E(1c)[Dab][3-(3-Pyridyl)-Ala] |
| SEQ ID NO: 21 | [4-Aminomethyl-phenylacetyl](1c)*FE(2c)WQ[Dab](2c)[Y(2-aminoethoxy)][2-Nal][2-Me-Leu]E(1c)[Dab][3-(3-Pyridyl)-Ala] |
| SEQ ID NO: 22 | [4-Aminomethyl-phenylacetyl](1c)*SE(2c)WA[Dab](2c)[Y(2-aminoethoxy)][2-Nal][2-Me-Leu]E(1c)[Dab][3-(3-Pyridyl)-Ala] |
| SEQ ID NO: 23 | [4-Aminomethyl-phenylacetyl](1c)*SE(2c)WF[Dab](2c)[Y(2-aminoethoxy)][2-Nal][2-Me-Leu]E(1c)[Dab][3-(3-Pyridyl)-Ala] |
| SEQ ID NO: 24 | [4-Aminomethyl-phenylacetyl](1c)*SE(2c)W[K(Ac)][Dab](2c)[Y(2-aminoethoxy)][2-Nal][2-Me-Leu]E(1c)[Dab][3-(3-Pyridyl)-Ala] |
| SEQ ID NO: 25 | [4-Aminomethyl-phenylacetyl](1c)*SE(2c)W[Dab(Ac)][Dab](2c)[Y(2-aminoethoxy)][2-Nal][2-Me-Leu]E(1c)[Dab][3-(3-Pyridyl)-Ala] |
| SEQ ID NO: 26 | [4-Aminomethyl-phenylacetyl](1c)*SE(2c)W[Cit][Dab](2c)[Y(2-aminoethoxy)][2-Nal][2-Me-Leu]E(1c)[Dab][3-(3-Pyridyl)-Ala] |
| SEQ ID NO: 27 | [4-Aminomethyl-phenylacetyl](1c)*SE(2c)W[Q(pyrrolidin)][Dab](2c)[Y(2-aminoethoxy)][2-Nal][2-Me-Leu]E(1c)[Dab][3-(3-Pyridyl)-Ala] |
| SEQ ID NO: 28 | [4-Aminomethyl-phenylacetyl](1c)*SE(2c)W[Orn][Dab](2c)[Y(2-aminoethoxy)][2-Nal][2-Me-Leu]E(1c)[Dab][3-(3-Pyridyl)-Ala] |
| SEQ ID NO: 29 | [4-Aminomethyl-phenylacetyl](1c)*SE(2c)W[Q(Me)][Dab](2c)[Y(2-aminoethoxy)][2-Nal][2-Me-Leu]E(1c)[Dab][3-(3-Pyridyl)-Ala] |
| SEQ ID NO: 30 | [4-Aminomethyl-phenylacetyl](1c)*SE(2c)W[Q(2Me)][Dab](2c)[Y(2-aminoethoxy)][2-Nal][2-Me-Leu]E(1c)[Dab][3-(3-Pyridyl)-Ala] |
| SEQ ID NO: 31 | [4-Aminomethyl-phenylacetyl](1c)*SE(2c)WQ[Dab](2c)[Y(2-aminoethoxy)][2-Nal][2-Me-Leu]E(1c)S[3-(3-Pyridyl)-Ala] |
| SEQ ID NO: 32 | [4-Aminomethyl-phenylacetyl](1c)*SE(2c)WQ[Dab](2c)[Y(2-aminoethoxy)][2-Nal][2-Me-Leu]E(1c)[Nle][3-(3-Pyridyl)-Ala] |
| SEQ ID NO: 33 | [4-Aminomethyl-phenylacetyl](1c)*SE(2c)WQ[Dab](2c)[Y(2-aminoethoxy)][2-Nal][2-Me-Leu]E(1c)Q[3-(3-Pyridyl)-Ala] |
| SEQ ID NO: 34 | [4-Aminomethyl-phenylacetyl](1c)*SE(2c)WQ[Dab](2c)[Y(2-aminoethoxy)][2-Nal][2-Me-Leu]E(1c)H[3-(3-Pyridyl)-Ala] |
| SEQ ID NO: 35 | [4-Aminomethyl-phenylacetyl](1c)*SE(2c)WQ[Dab](2c)[Y(2-aminoethoxy)][2-Nal][2-Me-Leu]E(1c)[THP][3-(3-Pyridyl)-Ala] |
| SEQ ID NO: 36 | [4-Aminomethyl-phenylacetyl](1c)*SE(2c)WQ[Dab](2c)[Y(2-aminoethoxy)][2-Nal][2-Me-Leu]E(1c)[hSer][3-(3-Pyridyl)-Ala] |
| SEQ ID NO: 37 | [4-Aminomethyl-phenylacetyl](1c)*SE(2c)WQ[Dab](2c)[Y(2-aminoethoxy)][2-Nal][2-Me-Leu]E(1c)[Ser(OMe)][3-(3-Pyridyl)-Ala] |
| SEQ ID NO: 38 | [4-Aminomethyl-phenylacetyl](1c)*SE(2c)WQ[Dab](2c)[Y(2-aminoethoxy)][2-Nal][2-Me-Leu]E(1c)[{d}S][3-(3-Pyridyl)-Ala] |
| SEQ ID NO: 39 | [4-Aminomethyl-phenylacetyl](1c)*SE(2c)WQ[Dab](2c)[Y(2-aminoethoxy)][2-Nal][2-Me-Leu]E(1c)[2-Me-Ser][3-(3-Pyridyl)-Ala] |
| SEQ ID NO: 40 | [4-Aminomethyl-phenylacetyl](1c)*AE(2c)WQ[Dab](2c)[Y(2-aminoethoxy)][2-Nal][2-Me-Leu]E(1c)S[3-(3-Pyridyl)-Ala] |
| SEQ ID NO: 41 | [4-Aminomethyl-phenylacetyl](1c)*AE(2c)WQ[Dab](2c)[hPhe][2-NaI][Aib]E(1c)[Dab] [3-(3-Pyridyl)-Ala] |
| SEQ ID NO: 42 | [4-Aminomethyl-phenylacetyl](1c)*AE(2c)WQ[Dab](2c)[Y(2-aminoethoxy)][Cyclopropyl-Ala][Aib]E(1c)[Dab][3-(3-Pyridyl)-Ala] |
| SEQ ID NO: 43 | [4-Aminomethyl-phenylacetyl](1c)*AE(2c)WQ[Dab](2c)[7-AzaTrp][2-NaI][Aib]E(1c) [Dab] [3-(3-Pyridyl)-Ala] |
| SEQ ID NO: 44 | [4-Aminomethyl-phenylacetyl](1c)*AE(2c)WQ[Dab](2c)[Y(2-aminoethoxy)][7-AzaTrp][Aib]E(1c)[Dab][3-(3-Pyridyl)-Ala] |
| SEQ ID NO: 45 | [4-Aminomethyl-phenylacetyl](1c)*AE(2c)WQ[Dab](2c)[beta-hTrp][2-Nal][Aib]E(1c)[Dab][3-(3-Pyridyl)-Ala] |
| SEQ ID NO: 46 | [4-Aminomethyl-phenylacetyl](1c)*AE(2c)WQ[Dab](2c)[Y(2-aminoethoxy)][beta-hTrp][Aib]E(1c)[Dab][3-(3-Pyridyl)-Ala] |
| SEQ ID NO: 47 | [4-Aminomethyl-phenylacetyl](1c)*AE(2c)WQ[Dab](2c)[7-F-Trp][2-Nal][Aib]E(1c)[Dab][3-(3-Pyridyl)-Ala] |

wherein:
* denotes that the (1c), (2a), (2c), and (4c) bridges use the peptide backbone amine or carboxylic acid at the N- or C-terminus, not the side chain amine or carboxylic acid
(1c) denotes the [2,11] lactam bridge; (2a) denotes the [4,7] 1,3-dithio-propan-2-one bridge; (2c) denotes the [4,7] lactam bridge; (4c) denotes the [1,11] lactam bridge.

For comparison purposes, four compounds from Heinis *et al.,* 2020 having two 1,3-dithio-propan-2-one bridges were synthesised (**Table 1-2**).

**Table 1-2: Synthesised compounds from Heinis et al., 2020**

| **Compound** | **Structure** |
|---|---|
| I1 (isomer 3) | H-GC(1a)TC(2a)WEC(2a)WWLC(1a)S-[NH2] |
| I3 (isomer 3) | H-DC(1a)SC(2a)WQC(2a)WWLC(1a)R-[NH2] |
| I4 (isomer 3) | H-EC(4a)WLC(5a)YYRNC(5a)NC(4a)M-[NH2] |
| I5 (isomer 3) | H-GC(1a)TC(2a)WEC(2a)WW[2-Me-Leu]C(1a)S-[NH2] |

| | |
|---|---|
| (1a) denotes the [2,11] 1,3-dithio-propan-2-one bridge; (2a) denotes the [4,7] 1,3-dithio-propan-2-one bridge; (4a) denotes the [1,11] 1,3-dithio-propan-2-one bridge; (5a) denotes the [4,9] 1,3-dithio-propan-2-one bridge. | |

Additionally, two compounds from the Protagonist patent applications (Compound C in WO 2016/011208, WO 2017/011820, and Sayago *et al.,* 2018; and Peptide 993 in WO 2018/089693) having a bridging cystathionine amino acid residue at [2,7] was synthesised (**Table 1-3**).

**Table 1-3: Synthesised compound from the Protagonist patent applications**

| **Compound** | **Structure** |
|---|---|
| Compound C | [Ac]-[Abu](16i)QTWQC(16i)[Y(2-aminoethoxy)]W[2-Me-Lys]ENG-[NH2] |
| Peptide 993 | [Ac]-[{d}R][Abu](16i)QTWQC(16i)[Y(2-aminoethoxy)][2-Nal] [4-Aminotetrahydro-2H-pyran-4-carbonyl]ENN-[NH2] |

| | |
|---|---|
| (16i) denotes the [2,7] thioether bridge. The combination of Abu at position X2, Cys at position X7, and the [2,7] thioether bridge forms a cystathionine as the bridging amino acid residue. | |

Other reference compounds synthesised are described in **Table 1-4.** These reference compounds were disclosed in PCT/EP2022/084077.

**Table 1-4: Synthesised reference compounds**

| **Compound** | **Structure** |
|---|---|
| Ref 4 | H-DE(1c)SC(2a)WQC(2a)WWLK(1c)R-[NH2] |
| Ref 17 | [Ac]-DE(1c)SC(2a)WQC(2a)W[2-Nal]LK(1c)R-[NH2] |
| Ref 18 | [Ac]-K(1c)SC(2a)WQC(2a)W[2-Nal]LE(1c)R-[NH2] |
| Ref 37 | [(3-Aminomethyl)benzoyl](1c)*SC(2a)WQC(2a)W[2-Nal]LE(1c)R-[NH2] |
| Ref 41 | [(3-Aminomethyl)benzoyl](1c)*SC(2a)WQC(2a)[Y(2-aminoethoxy)][2-Nal][2-Me-Leu]E(1c)[{d}R]-[NH2] |
| Ref 52 | [4-Aminomethyl-phenylacetyl](1c)*SC(2a)WQC(2a)[Y(2-aminoethoxy)][2-Nal][2-Me-Leu]E(1c)R-[NH2] |
| Ref 78 | [(3-Aminomethyl)benzoyl](1c)*SC(2a)WQC(2a)[Y(2-aminoethoxy)][2-Nal][2-Me-Leu]E(1c)R[3-(3-Pyridyl)-Ala]-[NH2] |
| Ref 80 | [4-Aminomethyl-phenylacetyl](1c)*SC(2a)WQC(2a)[Y(2-aminoethoxy)][2-Nal][2-Me-Leu]E(1c)[{d}R]-[NH2] |
| Ref 81 | [4-Aminomethyl-phenylacetyl](1c)*SC(2a)WQC(2a)[Y(2-aminoethoxy)][2-Nal][2-Me-Leu]E(1c)S-[NH2] |
| Ref 82 | [4-Aminomethyl-phenylacetyl](1c)*SC(2a)WQC(2a)[Y(2-aminoethoxy)][2-Nal][2-Me-Leu]E(1c)[Dab]-[NH2] |
| Ref 120 | [4-Aminomethyl-phenylacetyl](1c)*SC(2a)WQC(2a)[Y(2-aminoethoxy)][2-Nal][2-Me-Leu]E(1c)[Dab][3-(3-Pyridyl)-Ala]-[NH2] |
| Ref 122 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala]-[NH2] |
| Ref 125 | [4-Aminomethyl-phenylacetyl](1c)*SE(2c)WQ[Dab](2c)[Y(2-aminoethoxy)][2-Nal][2-Me-Leu]E(1c)[Dab]-[NH2] |
| Ref 141 | [4-Aminomethyl-phenylacetyl](1c)*[N-Me-Ser]C(2a)WQC(2a)[Y(2-aminoethoxy)][2-Nal][2-Me-Leu]E(1c)[Dab]-[NH2] |
| Ref 146 | [4-Aminomethyl-phenylacetyl](1c)*SE(2c)W[Q(pyrrolidin)][Dab](2c)[Y(2-aminoethoxy)][2-Nal][2-Me-Leu]E(1c)[Dab]-[NH2] |
| Ref 155 | [Ac]-K(1c)TC(2a)WQC(2a)[Y(2-aminoethoxy)][2-Nal][2-Me-Leu]E(1c)[Dab][3-(3-Pyridyl)-Ala]-[NH2] |

| | |
|---|---|
| (1c) denotes the [2,11] lactam bridge; (2a) denotes the [4,7] 1,3-dithio-propan-2-one bridge; (2c) denotes the [4,7] lactam bridge, and * denotes that the (1c), (2a), and (2c) bridges use the peptide backbone amine or carboxylic acid at the *N*- or *C*-terminus, not the side chain amine or carboxylic acid. | |

Unless otherwise specified, reagents and solvents employed in the following were available commercially in standard laboratory reagent or analytical grade, and were used without further purification.

### Apparatus and synthetic strategy

Peptides were synthesized batchwise on a peptide synthesiser, such as a CEM Liberty Blue Peptide Synthesizer, according to solid phase peptide synthetic procedures using 9-fluorenylmethyloxycarbonyl (Fmoc) as N-α-amino protecting group and suitable common protection groups for side chain functionalities.

As polymeric support based resins, such as e.g. TentaGel^{™}, was used. The synthesizer was loaded with resin that prior to usage was swelled in DMF.

Non-naturally occurring amino acids and other suitable building blocks were employed without any changes to the general procedure.

Optical isomers of particular amino acids (including non-naturally occurring amino acids) were employed in the synthesis of the compounds and may be found in **Tables A and B** in the definitions. Definitions of particular R² groups may be found in **Table C** in the definitions.

### Coupling on a CEM Liberty Blue Peptide Synthesizer

A solution of Fmoc-protected amino acid (4 equiv.) was added to the resin together with a coupling reagent solution (4 equiv.) and a solution of base (8 equiv.). The mixture was either heated by the microwave unit to 50 °C and coupled for 10 minutes or coupled with no heat for 60 minutes. During the coupling nitrogen was bubbled through the mixture.

In the case of difficult couplings (e.g. coupling of a residue immediately after an N-methylated amino acid residue or other sterically hindered amino acid residue as recognized by a person of skill in the art) the coupling was repeated one or more times.

### Deprotection:

The Fmoc group was deprotected using piperidine in DMF or other suitable solvents. The deprotection solution was added to the reaction vessel and the mixture was heated for 5 minutes reaching approx. 50 °C. After draining the reaction vessel the resin was washed with DMF or other suitable solvents.

### Lactam formation:

The following procedure for the coupling of Glu and Lys is representative for all lactam formations where the amino acid side chain containing the carboxyl-function is protected with Oall and the amino acid side chain containing the amino group is Alloc-protected. After assembly of the full peptide sequence, deprotection of Glu(Oall) and Lys(Alloc) was performed using Pd(PPh₃)₄ (0.05 equiv.) and PhSiH₃ (10 equiv.) in DCM. Subsequently, the lactam bridge was formed between the side chain carboxylic acid of Glu and side chain amine of Lys using PyBOP (2 equiv.) and DIPEA (3.0 equiv.) in DMF. Both steps were performed with the peptide still attached to the resin.

Peptides with a lactam from a side chain to the *N*-terminal amine were prepared similarly. After assembly of the full peptide sequence, the Fmoc protection group of the *N*-terminal amine is left intact. Glu(Oall) is deprotected as described with Pd(PPh₃)₄ followed by a Fmoc deprotection (see section "cleavage"). The lactam bridge was formed similarly with PyBOP.

### Cleavage:

The dried peptide resin was treated with TFA and suitable scavengers for approximately 2 hours. The volume of the filtrate was reduced and the crude peptide was precipitated after addition of diethylether. The crude peptide precipitate was washed several times with diethylether and finally dried.

### HPLC purification of the crude peptide:

The crude peptide was purified by preparative reverse phase HPLC using a conventional HPLC apparatus, such as a Gilson GX-281 with 331/332 pump combination, for binary gradient application equipped with a column, such as 5 x 25 cm Gemini NX 5u C18 110A column, and a fraction collector using a flow 20-40 ml/min with a suitable gradient of buffer A (0.1% Formic acid, aq.) or A (0.1% TFA, aq.) and buffer B (0.1% Formic acid, 90% MeCN, aq.) or B (0.1% TFA, 90% MeCN, aq.). Fractions were analyzed by analytical HPLC and MS and selected fractions were pooled and lyophilized. The final product was characterized by HPLC and MS.

### Dithioether formation:

The methodology for dithioether formation from the reaction between two unprotected cysteines and a linker can be performed as previously described in Heinis *et al.,* 2020, or through the modified method below.

The crude intermediate peptide was dissolved in 3:7 mixture of water / acetonitrile (1 mg / mL). DODT (2 equiv.) was added and the mixture was stirred at room temperature for 10 min. The pH was adjusted to pH 8 by addition of 0.2 M ammonium carbonate. The linker (2 equiv.) was added directly to the solution and the mixture was left shaken at room temperature over night. The solution was filtered with 0.45 µm filter and loaded directly on a prep-HPLC column for a final purification.

Linkers used in the synthesized compounds include:
1,3-dibromopropan-2-one, CAS # 816-39-7.

### Gly-CF3 and D-Gly-CF3:

The di-amino acid "Fmoc-Gly-CF3-3Pal-OH" and "Fmoc-D-Gly-CF3-3Pal-OH" was synthesized as described in: Solid-Phase Synthesis of Gly-Ψ[CH(CF3)NH]-Peptides, Sgorbati et al., J. Org. Chem, 2021, 86, 9225-9232, using the same synthesis procedure as for "Fmoc-Gly-CF3-Phe-OH" (that is, compound 4c as shown in Table 1 following hydrolysis step (i) in Scheme 1b). These di-amino acids were utilized in the synthesis of, for example, compounds 16 and 17.

### Iso-amino acids:

Peptides with iso-amino acids were synthesized by standard Fmoc SPPS, using amino acid building blocks where the protecting groups at the *N*-terminal and side chain are switched or interchanged. For example, a peptide with a standard Lysine in its sequence is synthesised with the building block Fmoc-Lys(Boc)-OH, where Fmoc is the protecting group at the *N*-terminal amine and Boc at the side chain. In contrast, a peptide with iso-Lys would thus be synthesised with a building block of Boc-Lys(Fmoc)-OH instead of Fmoc-Lys(Boc)-OH. Coupling conditions for attaching the building block to the peptide and subsequent deprotecting conditions for removal of the Fmoc group are the same as described for standard Fmoc SPPS synthesis.

The side-chain protecting group "Boc" may be replaced with an "Alloc" protecting group in cases where side chain on-resin modifications are performed.

### Analytical HPLC:

Final purities were determined by analytic HPLC (Agilent 1100/1200 series) equipped with auto sampler, degasser, 20 µl flow cell and Chromeleon software. The HPLC was operated with a flow of 1.2 ml/min at 40 °C using an analytical column, such as Kinetex 2.6 µm XB-C18 100A 100x4,6 mm column. The compound was detected and quantified at 215 nm. Buffers A (0.1% TFA, aq.) and buffer B (0.1% TFA, 90% MeCN, aq.).

### Mass spectroscopy:

Final MS analysis was performed on a conventional mass spectrometer, e.g. Waters Xevo G2 Tof, equipped with electrospray detector with lock-mass calibration and MassLynx software. It was operated in positive mode using direct injection and a cone voltage of 15V (1 TOF), 30 V (2 TOF) or 45 V (3 TOF) as specified on the chromatogram. Precision was 5 ppm with a typical resolution of 15,000-20,000.

One of skill in the art will appreciate that standard methods of peptide synthesis may be used to generate the compounds of the invention.

### Example 2: Structure-Activity-Relationship (SAR) of the compounds

The SAR from the peptides I1 (isomer 3), I3 (isomer 3), I4 (isomer 3), and I5 (isomer 3) from Heinis *et al.,* 2020 and the compounds disclosed in PCT/EP2022/084077 are described in Example 2 of PCT/EP2022/084077, which is incorporated by reference.

The inventors identified Ref 122 as a favourable compound from PCT/EP2022/084077 (compound 122 of PCT/EP2022/084077):

**Ref 122 Amino Acid Sequence**

| **N** | **1** | **2** | **3** | **4** | **5** | **6** | **7** | **8** | **9** | **10** | **11** | **12** | **13** | **14** | **C** |
|---|---|---|---|---|---|---|---|---|---|---|---|---|---|---|---|
| - | - | α (1c) | S | E (2c) | W | Q | Dab (2c) | β | 2-Nal | γ | E (1c) | Dab | 3-Pal | - | NH2 |

| | | | | | | | | | | | | | | | |
|---|---|---|---|---|---|---|---|---|---|---|---|---|---|---|---|
| α = 4-aminomethyl-phenylacetyl; β = Y(2-aminoethoxy); γ = 2-Me-Leu; (1c) = [2,11] lactam bridge; (2c) = [4,7] lactam bridge. | | | | | | | | | | | | | | | |

Ref 122 was potent (Kᵢ hIL-23R of 0.00541 nM, equivalent to IC₅₀ (hIL-23R) of 0.0066 nM, and IC₅₀ (pSTAT3) of 0.028 nM - see **Examples 3-1 and 3-2, Tables 3-1 and 3-2** below). However, Ref 122 was not stable under the SIF assay (only 25% of intact Ref 122 was remaining in the SIF assay after 4 h - see **Example 3-3, Table 3-3** below) meaning it would not be sufficiently stable in the gastrointestinal tract.

The inventors identified a major metabolite formed after incubation of Ref 122 in the SIF assay which included the peptide without the C-terminal 3Pal (3-(3-Pyridyl)-Ala) amino acid.

Based on this insight and using Ref 122 as the peptide starting point the inventors thus tried four different approaches to improve SIF by addressing the C-terminus:
(i) Changing the C-terminal amino acid to a D-isomer amino acid;
(ii) Methylating the C-terminal amino acid;
(iii) Adding an unnatural amino acid at the C-terminus; and
(iv) Derivatisation of the C-terminus.

### (i) Changing the C-terminal amino acid to a D-isomer amino acid

The inventors changed the C-terminal 3-Pal in Ref 122 to D-3-Pal and D-His. The D-His and D-3-Pal modifications moderately increased SIF stability to 41% and 56 % of intact peptide remaining after 4 h (**Table 2-1**). However, both modifications slightly decreased the potency of the compound.

**Table 2-1: IC₅₀ and SIF data for D-isomer amino acids**

| | **13** | **14** | **C** | **IC₅₀ (hIL-23R binding) (nM)** | **IC₅₀ (pSTAT3) (nM)** | **SIF (% after 4 h)** |
|---|---|---|---|---|---|---|
| Ref 122 | 3-Pal | - | NH2 | 0.0066 | 0.028 | 25 |
| Compound 5 | {d}3-Pal | - | NH2 | 0.093 | 2.1 | 41 |
| Compound 13 | {d}H | - | NH2 | 0.059 | 6.8 | 56 |

All other amino acid residues are the same in the above compounds.

### (ii) Methylating the C-terminal amino acid

The inventors also methylated the C-terminal 3-Pal in Ref 122 to prevent cleavage. The 2-position of 3-Pal was methylated (2-Me-3-Pal), the amine of 3-Pal was methylated (N-Me-3-Pal), and the C-terminal amide was methylated (C = NHMe).

All three modifications greatly improved SIF stability to above 80% of intact peptide remaining after 4 h (**Table 2-2**). The 2-Me-3-Pal and N-Me-3-Pal slightly decreased the potency of the compound. However, the methylation of the C-terminal amide retained potency. Thus, methylation at the C-terminus improved SIF stability whilst retaining potency of the compound.

**Table 2-2: IC₅₀ and SIF data for methylated C-terminal amino acid**

| | **13** | **14** | **C** | **IC₅₀ (hIL-23R binding) (nM)** | **IC₅₀ (pSTAT3) (nM)** | **SIF (% after 4 h)** |
|---|---|---|---|---|---|---|
| Ref 122 | 3-Pal | - | NH2 | 0.0066 | 0.028 | 25 |
| Compound 3 | α | - | NH2 | 0.034 | 1.7 | 109 |
| Compound 4 | β | - | NH2 | 0.17 | 6.5 | 89 |
| Compound 8 | 3-Pal | - | NHMe | 0.0098 | 0.053 | 105 |

| | | | | | | |
|---|---|---|---|---|---|---|
| α = 2-Me-3-Pal; β = N-Me-3-Pal | | | | | | |

All other amino acid residues are the same in the above compounds.

### (iii) Adding an unnatural amino acid at the C-terminus

The inventors also tested if adding an additional unnatural amino acid residue at the C-terminus would prevent the 3-Pal cleavage in Ref 122. The addition of sarcosine (Sar), D-hisitidine ({d}H), and beta-homo-Leu at position 14 were tested.

The addition of D-hisitidine did not increase SIF stability, but the modification did retain potency of the compound (**Table 2-3**).

Nevertheless, the addition of Sar and beta-homo-Leu greatly improved SIF stability to above 102% of intact peptide remaining after 4 h (Table 2-3). The addition of beta-homo-Leu slightly decreased the potency of the compound, but the addition of Sar matained the potency of the compound.

**Table 2-3: IC₅₀ and SIF data for additional unnatural amino acid**

| | **13** | **14** | **C** | **IC₅₀ (hIL-23R binding) (nM)** | **IC₅₀ (pSTAT3) (nM)** | **SIF (% after 4 h)** |
|---|---|---|---|---|---|---|
| Ref 122 | 3-Pal | - | NH2 | 0.0066 | 0.028 | 25 |
| Compound 9 | 3-Pal | Sar | NH2 | 0.0053 | 0.025 | 105 |
| Compound 10 | 3-Pal | {d}H | NH2 | 0.0058 | 0.038 | 11 |
| Compound 11 | 3-Pal | Y | NH2 | 0.024 | 0.68 | 102 |

| | | | | | | |
|---|---|---|---|---|---|---|
| γ = beta-homo-Leu | | | | | | |

All other amino acid residues are the same in the above compounds.

### (iv) Derivatisation of the C-terminus

Finally, the inventors also tested if removing the 3-Pal and derivatising the group at the C-terminus would prevent degradation of Ref 122. An alkyl group substituted with a pyridyl group was used to derivatise the C-terminus.

Both of the tested C-terminal derivatives greatly improved SIF stability and prevented the degradation of the intact peptide remaining after 4 h **(Table 2-4).** The C-terminal derivatives slightly reduced the potency of the compound, with Compound 7 comprising the shorter derivative "NH-(2-(pyridin-3-yl)ethyl)" being more potent than Compound 6 with the longer derivative "NH-(4-(pyridin-3-yl)butanyl)".

**Table 2-4: IC₅₀ and SIF data for the derivatisation of the C-terminus**

| | **13** | **14** | **C** | **IC₅₀ (hIL-23R binding) (nM)** | **IC₅₀ (pSTAT3) (nM)** | **SIF (% after 4 h)** |
|---|---|---|---|---|---|---|
| Ref 122 | 3-Pal | - | NH2 | 0.0066 | 0.028 | 25 |
| Compound 6 | - | - | δ | 0.037 | 3.2 | 73 |
| Compound 7 | - | - | ε | 0.012 | 0.13 | 130 |

| | | | | | | |
|---|---|---|---|---|---|---|
| δ = NH-(4-(pyridin-3-yl)butanyl); ε = NH-(2-(pyridin-3-yl)ethyl) | | | | | | |

All other amino acid residues are the same in the above compounds.

In conclusion, the inventors have found four ways to improve the SIF stability of the compounds disclosed herein. The above modifications can be used alone or in combination to improve the SIF stability of the compounds, and in many cases also retain or improve the potency of the compounds against IL-23R.

In particular, the inventors identified Compound 8 from (ii), Compound 9 from (iii), and Compound 7 from (iv) as particularly potent and SIF stable compounds.

### Example 3: Biological Assays

### Example 3-1: Binding assay for estimating binding affinity of compounds to human IL-23R

The compound binding affinity for IL-23R was estimated by the ability of compounds to displace a fluorophore-labelled reference compound from the human IL-23R. The assay principle relies on Bioluminescence Resonance Energy Transfer (BRET) between a fluorophore-labelled reference compound binding to the IL-23R part of a fusion protein consisting of the IL-23R fused to a Nanoluc luciferase enzyme (Nanoluc). The Nanoluc is placed in the *N*-terminus in close proximity to the binding domain of the ligand. Upon close proximity between the fluorophore of the fluorophore-labelled compound and the Nanoluc of the fusion protein, bioluminescence energy generated from conversion of a Nanoluc substrate, is transferred to the fluorophore resulting in an increase in BRET. In presence of an unlabelled compound, the unlabelled compound displaces the fluorophore-labelled peptide from its binding site resulting in a decrease in BRET. The concentration for which half of the fluorophore-labelled peptide is displaced by the unlabelled compound depends on the affinity of the compound for the IL-23R, and is termed the IC₅₀ concentration.

The fusion protein was generated by subcloning of the cDNA encoding the mature human IL-23R (primary accession number UniProtKB - Q5VWK5, amino acids 22-629) and a small linker sequence in frame with a mammalian expression plasmid encoding a secretion signal and the Nanoluc protein (N1371, Promega). The plasmid also contained a gene to confer resistance for the antibiotic hygromycin. A cell line stably expressing the Nluc-IL23R fusion protein was generated by transfection of HEK293 cells with the expression plasmid and selected with hygromycin for 3 weeks in Growth medium consisting of DMEM w/ Glutamax-I containing, 10% V/V FBS, 1% V/V P/S, 1 mM Sodium Pyruvate, and 1X NEAA, and 0.3 mg/mL hygromycin. The remaining cells were propagated and considered a stable expressing Nluc-IL23R fusion protein pool clone.

Cells expressing the Nluc-IL23R fusion protein were expanded in Growth medium and membranes prepared by homogenization of a cell pellet from 18 T175 flasks (4 °C in subsequent steps). The cell pellet was lysed Tris 10 mM, 7.5, 1 mM EDTA and protease inhibitors (Complete, Roche) and homogenized using a 15 mL glass-dounce by 50 strokes. The homogenate was spun at spin @1500 rpm for 10 min, the supernatant transferred to SV-34 tubes and spun at 40000g for 20 min at 4 °C to pellet the crude membranes. The supernatant was then removed, the pellet resuspended in 5 mL buffer containing 50 mM HEPES pH 7.4, 5 mM EGTA, and 5 mM MgCl₂, and homogenized. Aliquots of the resuspended and homogenized membranes containing the Nluc-IL23R fusion protein were stored at -80 °C until use.

Compounds to be tested for binding to IL-23R were serially diluted in Assay buffer (50 mM HEPES pH 7.4, 5 mM EGTA, 5 mM MgCl₂, 0.005% Tween-20, and 0.05% casein) and added to the wells of a white 384-well plate (Corning 3572) in a volume of 6.25 µL, together with 12.5 µL diluted membranes containing the Nluc-IL23R fusion protein (0.42 µg/well) and 6.25 µL fluorescently labelled peptide to a final at a concentration of 3.1 nM, also prepared in Assay buffer. The plate was sealed with light impermeable plate seals, and incubated on an orbital shaker 400 rpm for 2 hours at room temperature. To determine the BRET ratio, the plate seal was removed and 25 µL of the Nanoluc substrate (Promega N1572) diluted 1:500 was added to each well and incubated for 1-2 minutes on an orbital shaker at 400 rpm. Then plate was read on an Envision plate reader equipped with a luminescence mirror module (barcode 404) using filters corresponding to Nanoluc substrate luminescence (M470 filter; 470 nm, bandwidth 24 nm) and TAMRA fluorescence (M595p filter 595 nm, bandwidth 60 nm). The BRET ratio was calculated as the fluorescence from the TAMRA/nanoluc bioluminescence.

For data analysis BRET ratios were normalized relative to the BRET signal by 3.1 nM TAMRA-labelled peptide alone (no unlabelled compound added) and the BRET signal under complete displacement (by adding a very high concentration of unlabelled peptide). Compound potency (IC₅₀) and maximal displacement (% displacement) were estimated by computer-aided curve fitting using a 4-parameter logistic (4PL) non-linear model. The IC₅₀ and maximal for each compound was determined by computer-aided curve fitting using a 4-parameter logistic (4PL) non-linear model. Data for the compound potency (IC₅₀) and maximal displacement (% displacement) are shown in **Table 3-1.** Typically compounds with low IC₅₀ are desired. Typically compounds with a high degree of displacement of the TAMRA-labelled peptide are desired. Due to experimental errors in the particular assay, values equal to or above 90% are typically considered as capable of completely the TAMRA-labelled peptide.

In the previous application PCT/EP2022/084077, the data were provided as calculated Kᵢ values. In this application, the inventors provide the data as IC₅₀ values. The Cheng-Prussov equation describes the relationship between the Kᵢ and the IC₅₀ as: Kᵢ = IC₅₀ / (1+[L_{L}]/K_{dL}), where [L_{L}] is the concentration of labelled compound used and K_{dL} is the equilibrium dissociation constant for the labelled compound (Cheng and Prusoff, 1973). To enable comparison between values, the inventors have determined Kᵢ and IC₅₀ for a number of compounds from PCT/EP2022/084077:

**Kᵢ and corresponding IC₅₀ values for compounds in PCT/EP2022/084077**

| **Compound** | **Kᵢ hIL-23R (nM)** | **IC₅₀ (hIL-23R binding) (nM)** |
|---|---|---|
| Ref 4 | 143 | 180 |
| Ref 17 | 70.7 | 87 |
| Ref 18 | 2.85 | 3.5 |
| Ref 37 | 9.87 | 12 |
| Ref 41 | 2.31 | 2.8 |
| Ref 52 | 0.0475 | 0.058 |
| Ref 78 | 0.0205 | 0.025 |
| Ref 80 | 0.150 | 0.18 |
| Ref 81 | 0.318 | 0.39 |
| Ref 82 | 0.0665 | 0.082 |
| Ref 120 | 0.00938 | 0.011 |
| Ref 122 | 0.00541 | 0.0066 |
| Ref 125 | 0.599 | 0.73 |
| Ref 141 | - | 3.5 |
| Ref 146 | 1.01 | 1.2 |
| Ref 155 | 0.00805 | 0.0099 |
| I1 (isomer 3) | 104 | 130 |
| I3 (isomer 3) | 38.0 | 47 |
| I4 (isomer 3) | Tested | Tested |
| I5 (isomer 3) | 73.6 | 90 |
| Compound C | 2.11 | 2.6 |

| | | |
|---|---|---|
| Tested = Kᵢ or IC₅₀ estimated higher than 1 µM when tested in assays | | |

**Table 3-1: IC₅₀ (hIL-23R binding) and % displacement (hIL-23R binding) data**

| **Compound** | **IC₅₀ (hIL-23R binding) (nM)** | **% displacement (hIL-23R binding)** |
|---|---|---|
| 1 | 5.4 | 105 |
| 2 | 2.9 | 103 |
| 3 | 0.034 | 99 |
| 4 | 0.17 | 111 |
| 5 | 0.093 | 99 |
| 6 | 0.037 | 103 |
| 7 | 0.012 | 102 |
| 8 | 0.0098 | 103 |
| 9 | 0.0053 | 100 |
| 10 | 0.0058 | 91 |
| 11 | 0.024 | 103 |
| 12 | 0.057 | 104 |
| 13 | 0.059 | 100 |
| 14 | 0.11 | 102 |
| 15 | 0.45 | 96 |
| 16 | 0.13 | 101 |
| 17 | 1.2 | 98 |
| 18 | 0.015 | 96 |
| 19 | 0.054 | 103 |
| 20 | 0.012 | 97 |
| 21 | 0.014 | 95 |
| 22 | 0.035 | 96 |
| 23 | 0.12 | 97 |
| 24 | 0.014 | 97 |
| 25 | 0.011 | 93 |
| 26 | 0.015 | 95 |
| 27 | 0.017 | 97 |
| 28 | 0.011 | 97 |
| 29 | 0.011 | 96 |
| 30 | 0.018 | 104 |
| 31 | 0.012 | 97 |
| 32 | 0.027 | 98 |
| 33 | 0.014 | 92 |
| 34 | 0.013 | 90 |
| 35 | 0.039 | 101 |
| 36 | 0.014 | 94 |
| 37 | 0.013 | 95 |
| 38 | 0.066 | 102 |
| 39 | 0.024 | 99 |
| 40 | 0.019 | 100 |
| 41 | 0.28 | 104 |
| 42 | 12 | 100 |
| 43 | 0.048 | 101 |
| 44 | 0.27 | 100 |
| 45 | 4.2 | 104 |
| 46 | 57 | 101 |
| 47 | 0.069 | 103 |
| Ref 4 | 180 | 100 |
| Ref 17 | 87 | 101 |
| Ref 18 | 3.5 | 103 |
| Ref 37 | 12 | 103 |
| Ref 41 | 2.8 | 110 |
| Ref 52 | 0.058 | 100 |
| Ref 78 | 0.025 | 100 |
| Ref 80 | 0.18 | 98 |
| Ref 81 | 0.39 | 101 |
| Ref 82 | 0.082 | 103 |
| Ref 120 | 0.011 | 94 |
| Ref 122 | 0.0066 | 97 |
| Ref 125 | 0.73 | 101 |
| Ref 141 | 3.5 | 102 |
| Ref 146 | 1.2 | 105 |
| Ref 155 | 0.0099 | 97 |
| I1 (isomer 3) | 130 | 105 |
| I3 (isomer 3) | 47 | 101 |
| I4 (isomer 3) | Tested | 84 |
| I5 (isomer 3) | 90 | 102 |
| Compound C | 2.6 | 102 |
| Peptide 993 | 0.24 | 101 |

| | | |
|---|---|---|
| Tested = IC₅₀ estimated higher than 1 µM when tested in assays | | |

### Example 3-2: Functional inhibition of IL-23 mediated STAT3 signalling by compounds

The ability of compounds to inhibit IL-23 mediated signalling was determined in the human-derived DB cell line (CRL-2289) (hereafter referred to as DB cells), which endogenously expresses the human IL-23R and human IL-12R β1 subunits. Upon binding of IL-23, the IL-23R forms a heterodimer signalling complex together with IL-12R β1, which through the JAK2/STAT3 pathway promotes phosphorylation of STAT3 to form phospho-STAT3. In the assay, the functional antagonism of IL-23 mediated phospho-STAT3 formation in DB cells by the compound is quantified using reagents capable of measuring the phosphorylation state of Tyr705 of STAT3 in the form of a phospho-STAT3 (Tyr705) MSD (Meso Scale Discovery) kit.

The assay was used to quantify the functional antagonism of a compound and to rank order the inhibitor compounds according to their potency. For compounds tested in this assay, the response was normalized relative to control values to calculate the IC₅₀ and maximal inhibitory response from a concentration response curve of the compound in presence of a fixed concentration of human IL-23.

The assay procedure was as follows. DB cells were maintained in growth medium consisting of RPMI-1640 [Invitrogen 61870-010] supplemented with 10% V/V Foetal Bovine Serum (FBS) [(heat inactivated), Invitrogen 10270-106], and 1% V/V Penicillin-Streptomycin (Pen-Strep) Solution [Invitrogen 15140]. On the day of assay, cells were resuspended in Assay buffer consisting of RPMI-1640 [Invitrogen 61870-010] supplemented with 0.1 % w/V BSA [Sigma-Aldrich A9430] to a density of 7.5x10⁶ cells/mL. Compounds to be tested for inhibition of hIL-23 mediated signalling were serially diluted in Assay buffer to 3X the final concentration. A solution of hIL-23 corresponding to 3X the EC₈₀ of hIL-23 (1.7 nM) was also prepared in Assay buffer. To initiate the assay, 20 µL of DB cell suspension (corresponding to 150.000 cells/well) was added to the wells of a 96-well V-bottom Polypropylene plate [Corning 3363] followed by addition of 3X 20 µL of the diluted test compounds to separate wells. Following preincubation of the DB cells with inhibitor for 15 min in a cell incubator (37 °C, 5% CO₂), 20 µL of the prepared 3X the EC₈₀ of hIL-23 solution was added to each well and incubated for 90 min in a cell incubator (37 °C, 5% CO₂). For some wells buffer only or IL-23 only corresponding to the EC₈₀ was added to obtain readouts needed for normalization. To terminate the assay, the plate was spun at 1000 G for 5 min to pellet cells, the supernatant removed using an 8-channel manual pipette, and then added 50 µL/well Complete Lysis Buffer from the MSD STAT3 kit (Cat# K150SVD, Mesoscale) to the cell pellet. To completely lyse cells to release phospho-STAT3 for detection, the plate was shaken for 10 min at room temperature (500 rpm) sealed with aluminium foil and placed at -80 °C for a minimum of 15 min. Detection of the phospho-STAT3 level in the cell lysate from the individual wells was determined using the MSD STAT3 kit (Cat# K150SVD, Mesoscale) and read on a Meso QuickPlex SQ 120 plate reader (Mesoscale).

For data analysis raw data counts from the Meso QuickPlex SQ 120 plate reader were normalized relative to the response by the EC₈₀ of hIL-23 alone (no compound added) and the buffer level. Compound potency (IC₅₀) and maximal inhibitory response (% inhibition) were estimated by computer-aided curve fitting using a 4-parameter logistic (4PL) non-linear model. Data for the compound potency (IC₅₀) and maximal inhibitory response (% displacement) are shown in **Table 3-2.** The lower the IC₅₀ value, the more potent the compound. Typically compounds with a low IC₅₀ are desired. Typically compounds capable of completely inhibiting the IL23-induced response are desired. Due to experimental errors in the particular assay, values equal to or above 95% are typically considered as capable of completely inhibiting IL23R-induced signaling.

**Table 3-2: hIL-23 (pSTAT3) antagonist IC₅₀ and % inhibition data**

| **Compound** | **hIL-23 (pSTAT3) antagonist IC₅₀ (nM)** | **hIL-23 (pSTAT3) antagonist % inhibition** |
|---|---|---|
| 3 | 1.7 | 98 |
| 4 | 6.5 | 100 |
| 5 | 2.1 | 95 |
| 6 | 3.2 | 99 |
| 7 | 0.13 | 98 |
| 8 | 0.053 | 101 |
| 9 | 0.025 | 97 |
| 10 | 0.038 | 99 |
| 11 | 0.68 | 98 |
| 12 | 3.5 | 100 |
| 13 | 6.8 | 99 |
| 14 | 7.2 | 99 |
| 15 | 12 | 97 |
| 16 | 1.4 | 97 |
| 20 | 0.026 | 101 |
| 21 | 0.063 | 102 |
| 22 | 3.4 | 101 |
| 23 | 5.2 | 99 |
| Ref 4 | 850 | 87 |
| Ref 17 | 370 | - |
| Ref 18 | 18 | 97 |
| Ref 37 | 45 | 89 |
| Ref 41 | 29 | 99 |
| Ref 52 | 7.8 | 103 |
| Ref 78 | 0.13 | 100 |
| Ref 80 | 11 | 97 |
| Ref 81 | 21 | 95 |
| Ref 82 | 11 | 97 |
| Ref 120 | 0.054 | 101 |
| Ref 122 | 0.028 | 99 |
| Ref 125 | 21 | 98 |
| Ref 146 | 42 | 98 |
| Ref 155 | 0.031 | 100 |
| I1 (isomer 3) | Tested | 83 |
| I3 (isomer 3) | 410 | 100 |
| I4 (isomer 3) | Tested | 23 |
| I5 (isomer 3) | 930 | 91 |
| Compound C | 13 | 99 |
| Peptide 993 | 1.4 | 98 |

| | | |
|---|---|---|
| Tested = IC₅₀ estimated higher than 1 µM when tested in assays | | |

### Examples 3-3 and 3-4: Determination of peptide stability in simulated gastric fluid (SGF) and simulated intestinal fluid (SIF)

SGF and SIF were prepared based on the United States Pharmacopeia specifications (Test Solutions, United States Pharmacopeia 35, NF 30, 2012). SGF was prepared by dissolving sodium chloride (0.2 g) in 50 mL of water. A volume of 0.7 mL of 10 M HCl was added to adjust the pH of the solution to 1.2 and the volume made up to 100 mL with water. 64 mg porcine pepsin (P7125, Sigma Aldrich) was gently dissolved into 20 mL preheated (37 °C) solution (3.2 g/L) immediately prior to the incubation. SIF was prepared by dissolving monobasic potassium phosphate (0.68 g) in 50 mL of water and adjusting the pH to 6.8 with 1 M NaOH. The volume was subsequently made up to 100 mL with water. 200 mg porcine pancreatin (P1625, Sigma Aldrich) was gently dissolved into 20 mL preheated (37 °C) solution (10 g/L) immediately prior to the incubation.

To initiate the incubations, 20 µL of peptide stock solution in 50 % v/v isopropanol was deposited at the bottom of a well plate and 580 µL matrix solution was added to give a final substrate concentration of 10 µM. The incubation was performed at 37 °C with gentle shaking. 70 µL aliquots were removed at 0, 1 and 4 hours and quenched into 210 µL ice-cold precipitant solution (95 % v/v acetonitrile with 0.1 % v/v formic acid). After the last timepoint, the sampling plate was mixed for 10 min on a shaking table and centrifuged 10 min at 2200 g. 70 µL resulting supernatant was diluted with 150 µL water, mixed and centrifugated before analysis by liquid chromatography high resolution mass spectrometry. The zero sample was reinjected after the 4-hour sample to confirm that no drift in instrument sensitivity had occurred during the run. Percent remaining at each timepoint was calculated relative to timepoint zero based on absolute peak areas.

The *in vitro* SIF results (expressed as % peptide remaining after the specified time period) are summarized in **Table 3-3** below. Compounds with a SIF stability (after 4 h) above 70% are considered highly SIF stable. Compounds with a SIF stability (after 4 h) between 30-70% (including 30% and 70%) are considered moderately SIF stable. Compounds with a SIF stability (after 4 h) below 30% are considered SIF unstable. SIF stability values above 100% are due to assay uncertainty and indicate the compound has not degraded. Preferred compounds are compounds that exhibit minimal to no degradation, that is have high (at least 70%) SIF stability, preferably as close to 100% SGF stability.

**Table 3-3: SIF data**

| **Compound** | **SIF (% remaining 1 h)** | **SIF (% remaining 4 h)** |
|---|---|---|
| 3 | 115 | 109 |
| 4 | 96 | 89 |
| 5 | 91 | 41 |
| 6 | 97 | 73 |
| 7 | 103 | 130 |
| 8 | 96 | 105 |
| 9 | 102 | 105 |
| 10 | 34 | 11 |
| 11 | 101 | 102 |
| 12 | 60 | 2 |
| 13 | 84 | 56 |
| 14 | 98 | 75 |
| 20 | 109 | 90 |
| 21 | 53 | 57 |
| 24 | 91 | 89 |
| 25 | 88 | 91 |
| 26 | 89 | 91 |
| 27 | 90 | 85 |
| 28 | 106 | 110 |
| 29 | 91 | 92 |
| 30 | 94 | 94 |
| 31 | 77 | 41 |
| 32 | 94 | 65 |
| 33 | 92 | 69 |
| 34 | 87 | 56 |
| 35 | 110 | 96 |
| 36 | 99 | 55 |
| 37 | 78 | 69 |
| 38 | 82 | 65 |
| 39 | 100 | 85 |
| 40 | 93 | 80 |
| 41 | 100 | 95 |
| 43 | 96 | 92 |
| 47 | 97 | 92 |
| Ref 4 | 0 | 0 |
| Ref 17 | 0 | 0 |
| Ref 37 | 0 | 0 |
| Ref 41 | 83 | 79 |
| Ref 78 | 5 | 0 |
| Ref 80 | 84 | 86 |
| Ref 81 | 99 | 77 |
| Ref 82 | 95 | 77 |
| Ref 120 | 82 | 39 |
| Ref 122 | 73 | 25 |
| I1 (isomer 3) | 0 | 0 |
| I3 (isomer 3) | 0 | 0 |
| I4 (isomer 3) | 0 | 0 |
| I5 (isomer 3) | 78 | 22 |
| Compound C | 92 | 76 |
| Peptide 993 | 47 | 22 |

The *in vitro* SGF results (expressed as % peptide remaining after the specified time period) are summarized in **Table 3-4** below. Compounds with a SGF stability (after 4 h) above 70% are considered highly SGF stable. Compounds with a SGF stability (after 4 h) between 30-70% (including 30% and 70%) are considered moderately SGF stable. Compounds with a SGF stability (after 4 h) below 30% are considered SGF unstable. SGF stability values above 100% are due to assay uncertainty and indicate the compound has not degraded. Preferred compounds are compounds that exhibit minimal to no degradation, that is have high (at least 70%) SGF stability, preferably as close to 100% SGF stability.

**Table 3-4: SGF data**

| **Compound** | **SGF (% remaining 1 h)** | **SGF (% remaining 4 h)** |
|---|---|---|
| 3 | 99 | 104 |
| 4 | 97 | 103 |
| 5 | 99 | 105 |
| 6 | 110 | 108 |
| 7 | 77 | 82 |
| 8 | 91 | 87 |
| 9 | 96 | 95 |
| 10 | 97 | 86 |
| 11 | 98 | 97 |
| 12 | 94 | 93 |
| 13 | 94 | 78 |
| 14 | 93 | 82 |
| 20 | 107 | 103 |
| 21 | 101 | 101 |
| 24 | 93 | 90 |
| 25 | 93 | 88 |
| 26 | 105 | 99 |
| 27 | 101 | 96 |
| 28 | 100 | 110 |
| 29 | 94 | 90 |
| 30 | 92 | 89 |
| 31 | 101 | 99 |
| 32 | 96 | 90 |
| 33 | 98 | 93 |
| 34 | 96 | 95 |
| 35 | 101 | 94 |
| 36 | 96 | 103 |
| 37 | 97 | 102 |
| 38 | 100 | 91 |
| 39 | 99 | 96 |
| 40 | 89 | 89 |
| 41 | 101 | 102 |
| 43 | 105 | 98 |
| 47 | 103 | 85 |
| Ref 4 | 1 | 0 |
| Ref 17 | 0 | 0 |
| Ref 37 | 2 | 1 |
| Ref 41 | 93 | 87 |
| Ref 120 | 84 | 81 |
| Ref 122 | 92 | 90 |
| I1 (isomer 3) | 92 | 94 |
| I3 (isomer 3) | 5 | 0 |
| I4 (isomer 3) | 99 | 99 |
| I5 (isomer 3) | 81 | 54 |
| Compound C | 100 | 100 |

### REFERENCES

Cheng and Prusoff, Biochem. Pharmacol., 1973, 22(23), 3099-3108.
Heinis et al., Nature Biomedical Engineering, 2020, 4, 560-571.
Sgorbati et al., J. Org. Chem, 2021, 86, 9225-9232.
PCT/EP2022/084077
Sayago et al., ACS Med. Chem. Lett., 2018, 9, 912-916.
WO 2016/011208
WO 2017/011820
WO 2018/022937
WO 2018/089693
WO 2018/136646
WO 2020/014646
WO 2021/007433
WO 2021/146441
WO 2021/146458
WO 2023/288017
WO 2023/288019
WO 2023/288028
US 2013/0029907

### CLAUSES

1. A compound of the formula:

Z-R²

wherein
R² is NHR³ wherein R³ is hydrogen or C₁₋₄ alkyl optionally substituted with a pyridyl ring; and
Z is an amino acid sequence of formula I:

X1-X2-X3-X4-X5-X6-X7-X8-X9-X10-X11-X12-X13-X14 (I)

wherein
X1 is absent or is selected from 3-(3-Pyridyl)-Ala or 3-aminopropanoyl;
X2 is selected from the group consisting of 4-aminomethyl-phenylacetyl, 3-(3-Pyridyl)-Ala, and 3-aminopropanoyl;
X3 is selected from the group consisting of Ser, Ala, Phe, and N-Me-Ser;
X4 is Glu or Cys;
X5 is Trp;
X6 is selected from the group consisting of Gin, Q(Me), Q(2Me), Q(pyrrolidin), Ala, Phe, Lys(Ac), Dab(Ac), Cit, and Orn;
X7 is Dab or Cys;
X8 is selected from the group consisting of Y(2-aminoethoxy), homo-Phe, 7-AzaTrp, beta-homo-Trp, and 7-F-Trp;
X9 is selected from the group consisting of 2-Nal, 7-AzaTrp, beta-homo-Trp, and Cyclopropyl-Ala;
X10 is 2-Me-Leu or Aib;
X11 is Glu;
X12 is selected from the group consisting of Dab, iso-Dab, D-Arg, Gly-CF3, D-Gly-CF3, Nle, Gln, His, THP, Ser, D-Ser, 2-Me-Ser, Ser(OMe), and homo-Ser;
X13 is selected from the group consisting of 3-(3-Pyridyl)-Ala, D-3-(3-Pyridyl)-Ala, 2-Me-3-(3-Pyridyl)-Ala, N-Me-3-(3-Pyridyl)-Ala, 3-(3,5-Pyrimidyl)-Ala, His, D-His, and His(Me), or is absent; and
X14 is absent or is selected from the group consisting of Sar, D-His, beta-homo-Leu, and 3-(3-Pyridyl)-Ala;
wherein
(i) the Glu at X11 forms a lactam bridge with the amino acid residue at X1 or at X2 when X1 is absent; and
(ii) X4 and X7 are amino acid residues who together form a lactam bridge or a dithioether bridge;
or a pharmaceutically acceptable salt or solvate thereof;
wherein the compound is not:

| | |
|---|---|
| Ref 80 | [4-Aminomethyl-phenylacetyl](1c)*SC(2a)WQC(2a)[Y(2-aminoethoxy)][2-Nal][2-Me-Leu]E(1c)[{d}R]-[NH2] |
| Ref 81 | [4-Aminomethyl-phenylacetyl](1c)*SC(2a)WQC(2a)[Y(2-aminoethoxy)][2-Nal][2-Me-Leu]E(1c)S-[NH2] |
| Ref 82 | [4-Aminomethyl-phenylacetyl](1c)*SC(2a)WQC(2a)[Y(2-aminoethoxy)][2-Nal][2-Me-Leu]E(1c)[Dab]-[NH2] |
| Ref 120 | [4-Aminomethyl-phenylacetyl](1c)*SC(2a)WQC(2a)[Y(2-aminoethoxy)][2-Nal][2-Me-Leu]E(1c)[Dab][3-(3-Pyridyl)-Ala]-[NH2] |
| Ref 122 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala]-[NH2] |
| Ref 125 | [4-Aminomethyl-phenylacetyl](1c)*SE(2c)WQ[Dab](2c)[Y(2-aminoethoxy)][2-Nal][2-Me-Leu]E(1c)[Dab]-[NH2] |
| Ref 141 | [4-Aminomethyl-phenylacetyl](1c)*[N-Me-Ser]C(2a)WQC(2a)[Y(2-aminoethoxy)][2-Nal][2-Me-Leu]E(1c)[Dab]-[NH2] |
| Ref 146 | [4-Aminomethyl-phenylacetyl](1c)*SE(2c)W[Q(pyrrolidin)][Dab](2c)[Y(2-aminoethoxy)][2-Nal][2-Me-Leu]E(1c)[Dab]-[NH2] |

and pharmaceutically acceptable salts and solvates thereof;
wherein:
* denotes that the (1c), (2a) and (2c) bridges use the peptide backbone amine or carboxylic acid at the *N*- or *C*-terminus, not the side chain amine or carboxylic acid
(1c) denotes the [2,11] lactam bridge; (2a) denotes the [4,7] 1,3-dithio-propan-2-one bridge; (2c) denotes the [4,7] lactam bridge.
2. The compound according to clause 1, wherein the compound is not when X1 is absent; X2 is 4-aminomethyl-phenylacetyl; X3 is Ser or N-Me-Ser; X6 is Gin or Q(pyrrolidin); X8 is Y(2-aminoethoxy); X9 is 2-Nal, X10 is 2-Me-Leu; X12 is Dab, D-Arg, or Ser; X13 is 3-(3-Pyridyl)-Ala or absent; X14 is absent; and R² is NH₂.
3. The compound according to clause 1 or 2, wherein X1 is absent.
4. The compound according to any one of the preceding clauses, wherein X2 is 4-aminomethyl-phenylacetyl.
5. The compound according to any one of the preceding clauses, wherein X3 is Ser or Ala.
6. The compound according to clause 5, wherein X3 is Ser.
7. The compound according to any one of the preceding clauses, wherein X4 is Glu, X7 is Dab, and wherein X4 and X7 together form a lactam bridge.
8. The compound according to any one of the preceding clauses, wherein X6 is Gin.
9. The compound according to any one of the preceding clauses, wherein X8 is Y(2-aminoethoxy).
10. The compound according to any one of the preceding clauses, wherein X9 is 2-Nal.
11. The compound according to any one of the preceding clauses, wherein X10 is 2-Me-Leu.
12. The compound according to any one of the preceding clauses, wherein X12 is Dab.
13. The compound according to any one of the preceding clauses, wherein X13 is 3-(3-Pyridyl)-Ala or is absent.
14. The compound according to clause 13, wherein X13 is 3-(3-Pyridyl)-Ala.
15. The compound according to any one of the preceding clauses, wherein X14 is absent.
16. The compound according to any one of the preceding clauses, wherein R² is NHR³ wherein R³ is hydrogen or C₁₋₄ alkyl.
17. The compound according to clause 16, wherein R² is NH₂.
18. The compound according to clause 16, wherein R² is NHMe.
19. The compound according to any one of clauses 1-6 or 8-18, wherein X4 and X7 are both Cys, and X4 and X7 together form a dithioether bridge, wherein the dithioether bridge between X4 and X7 is of the formula -S-L-Y-L-S-, wherein:
each S is a sulfur atom and is part of the amino acid residue at X4 and X7;
each L is independently C₁₋₄ alkylene; and
Y is either absent or C(=O).
20. The compound according to clause 19, wherein each L is independently C₁₋₂ alkylene.
21. The compound according to clause 20, wherein each L is methylene.
22. The compound according to any one of clauses 19 to 21, wherein Y is C(=O).
23. The compound according to any one of clauses 19 to 22, wherein the dithioether bridge between X4 and X7 is of the formula -SCH₂C(=O)CH₂S-, wherein each S is a sulfur atom and is part of the amino acid residue at X4 and X7.
24. The compound according to any one of the preceding clauses, wherein X1 is absent and X2 is 4-aminomethyl-phenylacetyl.
25. The compound according to any one of the preceding clauses, wherein X1 is absent; X2 is 4-aminomethyl-phenylacetyl; X8 is Y(2-aminoethoxy); X9 is 2-Nal; and X10 is 2-Me-Leu.
26. The compound according to any one of the preceding clauses, wherein X1 is absent; X2 is 4-aminomethyl-phenylacetyl; X3 is Ser or Ala; X8 is Y(2-aminoethoxy); X9 is 2-Nal; and X10 is 2-Me-Leu.
27. The compound according to any one of the preceding clauses, wherein X1 is absent; X2 is 4-aminomethyl-phenylacetyl; X3 is Ser or Ala; X6 is Gin; X8 is Y(2-aminoethoxy); X9 is 2-Nal; and X10 is 2-Me-Leu.
28. The compound according to any one of the preceding clauses, wherein X13 is 3-(3-Pyridyl)-Ala and X14 is absent.
29. The compound according to any one of the preceding clauses, wherein X13 is 3-(3-Pyridyl)-Ala, X14 is absent, and R² is NHMe.
30. The compound according to clause 1 wherein Z is an amino acid sequence selected from the group consisting of a sequence listed in **Table 1-1 a.**
31. A compound according to clause 1 which is selected from a compound from **Table 1-1,** or a pharmaceutically acceptable salt or solvate thereof.
32. A pharmaceutical composition comprising a compound according to any one of the preceding clauses in combination with a pharmaceutically acceptable carrier, excipient or vehicle.
33. A method for the synthesis of a compound according to any one of clauses 1 to 31, comprising synthesising the analogue by solid-phase or liquid-phase peptide synthesis methodology, optionally isolating and/or purifying the final product, and optionally further comprising the step of forming an amide bond between the amino acid residues at position X1 or at position X2 when X1 is absent and at position X11, and optionally further comprising the step of forming an amide bond or forming two thioether bonds with a linker between the amino acid residues at positions X4 and X7.
34. A compound according to any one of clauses 1 to 31, or a pharmaceutical composition according to clause 32, for use in a method of medical treatment.
35. A compound according to any one of clauses 1 to 31, or a pharmaceutical composition according to clause 32, for use in a method of prevention or treatment of inflammatory bowel disease (IBD), Crohn's Disease, ulcerative colitis, and psoriasis.
36. The compound or pharmaceutical composition for use according to clause 35, wherein the compound or pharmaceutical composition is for use in a method of prevention or treatment of inflammatory bowel disease (IBD) and/or psoriasis.
37. Use of a compound according to any one of clauses 1 to 31, or a pharmaceutical composition according to clause 32, in the manufacture of a medicament for the prevention or treatment of inflammatory bowel disease (IBD), Crohn's Disease, ulcerative colitis, and psoriasis.
38. The use of the compound or pharmaceutical composition according to clause 37, wherein the use of the compound or pharmaceutical compositions is in the manufacture of a medicament for the prevention or treatment of inflammatory bowel disease (IBD) and/or psoriasis.
39. A method of prevention or treatment of inflammatory bowel disease (IBD), Crohn's Disease, ulcerative colitis, and psoriasis; which comprises administering to a subject an effective amount of a compound according to any one of clauses 1 to 31, or a pharmaceutical composition according to clause 32.
40. The method of prevention or treatment according to clause 39, wherein the method of prevention or treatment is for inflammatory bowel disease (IBD) and/or psoriasis.

## Claims

1. A compound of the formula:
Z-R²
wherein
R² is NHR³ wherein R³ is hydrogen or C₁₋₄ alkyl optionally substituted with a pyridyl ring; and
Z is an amino acid sequence of formula I:
X1-X2-X3-X4-X5-X6-X7-X8-X9-X10-X11-X12-X13-X14 (I)
wherein
X1 is absent or is selected from 3-(3-Pyridyl)-Ala or 3-aminopropanoyl;
X2 is selected from the group consisting of 4-aminomethyl-phenylacetyl, 3-(3-Pyridyl)-Ala, and 3-aminopropanoyl;
X3 is selected from the group consisting of Ser, Ala, Phe, and N-Me-Ser;
X4 is Glu or Cys;
X5 is Trp;
X6 is selected from the group consisting of Gin, Q(Me), Q(2Me), Q(pyrrolidin), Ala, Phe, Lys(Ac), Dab(Ac), Cit, and Orn;
X7 is Dab or Cys;
X8 is selected from the group consisting of Y(2-aminoethoxy), homo-Phe, 7-AzaTrp, beta-homo-Trp, and 7-F-Trp;
X9 is selected from the group consisting of 2-Nal, 7-AzaTrp, beta-homo-Trp, and Cyclopropyl-Ala;
X10 is 2-Me-Leu or Aib;
X11 is Glu;
X12 is selected from the group consisting of Dab, iso-Dab, D-Arg, Gly-CF3, D-Gly-CF3, Nle, Gln, His, THP, Ser, D-Ser, 2-Me-Ser, Ser(OMe), and homo-Ser;
X13 is selected from the group consisting of 3-(3-Pyridyl)-Ala, D-3-(3-Pyridyl)-Ala, 2-Me-3-(3-Pyridyl)-Ala, N-Me-3-(3-Pyridyl)-Ala, 3-(3,5-Pyrimidyl)-Ala, His, D-His, and His(Me), or is absent; and
X14 is absent or is selected from the group consisting of Sar, D-His, beta-homo-Leu, and 3-(3-Pyridyl)-Ala;
wherein
(i) the Glu at X11 forms a lactam bridge with the amino acid residue at X1 or at X2 when X1 is absent; and
(ii) X4 and X7 are amino acid residues who together form a lactam bridge or a dithioether bridge;
or a pharmaceutically acceptable salt or solvate thereof;
wherein the compound is not:
| | |
|---|---|
| Ref 80 | [4-Aminomethyl-phenylacetyl](1c)*SC(2a)WQC(2a)[Y(2-aminoethoxy)][2-Nal][2-Me-Leu]E(1c)[{d}R]-[NH2] |
| Ref 81 | [4-Aminomethyl-phenylacetyl](1c)*SC(2a)WQC(2a)[Y(2-aminoethoxy)][2-Nal][2-Me-Leu]E(1c)S-[NH2] |
| Ref 82 | [4-Aminomethyl-phenylacetyl](1c)*SC(2a)WQC(2a)[Y(2-aminoethoxy)][2-Nal][2-Me-Leu]E(1c)[Dab]-[NH2] |
| Ref 120 | [4-Aminomethyl-phenylacetyl](1c)*SC(2a)WQC(2a)[Y(2-aminoethoxy)][2-Nal][2-Me-Leu]E(1c)[Dab][3-(3-Pyridyl)-Ala]-[NH2] |
| Ref 122 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala]-[NH2] |
| Ref 125 | [4-Aminomethyl-phenylacetyl](1c)*SE(2c)WQ[Dab](2c)[Y(2-aminoethoxy)][2-Nal][2-Me-Leu]E(1c)[Dab]-[NH2] |
| Ref 141 | [4-Aminomethyl-phenylacetyl](1c)*[N-Me-Ser]C(2a)WQC(2a)[Y(2-aminoethoxy)][2-Nal][2-Me-Leu]E(1c)[Dab]-[NH2] |
| Ref 146 | [4-Aminomethyl-phenylacetyl](1c)*SE(2c)W[Q(pyrrolidin)][Dab](2c)[Y(2-aminoethoxy)][2-Nal][2-Me-Leu]E(1c)[Dab]-[NH2] |
and pharmaceutically acceptable salts and solvates thereof;
wherein:
* denotes that the (1c), (2a) and (2c) bridges use the peptide backbone amine or carboxylic acid at the *N*- or *C*-terminus, not the side chain amine or carboxylic acid
(1c) denotes the [2,11] lactam bridge; (2a) denotes the [4,7] 1,3-dithio-propan-2-one bridge; (2c) denotes the [4,7] lactam bridge.

2. The compound according to claim 1, wherein the compound is not when X1 is absent; X2 is 4-aminomethyl-phenylacetyl; X3 is Ser or N-Me-Ser; X6 is Gin or Q(pyrrolidin); X8 is Y(2-aminoethoxy); X9 is 2-Nal, X10 is 2-Me-Leu; X12 is Dab, D-Arg, or Ser; X13 is 3-(3-Pyridyl)-Ala or absent; X14 is absent; and R² is NH₂.

3. The compound according to claim 1 or 2, wherein X1 is absent;
optionally wherein X2 is 4-aminomethyl-phenylacetyl;
optionally wherein X3 is Ser or Ala, optionally wherein X3 is Ser;
optionally wherein X6 is Gin.

4. The compound according to any one of the preceding claims, wherein X8 is Y(2-aminoethoxy);
optionally wherein X9 is 2-Nal.
optionally wherein X10 is 2-Me-Leu.
optionally wherein X12 is Dab.

5. The compound according to any one of the preceding claims, wherein X13 is 3-(3-Pyridyl)-Ala or is absent; optionally wherein X13 is 3-(3-Pyridyl)-Ala;
optionally wherein X14 is absent.

6. The compound according to any one of the preceding claims, wherein R² is NHR³ wherein R³ is hydrogen or C₁₋₄ alkyl; optionally wherein R² is NH₂ or NHMe.

7. The compound according to any one of the preceding claims, wherein X4 is Glu, X7 is Dab, and wherein X4 and X7 together form a lactam bridge.

8. The compound according to any one of claims 1-6, wherein X4 and X7 are both Cys, and X4 and X7 together form a dithioether bridge, wherein the dithioether bridge between X4 and X7 is of the formula -S-L-Y-L-S-, wherein:
each S is a sulfur atom and is part of the amino acid residue at X4 and X7;
each L is independently C₁₋₄ alkylene; and
Y is either absent or C(=O);
optionally wherein each L is independently C₁₋₂ alkylene, optionally wherein each L is methylene;
optionally wherein Y is C(=O);
optionally wherein the dithioether bridge between X4 and X7 is of the formula - SCH₂C(=O)CH₂S-, wherein each S is a sulfur atom and is part of the amino acid residue at X4 and X7.

9. The compound according to any one of the preceding claims, wherein:
X1 is absent and X2 is 4-aminomethyl-phenylacetyl; or
X1 is absent; X2 is 4-aminomethyl-phenylacetyl; X8 is Y(2-aminoethoxy); X9 is 2-Nal; and X10 is 2-Me-Leu; or
X1 is absent; X2 is 4-aminomethyl-phenylacetyl; X3 is Ser or Ala; X8 is Y(2-aminoethoxy); X9 is 2-Nal; and X10 is 2-Me-Leu; or
X1 is absent; X2 is 4-aminomethyl-phenylacetyl; X3 is Ser or Ala; X6 is Gin; X8 is Y(2-aminoethoxy); X9 is 2-Nal; and X10 is 2-Me-Leu; or
X13 is 3-(3-Pyridyl)-Ala and X14 is absent; or
X13 is 3-(3-Pyridyl)-Ala, X14 is absent, and R² is NHMe.

10. The compound according to claim 1 wherein Z is an amino acid sequence selected from the group consisting of:
| **SEQ ID NO:** | **Sequence** |
|---|---|
| SEQ ID NO: 1 | [3-Aminopropanoyl](4c)*[3-(3-Pyridyl)-Ala]SC(2a)WQC(2a)[Y(2-aminoethoxy)][2-Nal][2-Me-Leu]E(4c)[{d}R] |
| SEQ ID NO: 2 | [3-(3-Pyridyl)-Ala](4c)*[3-Aminopropanoyl]SC(2a)WQC(2a)[Y(2-aminoethoxy)][2-Nal][2-Me-Leu]E(4c)[{d}R] |
| SEQ ID NO: 3 | [4-Aminomethyl-phenylacetyl](1c)*SE(2c)WQ[Dab](2c)[Y(2-aminoethoxy)][2-Nal][2-Me-Leu]E(1c)[Dab][2-Me-3-(3-Pyridyl)-Ala] |
| SEQ ID NO: 4 | [4-Aminomethyl-phenylacetyl](1c)*SE(2c)WQ[Dab](2c)[Y(2-aminoethoxy)][2-Nal][2-Me-Leu]E(1c)[Dab][N-Me-3-(3-Pyridyl)-Ala] |
| SEQ ID NO: 5 | [4-Aminomethyl-phenylacetyl](1c)*SE(2c)WQ[Dab](2c)[Y(2-aminoethoxy)][2-Nal][2-Me-Leu]E(1c)[Dab][{d}3-(3-Pyridyl)-Ala] |
| SEQ ID NO: 6 | [4-Aminomethyl-phenylacetyl](1c)*SE(2c)WQ[Dab](2c)[Y(2-aminoethoxy)][2-Nal][2-Me-Leu]E(1c)[Dab] |
| SEQ ID NO: 7 | [4-Aminomethyl-phenylacetyl](1c)*SE(2c)WQ[Dab](2c)[Y(2-aminoethoxy)][2-Nal][2-Me-Leu]E(1c)[Dab] |
| SEQ ID NO: 8 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala] |
| SEQ ID NO: 9 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala][Sar] |
| SEQ ID NO: 10 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala][{d}H] |
| SEQ ID NO: 11 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala][beta-hLeu] |
| SEQ ID NO: 12 | [4-Aminomethyl-phenylacetyl](1c)*SE(2c)WQ[Dab](2c)[Y(2-aminoethoxy)][2-Nal][2-Me-Leu]E(1c)[Dab]H[3-(3-Pyridyl)-Ala] |
| SEQ ID NO: 13 | [4-Aminomethyl-phenylacetyl](1c)*SE(2c)WQ[Dab](2c)[Y(2-aminoethoxy)][2-Nal][2-Me-Leu]E(1c)[Dab][{d}H] |
| SEQ ID NO: 14 | [4-Aminomethyl-phenylacetyl](1c)*SE(2c)WQ[Dab](2c)[Y(2-aminoethoxy)][2-Nal][2-Me-Leu]E(1c)[Dab][H(Me)] |
| SEQ ID NO: 15 | [4-Aminomethyl-phenylacetyl](1c)*SE(2c)WQ[Dab](2c)[Y(2-aminoethoxy)][2-Nal][2-Me-Leu]E(1c)[lso-Dab][2-Me-3-(3-Pyridyl)-Ala] |
| SEQ ID NO: 16 | [4-Aminomethyl-phenylacetyl](1c)*SE(2c)WQ[Dab](2c)[Y(2-aminoethoxy)][2-Nal][2-Me-Leu]E(1c)[{d}Gly-CF3][3-(3-Pyridyl)-Ala] |
| SEQ ID NO: 17 | [4-Aminomethyl-phenylacetyl](1c)*SE(2c)WQ[Dab](2c)[Y(2-aminoethoxy)][2-Nal][2-Me-Leu]E(1c)[Gly-CF3][3-(3-Pyridyl)-Ala] |
| SEQ ID NO: 18 | [4-Aminomethyl-phenylacetyl](1c)*[N-Me-Ser]E(2c)WQ[Dab](2c)[Y(2-aminoethoxy)][2-Nal][2-Me-Leu]E(1c)[Dab][3-(3-Pyridyl)-Ala] |
| SEQ ID NO: 19 | [4-Aminomethyl-phenylacetyl](1c)*SE(2c)WQ[Dab](2c)[Y(2-aminoethoxy)][2-Nal][2-Me-Leu]E(1c)[Dab][3-(3,5-Pyrimidyl)-Ala] |
| SEQ ID NO: 20 | [4-Aminomethyl-phenylacetyl](1c)*AE(2c)WQ[Dab](2c)[Y(2-aminoethoxy)][2-Nal][2-Me-Leu]E(1c)[Dab][3-(3-Pyridyl)-Ala] |
| SEQ ID NO: 21 | [4-Aminomethyl-phenylacetyl](1c)*FE(2c)WQ[Dab](2c)[Y(2-aminoethoxy)][2-Nal][2-Me-Leu]E(1c)[Dab][3-(3-Pyridyl)-Ala] |
| SEQ ID NO: 22 | [4-Aminomethyl-phenylacetyl](1c)*SE(2c)WA[Dab](2c)[Y(2-aminoethoxy)][2-Nal][2-Me-Leu]E(1c)[Dab][3-(3-Pyridyl)-Ala] |
| SEQ ID NO: 23 | [4-Aminomethyl-phenylacetyl](1c)*SE(2c)WF[Dab](2c)[Y(2-aminoethoxy)][2-Nal][2-Me-Leu]E(1c)[Dab][3-(3-Pyridyl)-Ala] |
| SEQ ID NO: 24 | [4-Aminomethyl-phenylacetyl](1c)*SE(2c)W[K(Ac)][Dab](2c)[Y(2-aminoethoxy)][2-Nal][2-Me-Leu]E(1c)[Dab][3-(3-Pyridyl)-Ala] |
| SEQ ID NO: 25 | [4-Aminomethyl-phenylacetyl](1c)*SE(2c)W[Dab(Ac)][Dab](2c)[Y(2-aminoethoxy)][2-Nal][2-Me-Leu]E(1c)[Dab][3-(3-Pyridyl)-Ala] |
| SEQ ID NO: 26 | [4-Aminomethyl-phenylacetyl](1c)*SE(2c)W[Cit][Dab](2c)[Y(2-aminoethoxy)][2-Nal][2-Me-Leu]E(1c)[Dab][3-(3-Pyridyl)-Ala] |
| SEQ ID NO: 27 | [4-Aminomethyl-phenylacetyl](1c)*SE(2c)W[Q(pyrrolidin)][Dab](2c)[Y(2-aminoethoxy)][2-Nal][2-Me-Leu]E(1c)[Dab][3-(3-Pyridyl)-Ala] |
| SEQ ID NO: 28 | [4-Aminomethyl-phenylacetyl](1c)*SE(2c)W[Orn][Dab](2c)[Y(2-aminoethoxy)][2-Nal][2-Me-Leu]E(1c)[Dab][3-(3-Pyridyl)-Ala] |
| SEQ ID NO: 29 | [4-Aminomethyl-phenylacetyl](1c)*SE(2c)W[Q(Me)][Dab](2c)[Y(2-aminoethoxy)][2-Nal][2-Me-Leu]E(1c)[Dab][3-(3-Pyridyl)-Ala] |
| SEQ ID NO: 30 | [4-Aminomethyl-phenylacetyl](1c)*SE(2c)W[Q(2Me)][Dab](2c)[Y(2-aminoethoxy)][2-Nal][2-Me-Leu]E(1c)[Dab][3-(3-Pyridyl)-Ala] |
| SEQ ID NO: 31 | [4-Aminomethyl-phenylacetyl](1c)*SE(2c)WQ[Dab](2c)[Y(2-aminoethoxy)][2-Nal][2-Me-Leu]E(1c)S[3-(3-Pyridyl)-Ala] |
| SEQ ID NO: 32 | [4-Aminomethyl-phenylacetyl](1c)*SE(2c)WQ[Dab](2c)[Y(2-aminoethoxy)][2-Nal][2-Me-Leu]E(1c)[Nle][3-(3-Pyridyl)-Ala] |
| SEQ ID NO: 33 | [4-Aminomethyl-phenylacetyl](1c)*SE(2c)WQ[Dab](2c)[Y(2-aminoethoxy)][2-Nal][2-Me-Leu]E(1c)Q[3-(3-Pyridyl)-Ala] |
| SEQ ID NO: 34 | [4-Aminomethyl-phenylacetyl](1c)*SE(2c)WQ[Dab](2c)[Y(2-aminoethoxy)][2-Nal][2-Me-Leu]E(1c)H[3-(3-Pyridyl)-Ala] |
| SEQ ID NO: 35 | [4-Aminomethyl-phenylacetyl](1c)*SE(2c)WQ[Dab](2c)[Y(2-aminoethoxy)][2-Nal][2-Me-Leu]E(1c)[THP][3-(3-Pyridyl)-Ala] |
| SEQ ID NO: 36 | [4-Aminomethyl-phenylacetyl](1c)*SE(2c)WQ[Dab](2c)[Y(2-aminoethoxy)][2-Nal][2-Me-Leu]E(1c)[hSer][3-(3-Pyridyl)-Ala] |
| SEQ ID NO: 37 | [4-Aminomethyl-phenylacetyl](1c)*SE(2c)WQ[Dab](2c)[Y(2-aminoethoxy)][2-Nal][2-Me-Leu]E(1c)[Ser(OMe)][3-(3-Pyridyl)-Ala] |
| SEQ ID NO: 38 | [4-Aminomethyl-phenylacetyl](1c)*SE(2c)WQ[Dab](2c)[Y(2-aminoethoxy)][2-Nal][2-Me-Leu]E(1c)[{d}S][3-(3-Pyridyl)-Ala] |
| SEQ ID NO: 39 | [4-Aminomethyl-phenylacetyl](1c)*SE(2c)WQ[Dab](2c)[Y(2-aminoethoxy)][2-Nal][2-Me-Leu]E(1c)[2-Me-Ser][3-(3-Pyridyl)-Ala] |
| SEQ ID NO: 40 | [4-Aminomethyl-phenylacetyl](1c)*AE(2c)WQ[Dab](2c)[Y(2-aminoethoxy)][2-Nal][2-Me-Leu]E(1c)S[3-(3-Pyridyl)-Ala] |
| SEQ ID NO: 41 | [4-Aminomethyl-phenylacetyl](1c)*AE(2c)WQ[Dab](2c)[hPhe][2-Nal][Aib]E(1c)[Dab][3-(3-Pyridyl)-Ala] |
| SEQ ID NO: 42 | [4-Aminomethyl-phenylacetyl](1c)*AE(2c)WQ[Dab](2c)[Y(2-aminoethoxy)][Cyclopropyl-Ala][Aib]E(1c)[Dab][3-(3-Pyridyl)-Ala] |
| SEQ ID NO: 43 | [4-Aminomethyl-phenylacetyl](1c)*AE(2c)WQ[Dab](2c)[7-AzaTrp][2-Nal][Aib]E(1c)[Dab][3-(3-Pyridyl)-Ala] |
| SEQ ID NO: 44 | [4-Aminomethyl-phenylacetyl](1c)*AE(2c)WQ[Dab](2c)[Y(2-aminoethoxy)][7-AzaTrp][Aib]E(1c)[Dab][3-(3-Pyridyl)-Ala] |
| SEQ ID NO: 45 | [4-Aminomethyl-phenylacetyl](1c)*AE(2c)WQ[Dab](2c)[beta-hTrp][2-Nal][Aib]E(1c)[Dab][3-(3-Pyridyl)-Ala] |
| SEQ ID NO: 46 | [4-Aminomethyl-phenylacetyl](1c)*AE(2c)WQ[Dab](2c)[Y(2-aminoethoxy)][beta-hTrp][Aib]E(1c)[Dab][3-(3-Pyridyl)-Ala] |
| SEQ ID NO: 47 | [4-Aminomethyl-phenylacetyl](1c)*AE(2c)WQ[Dab](2c)[7-F-Trp][2-Nal][Aib]E(1c)[Dab][3-(3-Pyridyl)-Ala] |
wherein:
* denotes that the (1c), (2a), (2c), and (4c) bridges use the peptide backbone amine or carboxylic acid at the *N*- or *C*-terminus, not the side chain amine or carboxylic acid
(1c) denotes the [2,11] lactam bridge; (2a) denotes the [4,7] 1,3-dithio-propan-2-one bridge; (2c) denotes the [4,7] lactam bridge; (4c) denotes the [1,11] lactam bridge.

11. A compound according to claim 1 which is selected from:
| **Compound** | **Structure** |
|---|---|
| 1 | [3-Aminopropanoyl](4c)*[3-(3-Pyridyl)-Ala]SC(2a)WQC(2a)[Y(2-aminoethoxy)][2-Nal][2-Me-Leu]E(4c)[{d}R]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CSCC(=O)CSC[C@H](NC(=O)[C@H](CO)NC(= O)[C@H](Cc3cccnc3)NC(=O)CCNC(=O)CC[C@@H](C(=O)N[C@H](CCCNC (=N)N)C(N)=O)NC1=O)C(=O)N[C@@H](Cc1c[nH]c3ccccc13)C(=O)N[C@@ H](CCC(N)=O)C(=O)N2 |
| 2 | [3-(3-Pyridyl)-Ala](4c)*[3-Aminopropanoyl]SC(2a)WQC(2a)[Y(2-aminoethoxy)][2-Nal][2-Me-Leu]E(4c)[{d}R]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CSCC(=O)CSC[C@H](NC(=O)[C@H](CO)NC(= O)CCNC(=O)[C@H](Cc3cccnc3)NC(=O)CC[C@@H](C(=O)N[C@H](CCCNC (=N)N)C(N)=O)NC1=O)C(=O)N[C@@H](Cc1c[nH]c3ccccc13)C(=O)N[C@@ H](CCC(N)=O)C(=O)N2 |
| 3 | [4-Aminomethyl-phenylacetyl](1c)*SE(2c)WQ[Dab](2c)[Y(2-aminoethoxy)][2-Nal][2-Me-Leu]E(1c)[Dab][2-Me-3-(3-Pyridyl)-Ala]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)N[C@@](C)( Cc3cccnc3)C(N)=O)NC1=O)C(=O)N[C@@H](Cc1c[nH]c3ccccc13)C(=O)N[C @@H](CCC(N)=O)C(=O)N2 |
| 4 | [4-Aminomethyl-phenylacetyl](1c)*SE(2c)WQ[Dab](2c)[Y(2-aminoethoxy)][2-Nal][2-Me-Leu]E(1c)[Dab][N-Me-3-(3-Pyridyl)-Ala]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)N(C)[C@@ H](Cc3cccnc3)C(N)=O)NC1=O)C(=O)N[C@@H](Cc1c[nH]c3ccccc13)C(=O) N[C@@H](CCC(N)=O)C(=O)N2 |
| 5 | [4-Aminomethyl-phenylacetyl](1c)*SE(2c)WQ[Dab](2c)[Y(2-aminoethoxy)][2-Nal][2-Me-Leu]E(1c)[Dab][{d}3-(3-Pyridyl)-Ala]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)N[C@H](Cc 3cccnc3)C(N)=O)NC1=O)C(=O)N[C@@H](Cc1c[nH]c3ccccc13)C(=O)N[C@ @H](CCC(N)=O)C(=O)N2 |
| 6 | [4-Aminomethyl-phenylacetyl](1c)*SE(2c)WQ[Dab](2c)[Y(2-aminoethoxy)][2-Nal][2-Me-Leu]E(1c)[Dab]-[NH-(4-(pyridin-3-yl)butanyl)] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)NCCCCc3cc cnc3)NC1=O)C(=O)N[C@@H](Cc1c[nH]c3ccccc13)C(=O)N[C@@H](CCC(N )=O)C(=O)N2 |
| 7 | [4-Aminomethyl-phenylacetyl](1c)*SE(2c)WQ[Dab](2c)[Y(2-aminoethoxy)][2-Nal][2-Me-Leu]E(1c)[Dab]-[NH-(2-(pyridin-3-yl)ethyl)] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)NCCc3cccn c3)NC1=O)C(=O)N[C@@H](Cc1c[nH]c3ccccc13)C(=O)N[C@@H](CCC(N)= O)C(=O)N2 |
| 8 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](CCC(N)=O)NC(=O)[C@H](Cc3c[nH]c4ccccc34)NC2=O)C(=O) N[C@@H](Cc2ccc(OCCN)cc2)C(=O)N[C@@H](Cc2ccc3ccccc3c2)C(=O)N[ C@@](C)(CC(C)C)C(=O)N1 |
| 9 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala][Sar]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)N[C@@H]( Cc3cccnc3)C(=O)N(C)CC(N)=O)NC1=O)C(=O)N[C@@H](Cc1c[nH]c3ccccc 13)C(=O)N[C@@H](CCC(N)=O)C(=O)N2 |
| 10 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala][{d}H]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)N[C@@H]( Cc3cccnc3)C(=O)N[C@H](Cc3cnc[nH]3)C(N)=O)NC1=O)C(=O)N[C@@H](C c1c[nH]c3ccccc13)C(=O)N[C@@H](CCC(N)=O)C(=O)N2 |
| 11 | [4-Aminomethyl-phenylacetyl](1c)*SE(2c)WQ[Dab](2c)[Y(2-aminoethoxy)][2-Nal][2-Me-Leu]E(1c)[Dab][3-(3-Pyridyl)-Ala][beta-hLeu]-[NH2] |
| | CC(C)C[C@@H](CC(N)=O)NC(=O)[C@H](Cc1cccnc1)NC(=O)[C@H](CCN) NC(=O)[C@@H]1CCC(=O)NCc2ccc(cc2)CC(=O)N[C@@H](CO)C(=O)N[C@ H]2CCC(=O)NCC[C@H](NC(=O)[C@H](CCC(N)=O)NC(=O)[C@H](Cc3c[nH] c4ccccc34)NC2=O)C(=O)N[C@@H](Cc2ccc(OCCN)cc2)C(=O)N[C@@H](C c2ccc3ccccc3c2)C(=O)N[C@@](C)(CC(C)C)C(=O)N1 |
| 12 | [4-Aminomethyl-phenylacetyl](1c)*SE(2c)WQ[Dab](2c)[Y(2-aminoethoxy)][2-Nal][2-Me-Leu]E(1c)[Dab]H[3-(3-Pyridyl)-Ala]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)N[C@@H]( Cc3cnc[nH]3)C(=O)N[C@@H](Cc3cccnc3)C(N)=O)NC1=O)C(=O)N[C@@H] (Cc1c[nH]c3ccccc13)C(=O)N[C@@H](CCC(N)=O)C(=O)N2 |
| 13 | [4-Aminomethyl-phenylacetyl](1c)*SE(2c)WQ[Dab](2c)[Y(2-aminoethoxy)][2-Nal][2-Me-Leu]E(1c)[Dab][{d}H]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)N[C@H](Cc 3cnc[nH]3)C(N)=O)NC1=O)C(=O)N[C@@H](Cc1c[nH]c3ccccc13)C(=O)N[C @@H](CCC(N)=O)C(=O)N2 |
| 14 | [4-Aminomethyl-phenylacetyl](1c)*SE(2c)WQ[Dab](2c)[Y(2-aminoethoxy)][2-Nal][2-Me-Leu]E(1c)[Dab][H(Me)]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)N[C@@H](CCN)C(=O)N[C@@H]( Cc3cn(C)cn3)C(N)=O)NC1=O)C(=O)N[C@@H](Cc1c[nH]c3ccccc13)C(=O)N [C@@H](CCC(N)=O)C(=O)N2 |
| 15 | [4-Aminomethyl-phenylacetyl](1c)*SE(2c)WQ[Dab](2c)[Y(2-aminoethoxy)][2-Nal][2-Me-Leu]E(1c)[Iso-Dab][2-Me-3-(3-Pyridyl)-Ala]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)NCC[C@H](N)C(=O)N[C@@](C)(C c3cccnc3)C(N)=O)NC1=O)C(=O)N[C@@H](Cc1c[nH]c3ccccc13)C(=O)N[C @@H](CCC(N)=O)C(=O)N2 |
| 16 | [4-Aminomethyl-phenylacetyl](1c)*SE(2c)WQ[Dab](2c)[Y(2-aminoethoxy)][2-Nal][2-Me-Leu]E(1c)[{d}Gly-CF3][3-(3-Pyridyl)-Ala]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)NC[C@@H](N[C@@H](Cc3cccnc3 )C(N)=O)C(F)(F)F)NC1=O)C(=O)N[C@@H](Cc1c[nH]c3ccccc13)C(=O)N[C @@H](CCC(N)=O)C(=O)N2 |
| 17 | [4-Aminomethyl-phenylacetyl](1c)*SE(2c)WQ[Dab](2c)[Y(2-aminoethoxy)][2-Nal][2-Me-Leu]E(1c)[Gly-CF3][3-(3-Pyridyl)-Ala]-[NH2] |
| | CC(C)C[C@]1(C)NC(=O)[C@H](Cc2ccc3ccccc3c2)NC(=O)[C@H](Cc2ccc(O CCN)cc2)NC(=O)[C@@H]2CCNC(=O)CC[C@H](NC(=O)[C@H](CO)NC(=O) Cc3ccc(cc3)CNC(=O)CC[C@@H](C(=O)NC[C@H](N[C@@H](Cc3cccnc3)C (N)=O)C(F)(F)F)NC1=O)C(=O)N[C@@H](Cc1c[nH]c3ccccc13)C(=O)N[C@ @H](CCC(N)=O)C(=O)N2 |
| 18 | [4-Aminomethyl-phenylacetyl](1c)*[N-Me-Ser]E(2c)WQ[Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N(C)[C@@H](CO)C(=O)N[C@H]2CCC(=O)NCC[C@H ](NC(=O)[C@H](CCC(N)=O)NC(=O)[C@H](Cc3c[nH]c4ccccc34)NC2=O)C(= O)N[C@@H](Cc2ccc(OCCN)cc2)C(=O)N[C@@H](Cc2ccc3ccccc3c2)C(=O) N[C@@](C)(CC(C)C)C(=O)N1 |
| 19 | [4-Aminomethyl-phenylacetyl](1c)*SE(2c)WQ[Dab](2c)[Y(2-aminoethoxy)][2-Nal][2-Me-Leu]E(1c)[Dab][3-(3,5-Pyrimidyl)-Ala]-[NHMe] |
| | CNC(=O)[C@H](Cc1cncnc1)NC(=O)[C@H](CCN)NC(=O)[C@@H]1CCC(=O )NCc2ccc(cc2)CC(=O)N[C@@H](CO)C(=O)N[C@H]2CCC(=O)NCC[C@H]( NC(=O)[C@H](CCC(N)=O)NC(=O)[C@H](Cc3c[nH]c4ccccc34)NC2=O)C(=O )N[C@@H](Cc2ccc(OCCN)cc2)C(=O)N[C@@H](Cc2ccc3ccccc3c2)C(=O)N[ C@@](C)(CC(C)C)C(=O)N1 |
| 20 | [4-Aminomethyl-phenylacetyl](1c)*AE(2c)WQ[Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](Cc2ccc(OCCN)cc2)C(=O)N[C@@H](Cc2ccc3ccccc3c2)C(=O)N[C @@](C)(CC(C)C)C(=O)N1 |
| 21 | [4-Aminomethyl-phenylacetyl](1c)*FE(2c)WQ[Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](Cc2ccccc2)C(=O)N[C@H]2CCC(=O)NCC[C @H](NC(=O)[C@H](CCC(N)=O)NC(=O)[C@H](Cc3c[nH]c4ccccc34)NC2=O) C(=O)N[C@@H](Cc2ccc(OCCN)cc2)C(=O)N[C@@H](Cc2ccc3ccccc3c2)C( =O)N[C@@](C)(CC(C)C)C(=O)N1 |
| 22 | [4-Aminomethyl-phenylacetyl](1c)*SE(2c)WA[Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](C)NC(=O)[C@H](Cc3c[nH]c4ccccc34)NC2=O)C(=O)N[C@@H] (Cc2ccc(OCCN)cc2)C(=O)N[C@@H](Cc2ccc3ccccc3c2)C(=O)N[C@@](C)( CC(C)C)C(=O)N1 |
| 23 | [4-Aminomethyl-phenylacetyl](1c)*SE(2c)WF[Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](Cc3ccccc3)NC(=O)[C@H](Cc3c[nH]c4ccccc34)NC2=O)C(=O) N[C@@H](Cc2ccc(OCCN)cc2)C(=O)N[C@@H](Cc2ccc3ccccc3c2)C(=O)N[ C@@](C)(CC(C)C)C(=O)N1 |
| 24 | [4-Aminomethyl-phenylacetyl](1c)*SE(2c)W[K(Ac)][Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](CCCCNC(C)=O)NC(=O)[C@H](Cc3c[nH]c4ccccc34)NC2=O)C( =O)N[C@@H](Cc2ccc(OCCN)cc2)C(=O)N[C@@H](Cc2ccc3ccccc3c2)C(=O )N[C@@](C)(CC(C)C)C(=O)N1 |
| 25 | [4-Aminomethyl-phenylacetyl](1c)*SE(2c)W[Dab(Ac)][Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](CCNC(C)=O)NC(=O)[C@H](Cc3c[nH]c4ccccc34)NC2=O)C(=O )N[C@@H](Cc2ccc(OCCN)cc2)C(=O)N[C@@H](Cc2ccc3ccccc3c2)C(=O)N[ C@@](C)(CC(C)C)C(=O)N1 |
| 26 | [4-Aminomethyl-phenylacetyl](1c)*SE(2c)W[Cit][Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](CCCNC(N)=O)NC(=O)[C@H](Cc3c[nH]c4ccccc34)NC2=O)C(= O)N[C@@H](Cc2ccc(OCCN)cc2)C(=O)N[C@@H](Cc2ccc3ccccc3c2)C(=O) N[C@@](C)(CC(C)C)C(=O)N1 |
| 27 | [4-Aminomethyl-phenylacetyl](1c)*SE(2c)W[Q(pyrrolidin)][Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](CCC(=O)N3CCCC3)NC(=O)[C@H](Cc3c[nH]c4ccccc34)NC2= O)C(=O)N[C@@H](Cc2ccc(OCCN)cc2)C(=O)N[C@@H](Cc2ccc3ccccc3c2) C(=O)N[C@@](C)(CC(C)C)C(=O)N1 |
| 28 | [4-Aminomethyl-phenylacetyl](1c)*SE(2c)W[Orn][Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](CCCN)NC(=O)[C@H](Cc3c[nH]c4ccccc34)NC2=O)C(=O)N[C @@H](Cc2ccc(OCCN)cc2)C(=O)N[C@@H](Cc2ccc3ccccc3c2)C(=O)N[C@ @](C)(CC(C)C)C(=O)N1 |
| 29 | [4-Aminomethyl-phenylacetyl](1c)*SE(2c)W[Q(Me)][Dab](2c)[Y(2-aminoethoxy)][2-Nal][2-Me-Leu]E(1c)[Dab][3-(3-Pyridyl)-Ala]-[NHMe] |
| | CNC(=O)CC[C@@H]1NC(=O)[C@H](Cc2c[nH]c3ccccc23)NC(=O)[C@@H]2 CCC(=O)NCC[C@H](NC1=O)C(=O)N[C@@H](Cc1ccc(OCCN)cc1)C(=O)N[ C@@H](Cc1ccc3ccccc3c1)C(=O)N[C@@](C)(CC(C)C)C(=O)N[C@H](C(=O) N[C@@H](CCN)C(=O)N[C@@H](Cc1cccnc1)C(=O)NC)CCC(=O)NCc1ccc(c c1)CC(=O)N[C@@H](CO)C(=O)N2 |
| 30 | [4-Aminomethyl-phenylacetyl](1c)*SE(2c)W[Q(2Me)][Dab](2c)[Y(2-aminoethoxy)][2-Nal][2-Me-Leu]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](CO)C(=O)N[C@H]2CCC(=O)NCC[C@H](N C(=O)[C@H](CCC(=O)N(C)C)NC(=O)[C@H](Cc3c[nH]c4ccccc34)NC2=O)C( =O)N[C@@H](Cc2ccc(OCCN)cc2)C(=O)N[C@@H](Cc2ccc3ccccc3c2)C(=O )N[C@@](C)(CC(C)C)C(=O)N1 |
| 31 | [4-Aminomethyl-phenylacetyl](1c)*SE(2c)WQ[Dab](2c)[Y(2-aminoethoxy)][2-Nal][2-Me-Leu]E(1c)S[3-(3-Pyridyl)-Ala]-[NHMe] |
| | CNC(=O)[C@H](Cc1cccnc1)NC(=O)[C@H](CO)NC(=O)[C@@H]1CCC(=O) NCc2ccc(cc2)CC(=O)N[C@@H](CO)C(=O)N[C@H]2CCC(=O)NCC[C@H](N C(=O)[C@H](CCC(N)=O)NC(=O)[C@H](Cc3c[nH]c4ccccc34)NC2=O)C(=O) N[C@@H](Cc2ccc(OCCN)cc2)C(=O)N[C@@H](Cc2ccc3ccccc3c2)C(=O)N[ C@@](C)(CC(C)C)C(=O)N1 |
| 32 | [4-Aminomethyl-phenylacetyl](1c)*SE(2c)WQ[Dab](2c)[Y(2-aminoethoxy)][2-Nal][2-Me-Leu]E(1c)[Nle][3-(3-Pyridyl)-Ala]-[NHMe] |
| | CCCC[C@H](NC(=O)[C@@H]1CCC(=O)NCc2ccc(cc2)CC(=O)N[C@@H](C O)C(=O)N[C@H]2CCC(=O)NCC[C@H](NC(=O)[C@H](CCC(N)=O)NC(=O)[ C@H](Cc3c[nH]c4ccccc34)NC2=O)C(=O)N[C@@H](Cc2ccc(OCCN)cc2)C(= O)N[C@@H](Cc2ccc3ccccc3c2)C(=O)N[C@@](C)(CC(C)C)C(=O)N1)C(=O) N[C@@H](Cc1cccnc1)C(=O)NC |
| 33 | [4-Aminomethyl-phenylacetyl](1c)*SE(2c)WQ[Dab](2c)[Y(2-aminoethoxy)][2-Nal][2-Me-Leu]E(1c)Q[3-(3-Pyridyl)-Ala]-[NHMe] |
| | CNC(=O)[C@H](Cc1cccnc1)NC(=O)[C@H](CCC(N)=O)NC(=O)[C@@H]1CC C(=O)NCc2ccc(cc2)CC(=O)N[C@@H](CO)C(=O)N[C@H]2CCC(=O)NCC[C @H](NC(=O)[C@H](CCC(N)=O)NC(=O)[C@H](Cc3c[nH]c4ccccc34)NC2=O) C(=O)N[C@@H](Cc2ccc(OCCN)cc2)C(=O)N[C@@H](Cc2ccc3ccccc3c2)C( =O)N[C@@](C)(CC(C)C)C(=O)N1 |
| 34 | [4-Aminomethyl-phenylacetyl](1c)*SE(2c)WQ[Dab](2c)[Y(2-aminoethoxy)][2-Nal][2-Me-Leu]E(1c)H[3-(3-Pyridyl)-Ala]-[NHMe] |
| | CNC(=O)[C@H](Cc1cccnc1)NC(=O)[C@H](Cc1cnc[nH]1)NC(=O)[C@@H]1 CCC(=O)NCc2ccc(cc2)CC(=O)N[C@@H](CO)C(=O)N[C@H]2CCC(=O)NCC [C@H](NC(=O)[C@H](CCC(N)=O)NC(=O)[C@H](Cc3c[nH]c4ccccc34)NC2= O)C(=O)N[C@@H](Cc2ccc(OCCN)cc2)C(=O)N[C@@H](Cc2ccc3ccccc3c2) C(=O)N[C@@](C)(CC(C)C)C(=O)N1 |
| 35 | [4-Aminomethyl-phenylacetyl](1c)*SE(2c)WQ[Dab](2c)[Y(2-aminoethoxy)][2-Nal][2-Me-Leu]E(1c)[THP][3-(3-Pyridyl)-Ala]-[NHMe] |
| | CNC(=O)[C@H](Cc1cccnc1)NC(=O)C1(NC(=O)[C@@H]2CCC(=O)NCc3ccc (cc3)CC(=O)N[C@@H](CO)C(=O)N[C@H]3CCC(=O)NCC[C@H](NC(=O)[C @H](CCC(N)=O)NC(=O)[C@H](Cc4c[nH]c5ccccc45)NC3=O)C(=O)N[C@@ H](Cc3ccc(OCCN)cc3)C(=O)N[C@@H](Cc3ccc4ccccc4c3)C(=O)N[C@@](C )(CC(C)C)C(=O)N2)CCOCC1 |
| 36 | [4-Aminomethyl-phenylacetyl](1c)*SE(2c)WQ[Dab](2c)[Y(2-aminoethoxy)][2-Nal][2-Me-Leu]E(1c)[hSer][3-(3-Pyridyl)-Ala]-[NHMe] |
| | CNC(=O)[C@H](Cc1cccnc1)NC(=O)[C@H](CCO)NC(=O)[C@@H]1CCC(=O )NCc2ccc(cc2)CC(=O)N[C@@H](CO)C(=O)N[C@H]2CCC(=O)NCC[C@H]( NC(=O)[C@H](CCC(N)=O)NC(=O)[C@H](Cc3c[nH]c4ccccc34)NC2=O)C(=O )N[C@@H](Cc2ccc(OCCN)cc2)C(=O)N[C@@H](Cc2ccc3ccccc3c2)C(=O)N[ C@@](C)(CC(C)C)C(=O)N1 |
| 37 | [4-Aminomethyl-phenylacetyl](1c)*SE(2c)WQ[Dab](2c)[Y(2-aminoethoxy)][2-Nal][2-Me-Leu]E(1c)[Ser(OMe)][3-(3-Pyridyl)-Ala]-[NHMe] |
| | CNC(=O)[C@H](Cc1cccnc1)NC(=O)[C@H](COC)NC(=O)[C@@H]1CCC(=O )NCc2ccc(cc2)CC(=O)N[C@@H](CO)C(=O)N[C@H]2CCC(=O)NCC[C@H]( NC(=O)[C@H](CCC(N)=O)NC(=O)[C@H](Cc3c[nH]c4ccccc34)NC2=O)C(=O )N[C@@H](Cc2ccc(OCCN)cc2)C(=O)N[C@@H](Cc2ccc3ccccc3c2)C(=O)N[ C@@](C)(CC(C)C)C(=O)N1 |
| 38 | [4-Aminomethyl-phenylacetyl](1c)*SE(2c)WQ[Dab](2c)[Y(2-aminoethoxy)][2-Nal][2-Me-Leu]E(1c)[{d}S][3-(3-Pyridyl)-Ala]-[NHMe] |
| | CNC(=O)[C@H](Cc1cccnc1)NC(=O)[C@@H](CO)NC(=O)[C@@H]1CCC(=O )NCc2ccc(cc2)CC(=O)N[C@@H](CO)C(=O)N[C@H]2CCC(=O)NCC[C@H]( NC(=O)[C@H](CCC(N)=O)NC(=O)[C@H](Cc3c[nH]c4ccccc34)NC2=O)C(=O )N[C@@H](Cc2ccc(OCCN)cc2)C(=O)N[C@@H](Cc2ccc3ccccc3c2)C(=O)N[ C@@](C)(CC(C)C)C(=O)N1 |
| 39 | [4-Aminomethyl-phenylacetyl](1c)*SE(2c)WQ[Dab](2c)[Y(2-aminoethoxy)][2-Nal][2-Me-Leu]E(1c)[2-Me-Ser][3-(3-Pyridyl)-Ala]-[NHMe] |
| | CNC(=O)[C@H](Cc1cccnc1)NC(=O)[C@](C)(CO)NC(=O)[C@@H]1CCC(=O) NCc2ccc(cc2)CC(=O)N[C@@H](CO)C(=O)N[C@H]2CCC(=O)NCC[C@H](N C(=O)[C@H](CCC(N)=O)NC(=O)[C@H](Cc3c[nH]c4ccccc34)NC2=O)C(=O) N[C@@H](Cc2ccc(OCCN)cc2)C(=O)N[C@@H](Cc2ccc3ccccc3c2)C(=O)N[ C@@](C)(CC(C)C)C(=O)N1 |
| 40 | [4-Aminomethyl-phenylacetyl](1c)*AE(2c)WQ[Dab](2c)[Y(2-aminoethoxy)][2-Nal][2-Me-Leu]E(1c)S[3-(3-Pyridyl)-Ala]-[NHMe] |
| | CNC(=O)[C@H](Cc1cccnc1)NC(=O)[C@H](CO)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](Cc2ccc(OCCN)cc2)C(=O)N[C@@H](Cc2ccc3ccccc3c2)C(=O)N[C @@](C)(CC(C)C)C(=O)N1 |
| 41 | [4-Aminomethyl-phenylacetyl](1c)*AE(2c)WQ[Dab](2c)[hPhe][2-Nal][Aib]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](CCc2ccccc2)C(=O)N[C@@H](Cc2ccc3ccccc3c2)C(=O)NC(C)(C)C( =O)N1 |
| 42 | [4-Aminomethyl-phenylacetyl](1c)*AE(2c)WQ[Dab](2c)[Y(2-aminoethoxy)][Cyclopropyl-Ala][Aib]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](Cc2ccc(OCCN)cc2)C(=O)N[C@@H](CC2CC2)C(=O)NC(C)(C)C(= O)N1 |
| 43 | [4-Aminomethyl-phenylacetyl](1c)*AE(2c)WQ[Dab](2c)[7-AzaTrp][2-Nal][Aib]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](Cc2c[nH]c3ncccc23)C(=O)N[C@@H](Cc2ccc3ccccc3c2)C(=O)NC( C)(C)C(=O)N1 |
| 44 | [4-Aminomethyl-phenylacetyl](1c)*AE(2c)WQ[Dab](2c)[Y(2-aminoethoxy)][7-AzaTrp][Aib]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](Cc2ccc(OCCN)cc2)C(=O)N[C@@H](Cc2c[nH]c3ncccc23)C(=O)NC (C)(C)C(=O)N1 |
| 45 | [4-Aminomethyl-phenylacetyl](1c)*AE(2c)WQ[Dab](2c)[beta-hTrp][2-Nal][Aib]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](Cc2c[nH]c3ccccc23)CC(=O)N[C@@H](Cc2ccc3ccccc3c2)C(=O)NC (C)(C)C(=O)N1 |
| 46 | [4-Aminomethyl-phenylacetyl](1c)*AE(2c)WQ[Dab](2c)[Y(2-aminoethoxy)][beta-hTrp][Aib]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](Cc2ccc(OCCN)cc2)C(=O)N[C@@H](Cc2c[nH]c3ccccc23)CC(=O)N C(C)(C)C(=O)N1 |
| 47 | [4-Aminomethyl-phenylacetyl](1c)*AE(2c)WQ[Dab](2c)[7-F-Trp][2-Nal][Aib]E(1c)[Dab][3-(3-Pyridyl)-Ala]-[NHMe] |
| | CNC(=O)[C@H](Cc1cccnc1)NC(=O)[C@H](CCN)NC(=O)[C@@H]1CCC(=O) NCc2ccc(cc2)CC(=O)N[C@@H](C)C(=O)N[C@H]2CCC(=O)NCC[C@H](NC (=O)[C@H](CCC(N)=O)NC(=O)[C@H](Cc3c[nH]c4ccccc34)NC2=O)C(=O)N[ C@@H](Cc2c[nH]c3c(F)cccc23)C(=O)N[C@@H](Cc2ccc3ccccc3c2)C(=O)N C(C)(C)C(=O)N1 |
or a pharmaceutically acceptable salt or solvate thereof;
wherein:
* denotes that the (1c), (2a), (2c), and (4c) bridges use the peptide backbone amine or carboxylic acid at the *N*- or *C*-terminus, not the side chain amine or carboxylic acid
(1c) denotes the [2,11] lactam bridge; (2a) denotes the [4,7] 1,3-dithio-propan-2-one bridge; (2c) denotes the [4,7] lactam bridge; (4c) denotes the [1,11] lactam bridge.

12. A pharmaceutical composition comprising a compound according to any one of the preceding claims in combination with a pharmaceutically acceptable carrier, excipient or vehicle.

13. A method for the synthesis of a compound according to any one of claims 1 to 11, comprising synthesising the analogue by solid-phase or liquid-phase peptide synthesis methodology, optionally isolating and/or purifying the final product, and optionally further comprising the step of forming an amide bond between the amino acid residues at position X1 or at position X2 when X1 is absent and at position X11, and optionally further comprising the step of forming an amide bond or forming two thioether bonds with a linker between the amino acid residues at positions X4 and X7.

14. A compound according to any one of claims 1 to 11, or a pharmaceutical composition according to claim 12, for use in a method of medical treatment.

15. A compound according to any one of claims 1 to 11, or a pharmaceutical composition according to claim 12, for use in a method of prevention or treatment of inflammatory bowel disease (IBD), Crohn's Disease, ulcerative colitis, and psoriasis;
optionally wherein the compound or pharmaceutical compoisition is for use in a method of prevention or treatment of inflammatory bowel disease (IBD) and/or psoriasis.
